(19) Europäisches Patentamt
European Patent Office
Office européen des brevets

(11) EP 3 553 054 B1

(12) # EUROPEAN PATENT SPECIFICATION

(45) Date of publication and mention of the grant of the patent:
**11.01.2023 Bulletin 2023/02**

(21) Application number: **18398004.4**

(22) Date of filing: **13.04.2018**

(51) International Patent Classification (IPC):
***C07D 311/16*** *(2006.01)* ***G01N 33/533*** *(2006.01)*

(52) Cooperative Patent Classification (CPC):
**C07D 311/16**

(54) **FLUORESCENT COUMARIN COMPOUNDS AND METHOD OF SYNTHESIS THEREOF**

FLUORESZIERENDE COUMARINVERBINDUNGEN UND SYNTHESEVERFAHREN DAFÜR

COMPOSÉS FLUORESCENTS DE COUMARIN ET LEUR PROCÉDÉ DE SYNTHÈSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**16.10.2019 Bulletin 2019/42**

(73) Proprietor: **Universidade de Évora**
**7000-803 Évora (PT)**

(72) Inventors:
- **Pereira, António Manuel Deométrio Rodrigues Lourenço**
  **7000-890 Évora (PT)**
- **Martins, Sérgio Miguel Amaral**
  **7000-513 Évora (PT)**
- **Caldeira, Ana Teresa Fialho Caeiro**
  **7000-651 Évora (PT)**
- **Candeias, António José Estevão Grande**
  **7000-651 Évora (PT)**

(74) Representative: **Alves Moreira, Pedro**
**RCF Protecting Innovation, S.A.**
**Rua Dom Francisco Manuel de Melo, 15, 3°**
**1070-085 Lisbon (PT)**

(56) References cited:
**WO-A1-87/03589**

- **SÉRGIO MARTINS ET AL: "Styryl and phenylethynyl based coumarin chromophores for dye sensitized solar cells", JOURNAL OF PHOTOCHEMISTRY AND PHOTOBIOLOGY, A: CHEMISTRY., vol. 353, 1 February 2018 (2018-02-01), pages 564-569, XP055484828, CH ISSN: 1010-6030, DOI: 10.1016/j.jphotochem.2017.12.018**
- **KAI KITAMURA ET AL: "Visible light-induced nitric oxide release from a novel nitrobenzene derivative cross-conjugated with a coumarin fluorophore", BIOORGANIC & MEDICINAL CHEMISTRY LETTERS, vol. 24, no. 24, 1 December 2014 (2014-12-01), pages 5660-5662, XP055732415, AMSTERDAM, NL ISSN: 0960-894X, DOI: 10.1016/j.bmcl.2014.10.075**
- **KAI KITAMURA ET AL: "Visible light-induced nitric oxide release from a novel nitrobenzene derivative cross-conjugated with a coumarin fluorophore", BIOORGANIC & MEDICINAL CHEMISTRY LETTERS, vol. 24, no. 24, 1 December 2014 (2014-12-01), pages 5660-5662, XP055732418, AMSTERDAM, NL ISSN: 0960-894X, DOI: 10.1016/j.bmcl.2014.10.075**
- **Martins Sérgio M. A. ET AL: "An efficient methodology for the synthesis of 3-styryl coumarins", JOURNAL OF THE BRAZILIAN CHEMICAL SOCIETY, vol. 23, no. 4, 1 January 2012 (2012-01-01), pages 688-693, XP055798556, São Paulo; BR ISSN: 0103-5053, DOI: 10.1590/S0103-50532012000400014 Retrieved from the Internet: URL:http://www.scielo.br/pdf/jbchs/v23n4/a14v23n4.pdf>**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- GORDO JOANA ET AL: "Convenient Synthesis of 3-Vinyl and 3-Styryl Coumarins", ORGANIC LETTERS, vol. 13, no. 19, 7 October 2011 (2011-10-07), pages 5112-5115, XP055798830, US ISSN: 1523-7060, DOI: 10.1021/ol201983u

## Description

### FIELD OF THE INVENTION

The present invention relates to a novel family of vinyl-coumarins and method of synthesis thereof. In particular, the vinyl-coumarins of the present invention are intermediates in the production of dye-sensitized solar cells, fluorescent labels for biomolecules and polymers.

### BACKGROUND OF THE INVENTION

Coumarins have been extensively described in the literature.

In the last two decades a huge amount of work on the application of organic dyes as photosensitizers for DSSCs (Dye-sensitized solar cells) and as fluorescent labels for biomolecules and polymers has been reported.

US Patent No. 4956480 entitled "7-amino-4-methyl-coumarin-3-carboxyalkyl derivates and fluorescent conjugates thereof" refers to coumarin compounds for use as fluorescent labelling agents having the following formulas

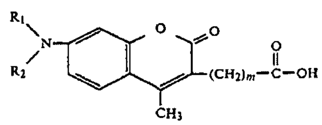 , 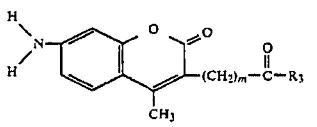 or 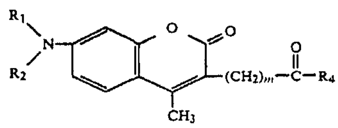 .

The synthesis of these compounds is afforded by different reactions at 3-position of the coumarin.

Martins et al. in "Styryl and phenylethynyl based coumarin chromophores for dye sensitized solar cells", Journal of Photochemistry and Photobiology A: Chemistry (2018), 353, 546-569, refers to coumarins as a well-known class of compounds of natural occurrence in several plant families, for which a growing interest in last decades due to their pharmacological activity and photophysical properties have aroused. They constitute one of the largest classes of fluorescence sensors, which accounts for its increased application as fluorescent probes and are also widely used in emission layers in organic light-emitting diodes (OLED).

The application of coumarin derivatives as organic dyes has been hindered due to their colour spectra falling in the UV range and the relatively low intensity of their absorption bands.

A recurring strategy that became essential for the development of new organic dyes and that has been applied for coumarins involves Donor-$\pi$ bridge-Acceptor molecules. This extends dye absorption bands to longer wavelengths and increases their molar extinction coefficients.

Martins *et al.* (2018) uses 3-bromo-coumarin derivatives (3-bromo-6,7-dimethoxycoumarin and 3-bromo-6,7-bis(undecyloxy)coumarin) as the starting material for synthesis of coumarin chromophores via several carbon coupling reactions. The result of these experiments was coumarin dyes with extended conjugation and enhanced photophysical properties, which can be used as sensitizers for solar cells due to its higher efficiencies and increased fluorescence.

A different approach based on delocalization of the conjugated $\pi$-electron system is disclosed by Gordo et al. in "Convenient Synthesis of 3-Vinyl and 3-Styryl Coumarins", Organic Letters (2011), Vol. 13, No. 19, 5112-5115. A variety of 2-hydroxy aldehydes are reacted with 3-butenoic acid in order to afford 3-vinyl-coumarins, either following a one-pot procedure or through isolation of intermediates. As expected, Heck coupling reactions with aryl halides produce compounds with increased fluorescence.

From the above documents, it is easily understood that the classic methodology for synthesis of vinyl-coumarins is usually provided with reactions at 3-position of the coumarin.

However, the formylation reactions in coumarins are not fully regioselective thereby producing mixtures of formylated products resulting in lowering of reactional yield and hard isolation workout of the intended product.

Also, a vinylation reaction starting from aldehydes generally involves variable reactional times that are not predictable. These can be around 1 hour but there are also reports of reactions during more than 48 hours. For these longer reactional times, it is observed an increased degree of polymerization in vinylic derivatives which is notably prejudicial for the reactional yields. Therefore, due to said reactional times, vinylic derivatives are easily polymerized, which leads to a substantially lowering of the reactional yield and hard isolation workout of the reactional product.

Other studies regarding the delocalization of the conjugated $\pi$-electron system were performed at the 5-position. Avó et al. in "A Family of Styrylcoumarins: Synthesis, Spectroscopic, Photophysical and Photochemical Properties", ChemPlusChem (2013), 78, 789-792, have studied the effect of delocalization of the conjugated $\pi$-electron system at the 5-position of 6-methoxycoumarin chromophore. These studies involved different chemical reactions such as bromination, Suzuki cross-coupling reaction, Heck palladium coupling reactions, finally leading to 5-styrylcoumarins. However, such experiments have revealed a complex and time-consuming process which is not so used in practice as would be

expected.

In view of the above, there is still the need to provide new compounds, acting as intermediates in the synthesis of photosensitizers for dye-sensitized solar cells, fluorescent labels for biomolecules and polymers; methods for obtaining said compounds that can produce full regioselective compounds with yields higher than 96% and easy to isolate; and new photosensitizers and fluorescent labels incorporating in their core structure the new compound intermediates.

## SUMMARY OF THE INVENTION

Surprisingly, the aforementioned problems were solved by the present invention.

The present invention provides a compound of formula (III)

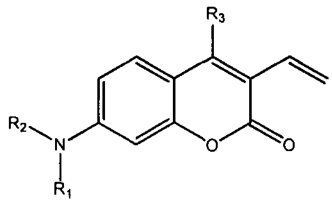

(III)

wherein:

$R_1$ and $R_2$ are independently $-(CH_2)_n-CH_3$, wherein n is 0, 1, 2 or 3; and
$R_3$ is $-(CH_2)_n-CH_3$, wherein n is 0 or 1.

The compound of formula (III) wherein $R_1$ and $R_2$ are $-(CH_2)-CH_3$ and $R_3$ is $-CH_3$ is a preferred embodiment of the present invention

Further, the present invention provides a method for synthesis of compounds of formula (III) comprising the following steps:

- reacting a compound of formula (I)

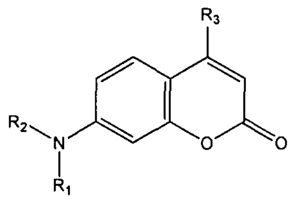

(I)

wherein:

$R_1$ and $R_2$ are independently $-(CH_2)_n-CH_3$, wherein n is 0, 1, 2 or 3; and
$R_3$ is $-(CH_2)_n-CH_3$, wherein n is 0 or 1,

with bromine in a reaction solvent selected from the group comprising chloroform, dichloromethane, 1,2-dichloroethane, acetonitrile, a mixed solvent thereof and the like, under stirring at a temperature between 0 °C and 5 °C for 10 hours to 24 hours;

- adding to the reactional mixture a base selected from the group comprising triethylamine, 4-(dimethylamino)pyridine, piperidine, piperazine, pyridine, 1,4-diazabicyclo[2.2.2]octane (DABCO), 1,8-diazabicyclo[5.4.0]undec-7-ene (DBU) and the like, and stirring the reactional mixture at a temperature between 20 °C and 25 °C for 30 minutes to 3 hours;
- evaporating the reaction mixture to dryness and purifying the residue to produce a compound of formula (II)

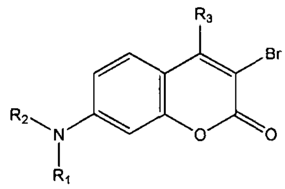

(II);

- reacting a compound of formula (II) with potassium vinyltrifluoroborate and [1,1'-bis(diphenylphosphino)ferrocene] dichloropalladium(II) complex with dichloromethane in a reaction solvent selected from the group comprising n-propanol, 2-propanol, ethanol, tert-butanol, 2-butanol, acetonitrile, a mixed solvent thereof and the like, and stirring under inert atmosphere at a temperature between 78 °C and 98 °C for 15 minutes to 2 hours; and
- evaporating the reaction mixture to dryness and purifying the residue to produce a compound of formula (III)

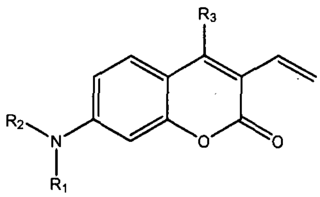

(III).

Also provided is a compound of formula (A)

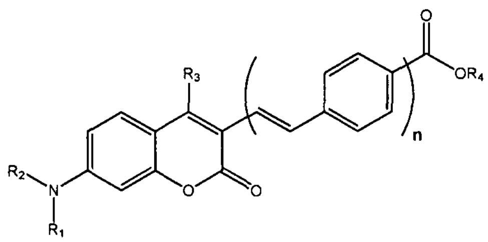

(A)

wherein:

n is 1 or 2,
$R_1$ and $R_2$ are independently $-(CH_2)_n-CH_3$, wherein n is 0, 1, 2 or 3;
$R_3$ is $-(CH_2)_n-CH_3$, wherein n is 0 or 1; and
R4 is

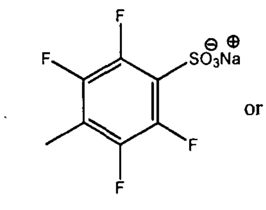

or

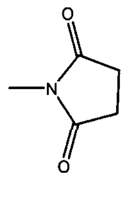

.

In an embodiment, the invention provides a compound of formula (A), wherein n is 1, $R_1$ and $R_2$ are $-(CH_2)-CH_3$, $R_3$ is $-CH_3$ and $R_4$ is

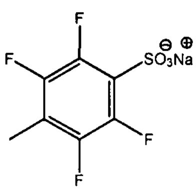

.

The present invention also provides a compound of formula (B)

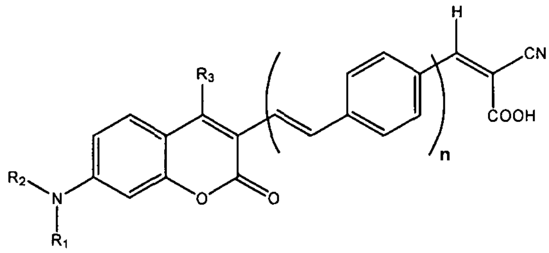

(B)

wherein:

n is 1 or 2,
$R_1$ and $R_2$ are independently $-(CH_2)_n-CH_3$, wherein n is 0, 1, 2 or 3; and
$R_3$ is $-(CH_2)_n-CH_3$, wherein n is 0 or 1.

In another embodiment, the invention provides a compound of formula (B) wherein n is 1, $R_1$ and $R_2$ are $-(CH_2)-CH_3$ and $R_3$ is $-CH_3$.

## BRIEF DESCRIPTION OF THE DRAWINGS

Figure 1 represents absorbance spectra of the chromophore obtained in Example 2 ((I-1): 7-diethylamino-4-methylcoumarin, (III-1): 7-diethylamino-4-methyl-3-vinyl-coumarin and (VI-1): (E)-4-(4-(2-(7-(diethylamino)-4-methylcoumarin-3-yl)vinyl)benzoyl)-2,3,5,6-tetrafluoro-benzenesulfonate).

Figure 2 represents emission spectra of the chromophore obtained in Example 2 ((I-1): 7-diethylamino-4-methylcoumarin, (III-1): 7-diethylamino-4-methyl-3-vinyl-coumarin and (VI-1): (E)-4-(4-(2-(7-(diethylamino)-4-methyl-coumarin-3-yl)vinyl)benzoyl)-2,3,5,6-tetrafluoro-benzenesulfonate).

Figure 3 represents absorbance spectra of dye-sensitized solar cells (DSSC) obtained in Example 3 ((I-1): 7-diethylamino-4-methylcoumarin, (III-1): 7-diethylamino-4-methyl-3-vinyl-coumarin and (VIII-1): 2-cyano-3-(4-((E)-2-(7-(diethylamino)-4-methylcoumarin-3-yl)vinyl)phenyl)acrylic acid).

Figure 4 represents emission spectra of the dye-sensitized solar cells (DSSC) obtained in Example 3 ((I-1): 7-diethylamino-4-methylcoumarin, (III-1): 7-diethylamino-4-methyl-3-vinyl-coumarin and (VIII-1): 2-cyano-3-(4-((E)-2-(7-(diethylamino)-4-methylcoumarin-3-yl)vinyl)phenyl)acrylic acid).

## DETAILED DESCRIPTION OF THE INVENTION

One object of the present invention is to provide novel compounds with a vinyl-coumarin core structure obtained via an efficient, faster and cheaper reaction, which act as intermediates for producing dye-sensitized solar cells, fluorescent labels for biomolecules and polymers.

In the present invention, "coumarin" means 2H-chromen-2-one, a benzo-alpha-pyrone (lactone of o-hydroxycinnamic acid. In the present invention, "vinyl-coumarin" means a benzo-alpha-pyrone substituted by one vinyl (ethenyl) group. By "room temperature" is meant a temperature in the range between 20 °C to 25 °C. By "nucleophilic aromatic substitution" is meant a substitution reaction in organic chemistry in which the nucleophile displaces a good leaving group, such as a halide, on an aromatic ring.

To solve the problems identified above, the present invention provides a novel family of vinyl-coumarins with general formula (III)

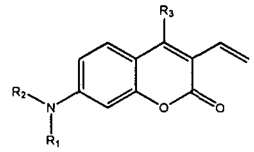

(III)

wherein:

$R_1$ and $R_2$ are independently $-(CH_2)_n-CH_3$, wherein n is 0, 1, 2 or 3; and
$R_3$ is $-(CH_2)_n-CH_3$, wherein n is 0 or 1.

Another object of the present invention is to provide a method for synthesis of the compounds of formula (III). In detail, a compound of formula (I)

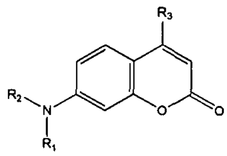

(I)

is reacted with bromine in a proper solvent under stirring. A base is added and stirring is continued. The reaction mixture is evaporated to dryness and the residue is purified by, for example, flash column chromatography on silica gel to produce a compound of formula (II)

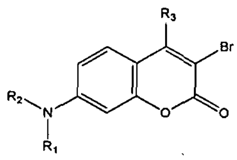

(II).

Preferred bases are selected from the group comprising triethylamine, 4-(dimethylamino)pyridine, piperidine, piperazine, pyridine, 1,4-diazabicyclo[2.2.2]octane (DABCO), 1,8-diazabicyclo[5.4.0]undec-7-ene (DBU) and the like. The reaction solvent is not specifically limited as long as it does not significantly inhibit the reaction. Preferred reaction solvents are selected from the group comprising chloroform, dichloromethane, 1,2-dichloroethane, acetonitrile, a mixed solvent thereof and the like. Further, water can be added to the reaction solvent. During the addition of bromine, the preferred temperature is between 0 °C and 5 °C. Although the reaction temperature after addition of the base is not limited, a range between 20 °C and 25 °C is preferred. The bromination reaction time is preferably from 10 hours to 24 hours. After the addition of the base, the reaction time is preferably from 30 minutes to 3 hours. The purification of the residue can be made by filtration through celite or flash column chromatography on silica gel. The chromatographic eluent is not specifically limited as long as it allows the purification of the compound of formula (II). Preferred chromatographic eluents are selected from the group comprising chloroform, dichloromethane, methanol, a mixed eluent thereof and the like. The mesh particle size of silica gel is not specifically limited as long as it allows the purification of the compound of formula (II). Preferred mesh particle sizes of silica gel are selected from the group comprising 60-120, 70-230, 200-400, 230-400.

Next, the compound of formula (II) is reacted with potassium vinyltrifluoroborate and [1,1-bis(diphenylphosphino)ferrocene]dichloropalladium(II) complex with dichloromethane and then the mixture is stirred under inert atmosphere. The reaction mixture is evaporated to dryness and the residue is purified by flash column chromatography on silica gel to produce a compound of formula (III),

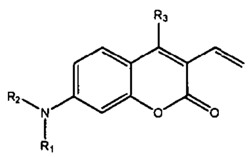

(III).

Preferred bases are selected from the group comprising triethylamine, 4-(dimethylamino)pyridine, piperidine, piperazine, pyridine, 1,4-Diazabicyclo[2.2.2]octane (DABCO), 1,8-diazabicyclo[5.4.0]undec-7-ene (DBU) and the like. The reaction solvent is not specifically limited as long as it does not significantly inhibit the reaction. Preferred reaction solvents are selected from the group comprising *n*-propanol, 2-propanol, ethanol, *tert*-butanol, 2-butanol, acetonitrile, a mixed solvent thereof and the like. Although the reaction temperature is not limited, a range between 78 °C and 98 °C is preferred. The reaction time is preferably from 15 minutes to 2 hours. The purification of the residue can be made by filtration through celite or flash column chromatography on silica gel. The chromatographic eluent is not specifically limited as long as it allows the purification of the compound of formula (III). Preferred chromatographic eluents are selected from the group comprising chloroform, dichloromethane, methanol, a mixed eluent thereof and the like. The mesh particle size of silica gel is not specifically limited as long as it allows the purification of the compound of formula (II). Preferred mesh particle sizes of silica gel are selected from the group comprising 60-120, 70-230, 200-400, 230-400. The purification of the residue can also be made by filtration through celite.

The compounds of the present invention represented by formula (III) allow a greater delocalization of $\pi$-electrons due to the presence of the vinyl group at 3-position. Although the vinylation reaction at 3-position of a coumarin is known from the prior art, the specific vinylation reaction in bromide precursor (II) was not previously disclosed for this type of coumarins with nitrogenous substituents.

It is common knowledge that the success of the reactions with coumarins having nitrogenous substituents depend on the pH at which they are performed. If the pH value is lower than 7, nitrogen atoms are susceptible to protonation which radically changes the reactivity and solubility of the reaction, thereby producing undesirable derivatives and low reactional yields. So, in order to obtain high yields, this type of reactions usually occurs at a pH value equal or higher than 7 with a need of duly control of said pH value during the reaction.

However, the method of the present invention is performed without control of the pH and surprisingly the reactional yield is still higher than 96%.

Also, by introducing a bromine atom at 3-position, the necessary conditions for performing a C-C coupling reaction with organometallic catalysis are achieved. Before the present invention and without said bromine atom at 3-position, the reaction to be performed for obtaining a vinyl coumarin would have to be the formylation as described in the state of the art with all known and related drawbacks.

Therefore, the presence of bromine atom at 3-position of the coumarin allows the molecule to behave as an aryl bromide. This type of compounds is known by its inability to suffer nucleophilic aromatic substitution reactions when vinyl group is introduced in the coumarin ring. However, the presence of the amine group (electrons donor) at 7-position renders the nucleophilic aromatic substitution reaction fully impossible at 3-position.

Surprisingly, the products obtained through the reactional route of the present invention have reactional yields above 96%, full regioselectivity and they are easily isolated. Further, the starting materials for these chemical reactions are cheaper than the coumarin reagents commonly used in the art, which represents a cost-saving in the complete synthesis but assuring the same or better properties of the achieved compounds. All reactions involved in the synthesis of these compounds are more simple and faster than those commonly used by the skilled person. So, the method of the present invention represents an alternative, simple and cost-saving method in view of the processes already known.

In a further embodiment of the present invention, the compounds of the present invention allow for producing new dye-sensitized solar cells, fluorescent labels for biomolecules and polymers of general formulas (A) and (B), respectively,

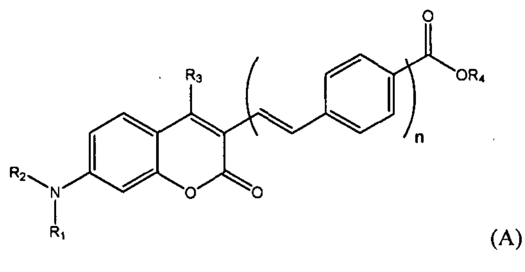

(A)

wherein:

n is 1 or 2,
$R_1$ and $R_2$ are independently $-(CH_2)_n-CH_3$, wherein n is 0, 1, 2 or 3;
$R_3$ is $-(CH_2)_n-CH_3$, wherein n is 0 or 1; and
R4 is

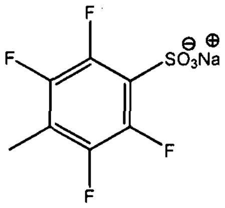

or

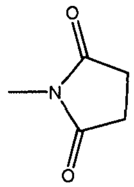

;

and

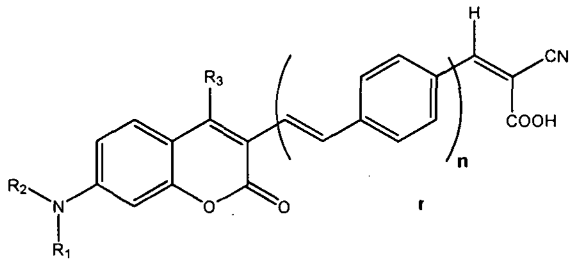

(B)

wherein:

n is 1 or 2,
$R_1$ and $R_2$ are independently $-(CH_2)_n-CH_3$, wherein n is 0, 1, 2 or 3; and
$R_3$ is $-(CH_2)_n-CH_3$, wherein n is 0 or 1.

It is critical to assess the fluorescent behaviour of the compounds of the present invention. It is important to assure that the absorbance of these compounds corresponds to wavelengths higher than those of the starting materials.

In the present invention, due to the electronic conjugation provided by the vinyl group at 3-position, the absorbance of the compounds is above 388 nm against 371 nm of the starting material. This highlights the effect of $\pi$-bridge by enhancing and allowing the delocalization to the electron acceptor.

EXAMPLES

Hereinafter, the present invention is described in more detail and specifically with reference to the examples, which are not intended to be limitative.

**Example 1: Synthesis of 7-diethylamino-4-methyl-3-vinyl coumarin (III-1)**

Step 1: Preparation of 3-bromo-7-diethylamino-4-methyl-coumarin (II-1)

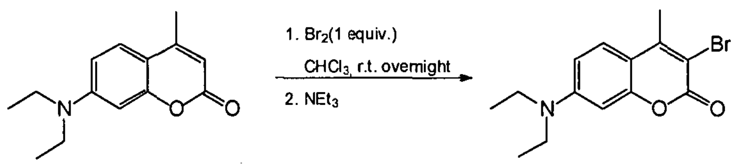


(I-1) (II-1)

To a solution of 7-diethylamino-4-methyl-coumarin (I-1) (100 mg, 0.432 mmol) in $CHCl_3$ (2 mL) was added bromine (0.022 mL, 0.432 mmol) in an ice bath. After stirring overnight at room temperature, $NEt_3$ (0.200 mL) was added cautiously and the mixture stirred for further 30 minutes. Then, the reaction mixture was evaporated to dryness and the residue was purified by flash column chromatography on silica gel (230-400 mesh; $CHCl_3$/MeOH (99:1)) to yield 3-bromo-7-diethylamino-4-methyl-coumarin (II-1) (131 mg, 0.424 mmol, 98%).

$^1H$ NMR (400 MHz, $CDCl_3$) $\delta$ (ppm): 1.19 (6H, t, $J$ = 7.1, $N(CH_2CH_3)_2$), 2.50 (3H, s, 4-$CH_3$), 3.40 (4H, q, $J$ =

7.1, N($CH_2CH_3$)$_2$), 6.46 (1H, s, H-8), 6.59 (1H, d, $J_{6,5}$ = 9.0, H-6), 7.40 (1H, d, $J_{5,6}$= 9.0, H-5).

$^{13}C$ NMR (100 MHz, $CDCl_3$) δ: 12.5 (C11, C13), 19.2 (C9), 44.9 (C10,C12), 97.3 (C8), 105.7 (C3), 109.0 (C4a), 126.2 (C6), 126.2 (C5), 150.7 (C7), 151.6 (C4), 154.5 (C8a), 158.3 (C2).

Step 2: Preparation of 7-diethylamino-4-methyl-3-vinyl-coumarin (III-1)

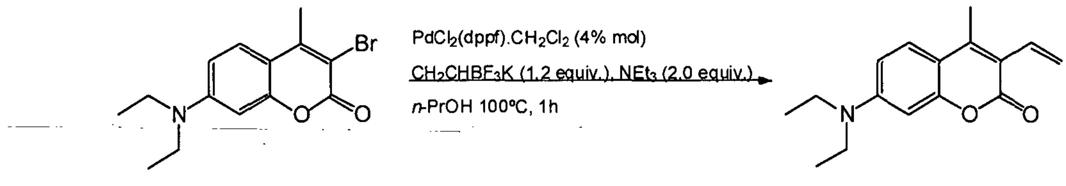


(II-1) (III-1)

To a solution of 3-bromo-7-(diethylamino)-4-methylcoumarin (II-1) (100 mg, 0.322 mmol), potassium vinyltrifluoroborate (52 mg, 0.387 mmol), PdCl$_2$(dppf).$CH_2Cl_2$ (11 mg, 0.013 mmol), and $NEt_3$ (0.090 mL, 0.645 mmol) in *n*-PrOH (6 mL) were added and stirred at reflux under inert atmosphere for a period of 1 hour. After cooling to room temperature, the reaction mixture was evaporated to dryness and the residue was purified by flash column chromatography on silica gel (230-400 mesh; $CH_2Cl_2$) to yield 7-diethylamino-4-methyl-3-vinyl coumarin **(III-1)** (80 mg, 0.309 mmol, 96%).

$^1H$ NMR (400 MHz, $CDCl_3$) δ (ppm): 1.19 (6H, t, *J* = 7.1, N($CH_2CH_3$)$_2$), 2.41 (3H, s, 4-$CH_3$), 3.39 (4H, q, *J* = 7.1, N($CH_2CH_3$)$_2$), 5.51 (1H, dd, $J_{2'a, 2'b}$ = 2.08, 2.1, $J_{2'a, 1'}$ = 11.7, H-2'a), 6.03 (1H, dd, $J_{2'b, 2'a}$ = 2.1, $J_{2'b, 1'}$ = 17.5, H-2'b), 6.46 (1H, d, $J_{8,6}$= 2.6, H-8), 6.58 (1H, dd, $J_{6,8}$ = 2.6, $J_{6,5}$ = 9.1, H-6), 6.70 (1H, dd, $J_{1', 2'a}$ = 11.7, $J_{1', 2'b}$ = 17.5, H-1'), 7.43 (1H, d, $J_{5,6}$= 9.1, H-5).

$^{13}C$ NMR (100 MHz, $CDCl_3$) δ: 12.7 (C11, C13), 15.1 (C9), 44.9 (C10,C12), 97.3 (C8), 108.8 (C6), 109.7 (C3), 116.4 (C4a), 120.3 (C2'), 126.2 (C5), 129.5 (C1'), 147.7 (C7), 150.3 (C4), 154.6 (C8a), 161.2 (C2).

MS-TOF(+) calc. for $C_{16}H_{19}NO_2Na$ $[M+Na]^+$ 280.1308 found 280.1309 IV $\nu_{max}^{KBr}$ $cm^{-1}$ : 2969, 2926, 1703, 1620, 1604, 1577, 1517, 1409, 1355, 1263, 1145, 1076, 823, 786. UV $\lambda_{max}^{CH_3CN}$ nm : 208, 261, 388.

**Example 2: Synthesis of sodium (*E*)-4-(4-(2-(7-(diethylamino)-4-methyl-coumarin-3-yl)vinyl)-benzoyl)-2,3,5,6-tetrafluorobenzenesulfonate (VI-1)**

Step 1: Preparation of *(E)*-4-(2-(7-(diethylamino)-4-methyl-coumarin-3-yl)vinyl)methylbenzoate (IV-1)

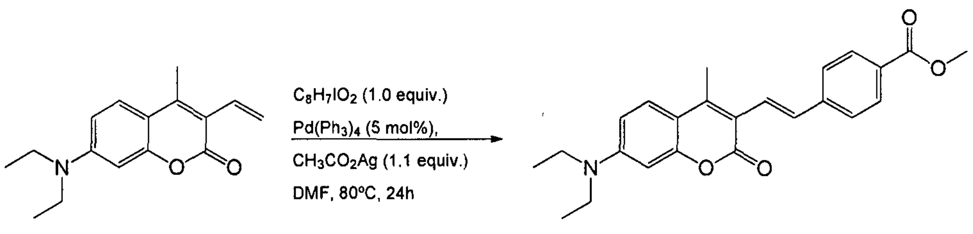


(III-1) (IV-1)

To a solution of 7-diethylamino-4-methyl-3-vinyl-coumarin (III-1) (100 mg, 0.389 mmol) from Example 1, methyl 4-iodobenzoate (93.0 mg, 0.353 mmol), $Pd(PPh_3)_4$ (20 mg, 0.018 mmol), and $CH_3CO_2Ag$ (65 mg, 0.389 mmol) in DMF (2.0 mL) were added and stirred at 80 °C, under inert atmosphere, for a period of 24 h. After cooling to room temperature, the reaction mixture was evaporated to dryness and the residue was purified by flash column chromatography on silica gel (230-400 mesh; $CH_2Cl_2$) to yield (E)-4-(2-(7-(diethylamino)-4-methyl-coumarin-3-yl)vinyl)methylbenzoate (IV-1) (127 mg, 0.325 mmol, 92%).

$^1H$ NMR (400 MHz, $CDCl_3$) δ (ppm): 1.21 (6H, t, *J* = 7.0, N($CH_2CH_3$)$_2$), 2.52 (3H, s, 4-$CH_3$), 3.41 (4H, q, *J* = 7.0, N($CH_2CH_3$)$_2$), 3.91 (3H, s, $OCH_3$), 6.48 (1H, s, H-8), 6.61 (1H, d, $J_{6,5}$ = 9.1, H-6), 7.26 (1H, d, $J_{2', 1'}$ = 16.1, H-2'),

7.49 (1H, d, $J_{5,6}$= 9.1, H-5), 7.56 (2H, d, $J_{4',5''}$ = 8.0, H-4', H-8'), 7.72 (1H, d, $J_{1',2'}$ = 16.1, H-1'), 7.99 (2H, d, $J_{5',4'}$ = 8.0, H-5', H-7').

$^{13}$C NMR (100 MHz, $CDCl_3$) δ: 12.6 (C11, C13), 15.1 (C9), 44.9 (C10,C12), 52.2 (OCH3) 97.3 (C8), 109.0 (C6), 109.9 (C3), 115.3 (C4a), 124.1 (C2'), 126.4 (C5, C5', C7'), 128.8 (C6'), 130.0 (C4', C8'), 132.3 (C1'), 143.1 (C3'), 148.5 (C7), 150.5 (C4), 154.8 (C8a), 160.9 (C2), 167.1 (*C*OOCH3).

MS-TOF(+) calc. for $C_{24}H_{26}NO_4$ $[M+H]^+$ 392.1856 found 392.1860. $IV\ \nu_{max}^{KBr}\ cm^{-1}$ : 2973, 1715, 1622, 1611, 1599, 1572, 1517, 1412, 1356, 1269, 1178, 1146, 1109, 758. $UV\ \lambda_{max}^{CH_3CN}\ nm$ : 203, 247, 309, 427.

Step 2: Preparation of *(E)*-4-(2-(7-(diethylamino)-4-methyl-coumarin-3-yl)vinyl)benzoic acid (V-1)

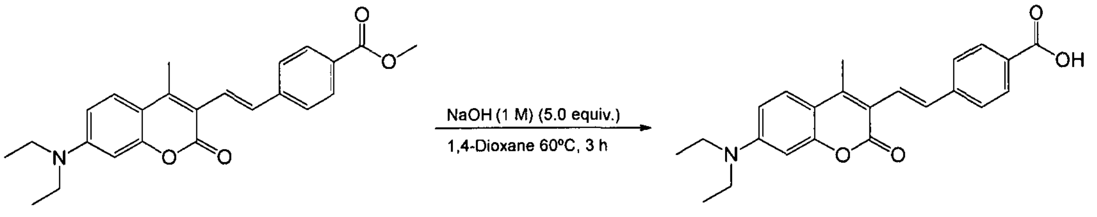


(IV-1) (V-1)

To a solution of *(E)*-4-(2-(7-(diethylamino)-4-methyl-coumarin-3-yl)vinyl)methylbenzoate (IV-1) (100 mg, 0.255 mmol) in 1,4-dioxane (1.3 mL) was added 1 M sodium hydroxide (1.3 mL, 1.277 mmol) and stirred at 60 °C for about 3 h. After cooling to room temperature, the reaction was neutralized with HCl (10%) solution. The reaction mixture was evaporated to dryness and the residue was purified by flash column chromatography on silica gel (230-400 mesh; $CHCl_3$/MeOH (9:1) to yield *(E)*-4-(2-(7-(diethylamino)-4-methyl-coumarin-3-yl)vinyl)benzoic acid (V-1) (87 mg, 0.230 mmol, 90%).

$^{1}$H NMR (400 MHz, DMSO-$d_6$) δ (ppm): 1.13 (6H, t, *J* = 6.7, $N(CH_2CH_3)_2$), 2.56 (3H, s, 4-$CH_3$), 3.44 (4H, q, *J* = 6.7, $N(CH_2CH_3)_2$), 6.52 (1H, s, H-8), 6.73 (1H, d, $J_{6,5}$ = 9.1, H-6), 7.38 (1H, d, $J_{2',1'}$ = 16.1, H-2'), 7.71-766 (4H, m, H-5, H-4', H-8', H-1'), 7.91 (2H, d, $J_{5',4'}$ = 7.9, H-5', H-7'), 12.87 (1H, s, COOH).

$^{13}$C NMR (100 MHz, $CDCl_3$) δ: 12.4 (C11, C13), 14.8 (C9), 44.1 (C10,C12), 96.3 (C8), 108.9 (C3), 109.2 (C6), 113.6 (C4a), 124.4 (C2'), 126.4 (C5', C7'), 127.2 (C5), 129.2 (C6'), 129.8 (C4', C8'), 130.7 (C1'), 142.4 (C3'), 149.8 (C7), 150.4 (C4), 154.3 (C8a), 159.6 (C2), 167.1 ($CO_2H$).

MS-TOF(+) calc. for $C_{23}H_{24}NO_4$ $[M+H]^+$ 378.169985 found 378.1709. $IV\ \nu_{max}^{KBr}\ cm^{-1}$ : 2965, 2925, 1678, 1593, 1566, 1513, 1409, 1354, 1262, 956, 827, 756. $UV\ \lambda_{max}^{CH_3CN}\ nm$ : 197, 245, 306, 418.

Step 3: Preparation of (E)-4-(4-(2-(7-(diethylamino)-4-methyl-coumarin-3-yl)vinyl)benzoyl)-2,3,5,6-tetrafluoro-benzenesulfonate (VI-1)

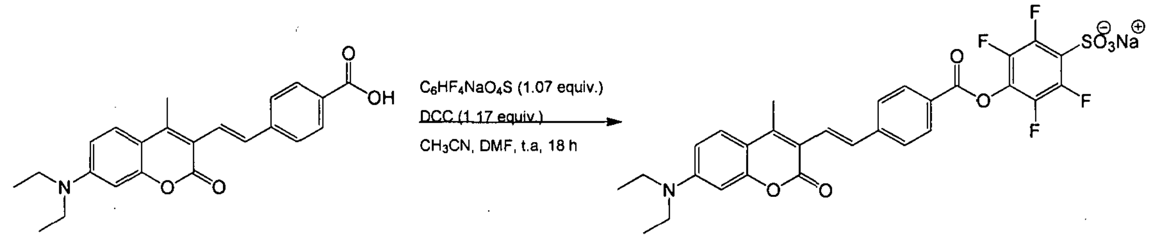


(V-1) (VI-1)

To a solution of (E)-4-(2-(7-(diethylamino)-4-methyl-coumarin-3-yl)vinyl) benzoic acid (V-1) (100 mg, 0.265 mmol), 4-sulfotetrafluorophenol sodium salt (76.0 mg, 0,283 mmol) and *N,N'*-dicyclohexylcarbodiimide (64.0 mg, 0.310 mmol) in $CH_3CN$ (4 mL) and DMF (1mL) was stirred at room temperature for a period of 18 h. The reaction mixture was evaporated to dryness and the residue was purified by flash column chromatography on silica gel (230-400 mesh; $CHCl_3/MeOH/H_2O$ (65:20:2) to yield sodium (E)-4-(4-(2-(7-(diethylamino)-4-methyl-coumarin-3-yl)vinyl)-benzoyl)-2,3,5,6-tetrafluorobenzenesulfonate (VI-1) (156 mg, 0.249 mmol, 94%).

$^1H$ NMR (400 MHz, DMSO-$d_6$) δ (ppm): 1.13 (6H, t, *J* = 7.0, $N(CH_2CH_3)_2$), 2.58 (3H, s, 4-$CH_3$), 3.44 (4H, q, *J* = 7.0, $N(CH_2CH_3)_2$), 6.52 (1H, d, $J_{8,6}$ = 2.3, H-8), 6.73 (1H, dd, $J_{6,8}$ = 2.3, $J_{6,5}$ = 9.2, H-6), 7.49 (1H, d, $J_{2',1'}$ = 16.0, H-2'), 7.68 (1H, d, $J_{5,6}$= 9.2, H-5), 7.77 (1H, d, $J_{1',2'}$ = 16.0, H-1'), 7.84 (2H, d, $J_{4',5'}$ = 8.4, H-4', H-8'), 8.14 (2H, d, $J_{5',4'}$ = 8.4, H-5', H-7').

$^{13}C$ NMR (100 MHz, DMSO-$d_6$) δ: 12.4 (C11, C13), 14.8 (C9), 44.1 (C10,C12), C8 (96.2), 108.9 (C3), 109.3 (C6), 113.2 (C4a), 117 (CN), 124.1 (C2'), 124.6 (C13'), 126.1 (C5), 127.0 (C5', C7'), 127.3 (C1'), 130.0 (C6'), 130.9 (C4', C8'), 139,0 (C12', C14'), 141.6 (C10'), 144.3 (C11', C15'), 145.0 (C3'), 150.6 (C4, C7), 154.5 (C8a), 159.5 (C2), 162.1 (COO).

MS-TOF(-) calc. for $C_{29}H_{22}NO_7F_4S$ $[M-H]^-$ 604.1059 found 604.1043. IV $\nu_{max}^{KBr}$ $cm^{-1}$ : 3420, 2968, 2924, 2853, 1750, 1703, 1595, 1566, 1480, 1412, 1355, 1214, 1175, 1090, 985, 822, 748, 626. UV $\lambda_{max}^{CH_3CN}$ nm : 197, 252, 434.

As can be seen in Table I, due to the electronic conjugation provided by the vinyl group at 3-position, the absorbance wavelength of the compound III-1 is above 388 nm against 371 nm of compound I-1 (figure 3). Also, absorbance of compound VI-1 has been determined to be 434 nm (figure 1).

Emission of compounds I-1, III-1 and VI-1 was also determined (figure 2) and the shift of the emission wavelength also confirms that the extension also resulted in an enhancement of the emissive properties (red-shifted), namely higher fluorescence.

Table I - Absorbance and emission wavelengths of compounds I-1, III-1 and VI-1.

| Compound | $\lambda_{abs}$ (nm) | $\lambda_{em}$ (nm) |
|---|---|---|
| I-1 | 371 | 434 |
| III-1 | 388 | 461 |
| VI-1 | 434 | 539 |

This confirms the effect of π-bridge by enhancing and allowing the delocalization to the electron acceptor.

**Example 3: Synthesis of 2-cyano-3-(4-((E)-2-(7-(diethylamino)-4-methyl-coumarin-3-yl)vinyl)phenyl)acrylic acid (compound VIII-1)**

Step 1: Preparation of *(E)*-4-(2-(7-(diethylamino)-4-methyl-coumarin-3-yl)vinyl)benzaldehyde (VII-1)

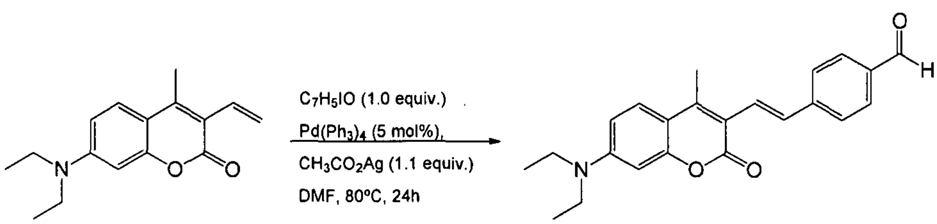


(III-1) (VII-1)

To a solution of 7-diethylamino-4-methyl-3-vinyl coumarin **(III-1)** (100 mg, 0.389 mmol), 4-Iodobenzaldehyde (82.0 mg, 0.353 mmol), $Pd(PPh_3)_4$ (20.0 mg, 0.018 mmol) and $CH_3CO_2Ag$ (65.0 mg, 0.389 mmol) in DMF (2.0 mL) were added and stirred at 80 °C, under inert atmosphere, for a period of 24 h. After cooling to room temperature, the reaction mixture was evaporated to dryness and the residue was purified by flash column chromatography on silica gel (230-400 mesh; $CH_2Cl_2$) to yield *(E)*-4-(2-(7-(diethylamino)-4-methyl-coumarin-3-yl)vinyl)benzaldehyde (VII-1) (115 mg, 0.318

mmol, 90%).

$^1$H NMR (400 MHz, $CDCl_3$) $\delta$ (ppm): 1.22 (6H, t, $J$ = 7.1, $N(CH_2CH_3)_2$), 2.54 (3H, s, 4-$CH_3$), 3.42 (4H, q, $J$ = 7.1, $N(CH_2CH_3)_2$), 6.49 (1H, d, $J_{8,6}$ = 2.5, H-8), 6.61 (1H, dd, $J_{6,8}$ = 2.5, $J_{6,5}$ = 9.3, H-6), 7.32 (1H, d, $J_{2',1'}$ = 16.1, H-2'), 7.50 (1H, d, $J_{5,6}$= 9.3, H-5), 7.65 (2H, d, $J_{4',5'}$ = 8.2, H-4', H-8'), 7.78 (1H, d, $J_{1',2'}$ = 16.1, H-1'), 7.84 (2H, d, $J_{5',4'}$ = 8.2, H-5', H-7'), 9.97 (1H, s, CHO).

$^{13}$C NMR (100 MHz, $CDCl_3$) $\delta$: 12.6 (C11, C13), 15.1 (C9), 44.9 (C10,C12), 97.3 (C8), 109.1 (C6), 109.8 (C3), 115.0 (C4a), 125.0 (C2'), 126.5 (C5), 127.0 (C5', C7'), 131.9 (C4', C8'), 131.9 (C1'), 135.2 (C6'), 144.8 (C3'), 148.9 (C7), 150.6 (C4), 154.9 (C8a), 160.8 (C2), 191.9 (CHO). IV $\nu_{max}^{KBr}$ $cm^{-1}$ : 2969, 2925, 1693, 1621, 1609, 1592, 1568, 1514, 1413, 1356, 1269, 1212, 1165, 1146, 1077, 965, 806, 766. UV $\lambda_{max}^{CH_3CN}$ nm : 203, 252, 312, 435.

Step 2: Preparation of 2-cyano-3-(4-((E)-2-(7-(diethylamino)-4-methylcoumarin-3-yl)vinyl)-phenyl)acrylic acid (VIII-1)

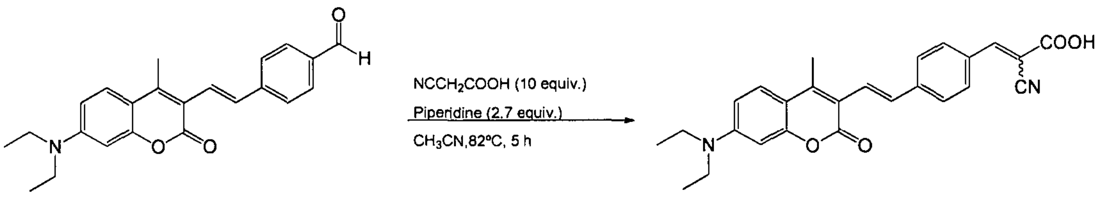


(VII-1) (VIII-1)

To a solution of *(E)*-4-(2-(7-(diethylamino)-4-methylcoumarin-3-yl)vinyl)-benzaldehyde (VII-1) (100 mg, 0.277 mmol), cyanoacetic acid (235 mg, 2.77 mmol) and piperidine (0.074 mL, 0.747 mmol) in $CH_3CN$ (12 mL) were added and stirred at 82 °C for a period of 5 h. After cooling to room temperature, the reaction mixture was evaporated to dryness and the residue was purified by flash column chromatography on silica gel (230-400 mesh; $CHCl_3$/MeOH/$H_2O$ (65:35:5) to yield 2-cyano-3-(4-((E)-2-(7-(diethylamino)-4-methyl-coumarin-3-yl)vinyl)phenyl)acrylic acid (VIII-1) (109 mg, 0.255 mmol, 92%).

$^1$H NMR (400 MHz, DMSO-$d_6$) $\delta$ (ppm): 1.13 (6H, t, $J$ = 7.0, $N(CH_2CH_3)_2$), 2.57 (3H, s, 4-$CH_3$), 3.45 (4H, q, $J$ = 7.0, $N(CH_2CH_3)_2$), 6.53 (1H, d, $J_{8,6}$ = 2.2, H-8), 6.74 (1H, dd, $J_{6,8}$ = 2.2, $J_{6,5}$ = 9.2, H-6), 7.46 (1H, d, $J_{2',1'}$ = 16.1, H-2'), 7.68 (1H, d, $J_{5,6}$= 9.2, H-5), 7.75 (1H, d, $J_{1',2'}$ = 16.1, H-1'), 7.79 (2H, d, $J_{4',5'}$ = 8.4, H-4', H-8'), 8.03 (2H, d, $J_{5',4'}$ = 8.4, H-5', H-7'), 8.25 (1H, s, H-9'), 9.97 (1H, s, COOH).

$^{13}$C NMR (100 MHz, DMSO-$d_6$) $\delta$: 12.4 (C11, C13), 14.7 (C9), 44.1 (C10,C12), 96.3 (C8), 108.9 (C6), 109.3 (C3, C4a); 113.4 (C10'),117.0 (CN), 125.3 (C2'), 127.0 (C5', C7'), 127.3 (C5), 130.3 (C1'), 130.5 (C6'), 131.2 (C4', C8'), 142.7 (C3'), 150.3 (C7), 150.5 (C4), 152.8 (C9'), 154.4 (C8a), 159.5 (C2), 162.5 (C12'). IV $\nu_{max}^{KBr}$ cm-1 : 2975, 2228, 1714, 1680, 1618, 1557, 1500, 1405, 1344, 1341, 1276, 1219, 1175, 1142, 1077, 964, 948, 824, 785, 766, 671. UV $\lambda_{max}^{CH_3CN}$ nm : 207, 256, 340, 452.

As can be seen in Table II, due to the electronic conjugation provided by the vinyl group at 3-position, the absorbance wavelength of the compound III-1 is above 388 nm against 371 nm of compound I-1 (figure 3). Also, absorbance of compound VIII-1 has been determined to be 452 nm (figure 3).

Emission of compounds I-1, III-1 and VIII-1 was also determined (figure 4) and the shift of the emission wavelength also confirms that the extension also resulted in an enhancement of the emissive properties (red-shifted), namely higher fluorescence

Table II - Absorbance and emission wavelengths of compounds I-1, III-1 and VIII-1.

| Compound | $\lambda_{abs}$ (nm) | $\lambda_{cm}$ (nm) |
|---|---|---|
| I-1 | 371 | 434 |
| III-1 | 388 | 461 |
| VIII-1 | 452 | 536 |

This confirms the effect of π-bridge by enhancing and allowing the delocalization to the electron acceptor.

**Example 4: Synthesis of 7-dimethylamino-4-methyl-3-vinyl-coumarin (III-2)**

Step 1: Preparation of 3-bromo-7-dimethylamino-4-methyl-coumarin (II-2)

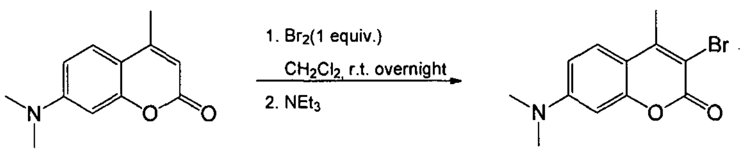


(I-2) (II-2)

To a solution of 7-dimethylamino-4-methyl-coumarin (1-2) (100 mg, 0.492 mmol) in dichloromethane (2 mL) is added bromine (0.025 mL, 0.492 mmol) in an ice bath. After stirring overnight at room temperature, $NEt_3$ (0.200 mL) is added cautiously and the mixture stirred for further 30 minutes. Then, the reaction mixture is evaporated to dryness and the residue is purified by flash column chromatography on silica gel (230-400 mesh; $CHCl_3$/MeOH (99:1)) to yield 3-bromo-7-dimethylamino-4-methyl-coumarin (II-2).

Step 2: Preparation of 7-dimethylamino-4-methyl-3-vinyl-coumarin (III-2)

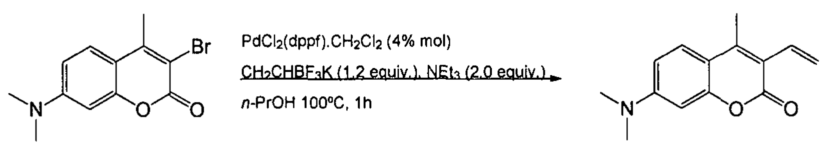


(II-2) (III-2)

To a solution of 3-bromo-7-(dimethylamino)-4-methylcoumarin (II-2) (100 mg, 0.354 mmol), potassium vinyltrifluoroborate (52 mg, 0.425 mmol), $PdCl_2$(dppf).$CH_2Cl_2$ (12 mg, 0.014 mmol), and $NEt_3$ (0.098 mL, 0.708 mmol) in *n*-PrOH (6 mL) are added and stirred at reflux under inert atmosphere for a period of 1 hour. After cooling to room temperature, the reaction mixture is evaporated to dryness and the residue is purified by flash column chromatography on silica gel (230-400 mesh; $CH_2Cl_2$) to yield 7-dimethylamino-4-methyl-3-vinyl coumarin (III-2).

**Example 5: Synthesis of sodium *(E)*-4-(4-(2-(7-(dimethylamino)-4-methyl-coumarin-3-yl)vinyl)benzoyl)-2,3,5,6-tetrafluoro-benzenesulfonate (VI-2)**

Step 1: Preparation of *(E)*-4-(2-(7-(dimethylamino)-4-methyl-coumarin-3-yl)vinyl)methylbenzoate (IV-2)

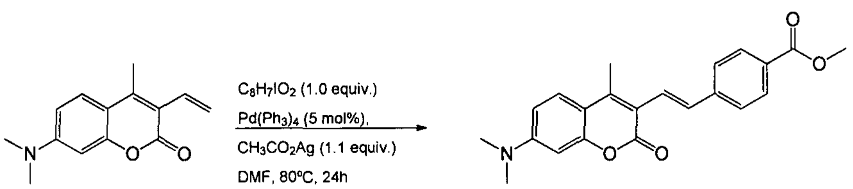


(III-2) (IV-2)

To a solution of 7-dimethylamino-4-methyl-3-vinyl coumarin (III-2) (100 mg, 0.436 mmol), methyl 4-bromobenzoate (85.2 mg, 0.396 mmol), $Pd(PPh_3)_4$ (22 mg, 0.020 mmol), and $CH_3CO_2Ag$ (72.8 mg, 0.436 mmol) in DMF (2.0 mL) are added and stirred at 80 °C, under inert atmosphere, for a period of 24 h. After cooling to room temperature, the

reaction mixture is evaporated to dryness and the residue is purified by flash column chromatography on silica gel (230-400 mesh; $CH_2Cl_2$) to yield (E)-4-(2-(7-(dimethylamino)-4-methyl-coumarin-3-yl)vinyl)methylbenzoate (IV-2).

Step 2: Preparation of *(E)*-4-(2-(7-(dimethylamino)-4-methyl-coumarin-3-yl)vinyl)benzoic acid (V-2).

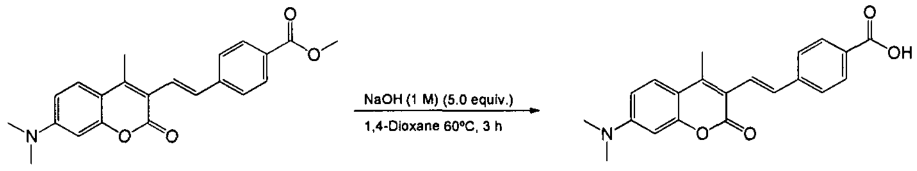


(IV-2) (V-2)

To a solution of *(E)*-methyl-4-(2-(7-(dimethylamino)-4-methyl-coumarin-3-yl)vinyl)benzoate (IV-2) (100 mg, 0.275 mmol) in 1,4-dioxane (1.3 mL) is added 1 M sodium hydroxide (1.3 mL, 1.277 mmol) and stirred at 60 °C for about 3 h. After cooling to room temperature, the reaction is neutralized with HCl (10%) solution. The reaction mixture is evaporated to dryness and the residue is purified by flash column chromatography on silica gel (230-400 mesh; $CHCl_3$/MeOH (9:1) to yield (*E*)-4-(2-(7-(dimcthylamino)-4-methyl-coumarin-3-yl)vinyl)benzoic acid (V-2).

Step 3: Preparation of *(E)*-4-(4-(2-(7-(dimethylamino)-4-methyl-coumarin-3-yl)vinyl)benzoyl)-2,3,5,6-tetrafluoro-benzenesulfonate (VI-2).

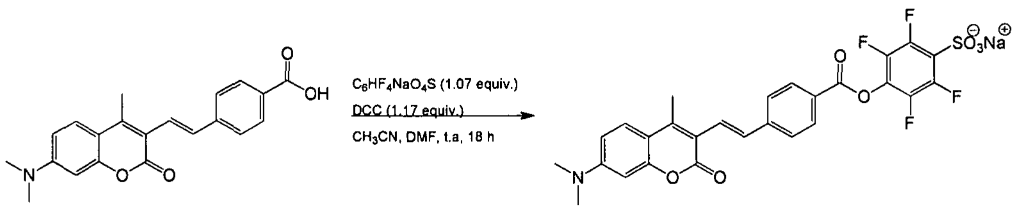


(V-2) (VI-2)

To a solution of *(E)*-4-(2-(7-(dimethylamino)-4-methyl-coumarin-3-yl)vinyl) benzoic acid (V-2) (100 mg, 0.286 mmol), 4-sulfotetrafluorophenol sodium salt (81.3 mg, 0,302mmol) and *N,N'*-dicyclohexylcarbodiimide (69.2 mg, 0.335 mmol) in $CH_3CN$ (4 mL) and DMF (1mL) are added and stirred at room temperature for a period of 18 h. The reaction mixture is evaporated to dryness and the residue is purified by flash column chromatography on silica gel (230-400 mesh; $CHCl_3$/MeOH/$H_2O$ (65:20:2) to yield sodium (*E*)-4-(4-(2-(7-(dimethylamino)-4-methyl-coumarin-3-yl)vinyl)-benzoyl)-2,3,5,6-tetrafluorobenzene-sulfonate (VI-2).

**Example 6: Synthesis of *(E)*-2,5-dioxopyrrolidin-1-yl-4-(2-(7-(dimethylamino)-4-methyl-coumarin-3-yl)vinyl)benzoate (IX-2)**

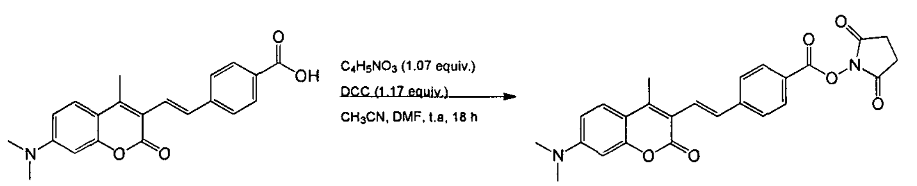


(V-2) (IX-2)

To a solution of (*E*)-4-(2-(7-(dimethylamino)-4-methyl-coumarin-3-yl)vinyl) benzoic acid (V-2) (100 mg, 0.286 mmol), *N*-hydroxysuccinimide (34.7 mg, 0,302mmol), *N,N'*-dicyclohexylcarbodiimide (69.2 mg, 0.335 mmol) in $CH_3CN$

(4 mL) and DMF (1mL) are added and stirred at room temperature for a period of 18 h. The reaction mixture is evaporated to dryness and the residue is purified by flash column chromatography on silica gel (230-400 mesh; $CHCl_3$/MeOH/$H_2O$ (65:20:2) to yield *(E)*-2,5-dioxopyrrolidin-1-yl-4-(2-(7-(dimethylamino)-4-methyl-coumarin-3-yl)vinyl)benzoate (IX-2).

**Example 7: Synthesis of sodium 4-((4-((E)-4-((E)-2-(7-(dimethylamino)-4-methyl-coumarin-3-yl)vinyl)styryl)benzoyl) oxy)-2,3,5,6-tetrafluorobenzenesulfonate (XII-2)**

Step 1: Preparation of methyl 4-((*E*)-4-((*E*)-2-(7-(dimethylamino)-4-methyl-coumarin-3-yl)vinyl)styryl)benzoate (X-2)

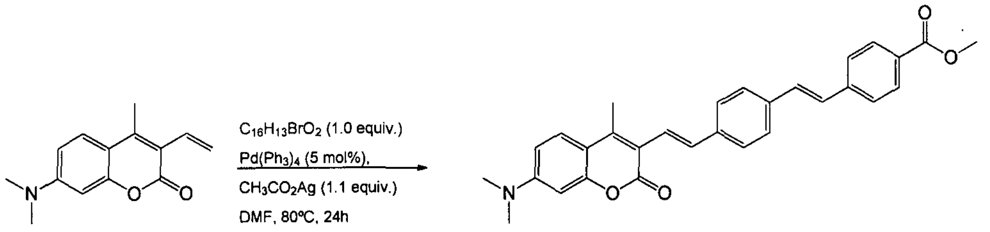


(III-2) (X-2)

To a solution of 7-dimethylamino-4-methyl-3-vinyl coumarin (III-2) (100 mg, 0.436 mmol), (E)-methyl 4-(4-bromostyryl)benzoate (125.6 mg, 0.396 mmol), $Pd(PPh_3)_4$ (22 mg, 0.020 mmol), and $CH_3CO_2Ag$ (72.8 mg, 0.436 mmol) in DMF (2.0 mL) are added and stirred at 80 °C, under inert atmosphere, for a period of 24 h. After cooling to room temperature, the reaction mixture is evaporated to dryness and the residue is purified by flash column chromatography on silica gel (230-400 mesh; $CH_2Cl_2$) to yield methyl 4-((*E*)-4-((*E*)-2-(7-(dimethylamino)-4-methyl-coumarin-3-yl)vinyl)styryl)benzoate (X-2).

Step 2: Preparation of 4-((E)-4-((E)-2-(7-(dimethylamino)-4-methyl-coumarin-3-yl)vinyl)styryl)benzoic acid (XI-2).

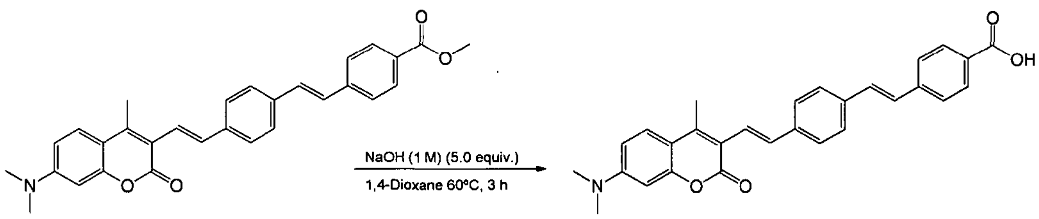


(X-2) (XI-2)

To a solution of methyl 4-((E)-4-((E)-2-(7-(dimethylamino)-4-methyl-coumarin-3-yl)vinyl)styryl)benzoate (X-2) (128 mg, 0.275 mmol) in 1,4-dioxane (1.3 mL) is added 1 M sodium hydroxide (1.3 mL, 1.277 mmol) and stirred at 60 °C for about 3 h. After cooling to room temperature, the reaction is neutralized with HCl (10%) solution. The reaction mixture is evaporated to dryness and the residue is purified by flash column chromatography on silica gel (230-400 mesh; $CHCl_3$/MeOH (9:1) to yield 4-((E)-4-((E)-2-(7-(dimethylamino)-4-methyl-coumarin-3-yl)vinyl)styryl)benzoic acid (XI-2).

Step 3: Preparation of sodium 4-((4-((E)-4-((E)-2-(7-(dimethylamino)-4-methyl-coumarin-3-yl)vinyl)styryl)benzoyl) oxy)-2,3,5,6-tetrafluorobenzenesulfonate (XII-2)

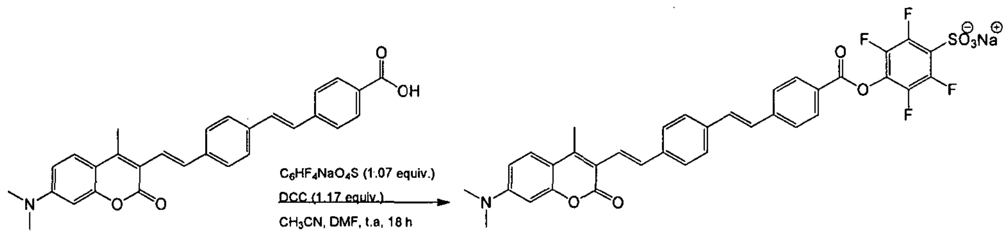


(XI-2) (XII-2)

To a solution of 4-((E)-4-((E)-2-(7-(dimethylamino)-4-methyl-coumarin-3-yl)vinyl)styryl)benzoic acid (XI-2) (129 mg, 0.286 mmol), 4-sulfotetrafluorophenol sodium salt (81.3 mg, 0,302mmol) and *N,N'*-dicyclohexylcarbodiimide (69.2 mg, 0.335 mmol) in $CH_3CN$ (4 mL) and DMF (1mL) are added and stirred at room temperature for a period of 18 h. The reaction mixture is evaporated to dryness and the residue is purified by flash column chromatography on silica gel (230-400 mesh; $CHCl_3$/MeOH/$H_2O$ (65:20:2) to yield sodium 4-((4-((E)-4-((E)-2-(7-(dimethylamino)-4-methyl-coumarin-3-yl)vinyl)styryl)benzoyl)oxy)-2,3,5,6-tetrafluorobenzenesulfonate (XII-2).

**Example 8: Synthesis of 5-dioxopyrrolidin-1-yl-4-((E)-4-((E)-2-(7-(dimethylamino)-4-methyl-coumarin-3-yl)vinyl) styryl)benzoate (XIII-2)**

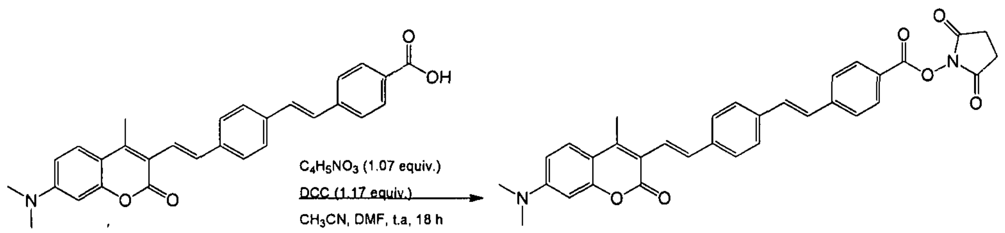


(XI-2) (XIII-2)

To a solution of 4-((E)-4-((E)-2-(7-(dimethylamino)-4-methyl-coumarin-3-yl)vinyl)styryl)benzoic acid (XI-2) (129 mg, 0.286 mmol), *N*-hydroxysuccinimide (34.7 mg, 0,302mmol) and *N,N'*-dicyclohexylcarbodiimide (69.2 mg, 0.335 mmol) in $CH_3CN$ (4 mL) and DMF (1mL) are added and stirred at room temperature for a period of 18 h. The reaction mixture is evaporated to dryness and the residue is purified by flash column chromatography on silica gel (230-400 mesh; $CHCl_3$/MeOH/$H_2O$ (65:20:2) to yield 5-dioxopyrrolidin-1-yl-4-((E)-4-((E)-2-(7-(dimethylamino)-4-methyl-coumarin-3-yl)vinyl)styryl)benzoate (XIII-2).

**Example 9: Synthesis of 2-cyano-3-(4-((*E*)-2-(7-(dimethylamino)-4-methylcoumarin-3-yl)vinyl)phenyl)acrylic acid (VIII-2)**

Step 1: Preparation of *(E)*-4-(2-(7-(dimethylamino)-4-methyl-coumarin-3-yl)vinyl)benzaldehyde (VII-2)

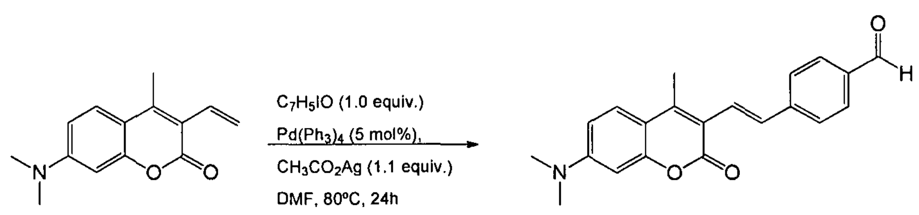


(III-2) (VII-2)

To a solution of 7-dimethylamino-4-methyl-3-vinyl coumarin (III-2) (89 mg, 0.389 mmol), 4-Iodobenzaldehyde (82.0 mg, 0.353 mmol), $Pd(PPh_3)_4$ (20.0 mg, 0.018 mmol) and $CH_3CO_2Ag$ (65.0 mg, 0.389 mmol) in DMF (2.0 mL) are added and stirred at 80 °C, under inert atmosphere, for a period of 24 h. After cooling to room temperature, the reaction

mixture is evaporated to dryness and the residue is purified by flash column chromatography on silica gel (230-400 mesh; $CH_2Cl_2$) to yield (*E*)-4-(2-(7-(dimethylamino)-4-methyl-coumarin-3-yl)vinyl)benzaldehyde (VII-2).

Step 2: Preparation of 2-cyano-3-(4-((E)-2-(7-(dimethylamino)-4-methylcoumarin-3-yl)vinyl)-phenyl)acrylic acid (VIII-2)

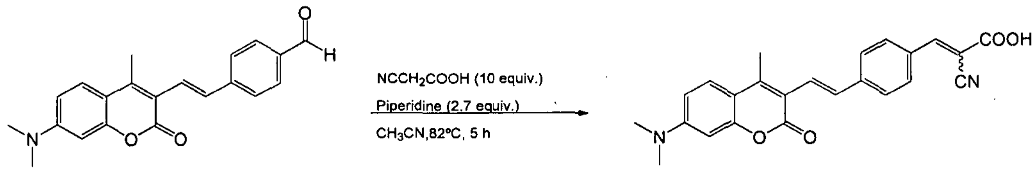


(VII-2) (VIII-2)

To a solution of (*E*)-4-(2-(7-(dimethylamino)-4-methylcoumarin-3-yl)vinyl)-benzaldehyde (VII-2) (92 mg, 0.277 mmol), cyanoacetic acid (235 mg, 2.77 mmol) and piperidine (0.074 mL, 0.747 mmol) in $CH_3CN$ (12 mL) are added and stirred at 82 °C for a period of 5 h. After cooling to room temperature, the reaction mixture is evaporated to dryness and the residue is purified by flash column chromatography on silica gel (230-400 mesh; $CHCl_3$/MeOH/$H_2O$ (65:35:5) to yield 2-cyano-3-(4-((*E*)-2-(7-(dimethylamino)-4-methyl-coumarin-3-yl)vinyl)phenyl)acrylic acid (VIII-2).

**Example 10: Synthesis of 2-cyano-3-(4-((E)-4-((E)-2-(7-(dimethylamino)-4-methyl-coumarin-3-yl)vinyl)styryl)phenyl)acrylic acid (XV-2)**

Step 1: Preparation of 4-((E)-4-((E)-2-(7-(dimethylamino)-4-methyl-coumarin-3-yl)vinyl)styryl)benzaldehyde (XIV-2)

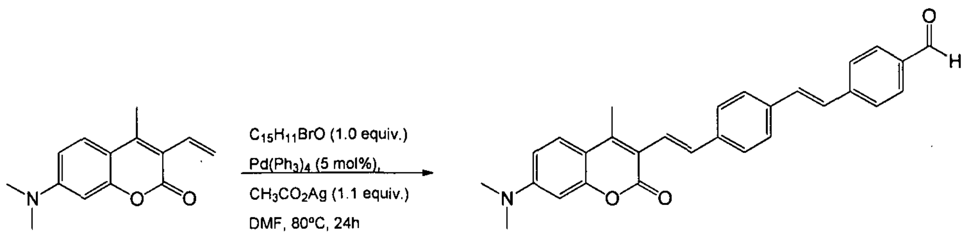


(III-2) (XIV-2)

To a solution of 7-dimethylamino-4-methyl-3-vinyl coumarin (III-2) (89 mg, 0.389 mmol), (E)-4-(4-bromostyryl)benzaldehyde (101 mg, 0.353 mmol), $Pd(PPh_3)_4$ (20.0 mg, 0.018 mmol), and $CH_3CO_2Ag$ (65.0 mg, 0.389 mmol) in DMF (2.0 mL) are added and stirred at 80 °C, under inert atmosphere, for a period of 24 h. After cooling to room temperature, the reaction mixture is evaporated to dryness and the residue is purified by flash column chromatography on silica gel (230-400 mesh; $CH_2Cl_2$) to yield (4-((E)-4-((E)-2-(7-(dimethylamino)-4-methyl-coumarin-3-yl)vinyl)styryl)benzaldehyde (XIV-2).

Step 2: Preparation of 2-cyano-3-(4-((E)-4-((E)-2-(7-(dimethylamino)-4-methyl-coumarin-3-yl)vinyl)styryl)phenyl)acrylic acid (XV-2)

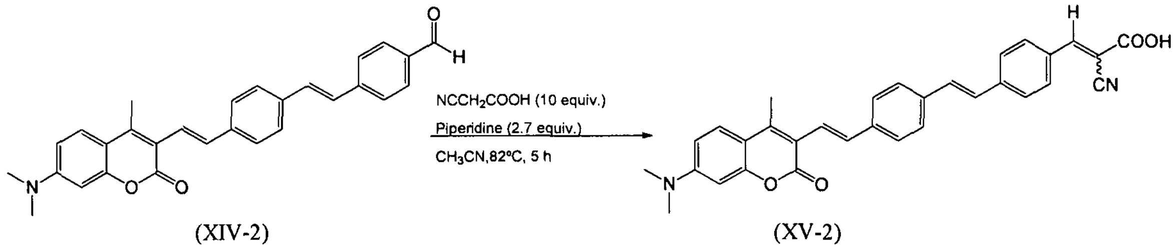


(XIV-2) (XV-2)

To a solution of (4-((E)-4-((E)-2-(7-(dimethylamino)-4-methyl-coumarin-3-yl)vinyl)styryl)benzaldehyde (XIV-2) (121 mg, 0.277 mmol), cyanoacetic acid (235 mg, 2.77 mmol) and piperidine (0.074 mL, 0.747 mmol) in $CH_3CN$ (12 mL) are added and stirred at 82 °C for a period of 5 h. After cooling to room temperature, the reaction mixture is evaporated to dryness and the residue is purified by flash column chromatography on silica gel (230-400 mesh; $CHCl_3$/MeOH/$H_2O$ (65:35:5) to yield 2-cyano-3-(4-((E)-4-((E)-2-(7-(dimethylamino)-4-methyl-coumarin-3-yl)vinyl)styryl)phenyl)acrylic acid (XV-2).

**Example 11: Preparation of 7-dimethylamino-4-ethyl-3-vinyl-coumarin (III-3)**

Step 1: Preparation of 3-bromo-7-dimethylamino-4-ethyl-coumarin (11-3)

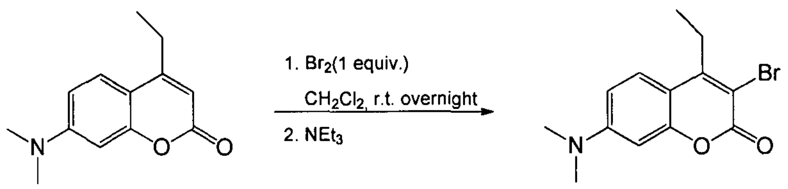


(I-3) (II-3)

To a solution of 7-dimethylamino-4-ethyl-coumarin (1-3) (107 mg, 0.492 mmol) in dichloromethane (2 mL) is added bromine (0.025 mL, 0.492 mmol) in an ice bath. After stirring overnight at room temperature, $NEt_3$ (0.200 mL) is added cautiously. and the mixture stirred for further 30 minutes. Then, the reaction mixture is evaporated to dryness and the residue is purified by flash column chromatography on silica gel (230-400 mesh; $CHCl_3$/MeOH (99:1)) to yield 3-bromo-7-dimethylamino-4-ethyl-coumarin (II-3).

Step 2: Preparation of 7-dimethylamino-4-ethyl-3-vinyl-coumarin (III-3)

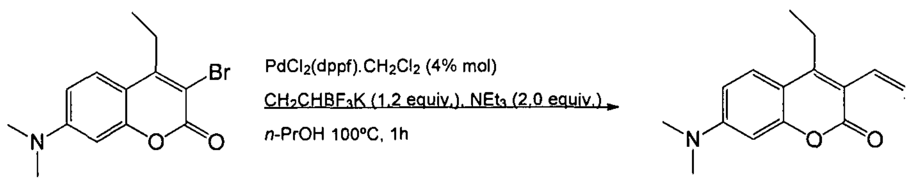


(II-3) (III-3)

To a solution of 3-bromo-7-(dimethylamino)-4-ethylcoumarin (II-3) (105 mg, 0.354 mmol), potassium vinyltrifluoroborate (52 mg, 0.425 mmol), $PdCl_2$(dppf).$CH_2Cl_2$ (12 mg, 0.014 mmol), and $NEt_3$ (0.098 mL, 0.708 mmol) in n-PrOH (6 mL) are added and stirred at reflux under inert atmosphere for a period of 1 hour. After cooling to room temperature, the reaction mixture is evaporated to dryness and the residue is purified by flash column chromatography on silica gel (230-400 mesh; $CH_2Cl_2$) to yield 7-dimethylamino-4-ethyl-3-vinyl coumarin (III-3).

**Example 12: Synthesis of *(E)*-4-(4-(2-(7-(dimethylamino)-4-ethyl-coumarin-3-yl)vinyl)benzoyl)-2,3,5,6-tetrafluoro-benzenesulfonate (VI-3)**

Step 1: Preparation of *(E)*-4-(2-(7-(dimethylamino)-4-ethyl-coumarin-3-yl)vinyl)methylbenzoate (IV-3)

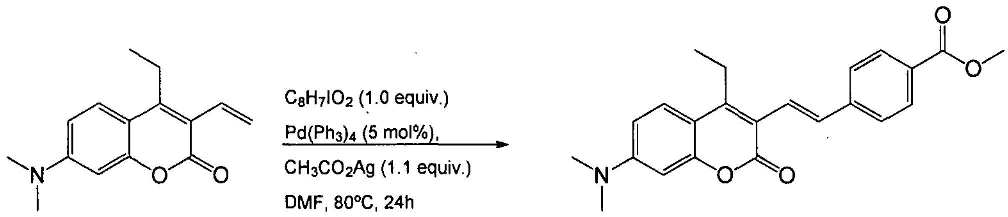


(III-3) (IV-3)

To a solution of 7-dimethylamino-4-ethyl-3-vinyl coumarin (III-3) (106 mg, 0.436 mmol), methyl 4-bromobenzoate (85.2 mg, 0.396 mmol), $Pd(PPh_3)_4$ (22 mg, 0.020 mmol), and $CH_3CO_2Ag$ (72.8 mg, 0.436 mmol) in DMF (2.0 mL) are added and stirred at 80 °C, under inert atmosphere, for a period of 24 h. After cooling to room temperature, the reaction mixture is evaporated to dryness and the residue is purified by flash column chromatography on silica gel (230-400 mesh; $CH_2Cl_2$) to yield (E)-4-(2-(7-(dimethylamino)-4-ethyl-coumarin-3-yl)vinyl)methylbenzoate (IV-3).

Step 2: Preparation of *(E)*-4-(2-(7-(dimethylamino)-4-ethyl-coumarin-3-yl)vinyl)benzoic acid (V-3)

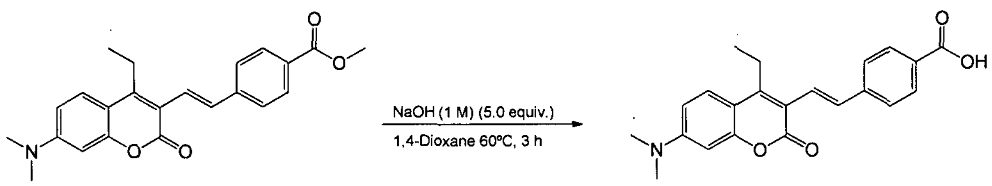


(IV-3) (V-3)

To a solution of *(E)*-methyl-4-(2-(7-(dimethylamino)-4-ethyl-coumarin-3-yl)vinyl)benzoate (IV-3) (104 mg, 0.275 mmol) in 1,4-dioxane (1.3 mL) is added 1 M sodium hydroxide (1.3 mL, 1.277 mmol) and stirred at 60 °C for about 3 h. After cooling to room temperature, the reaction is neutralized with HCl (10%) solution. The reaction mixture is evaporated to dryness and the residue is purified by flash column chromatography on silica gel (230-400 mesh; $CHCl_3$/MeOH (9:1) to yield *(E)*-4-(2-(7-(dimethylamino)-4-ethyl-coumarin-3-yl)vinyl)benzoic acid (V-3).

Step 3: Preparation of *(E)*-4-(4-(2-(7-(dimethylamino)-4-ethyl-coumarin-3-yl)vinyl)benzoyl)-2,3,5,6-tetrafluoro-benzenesulfonate (VI-3)

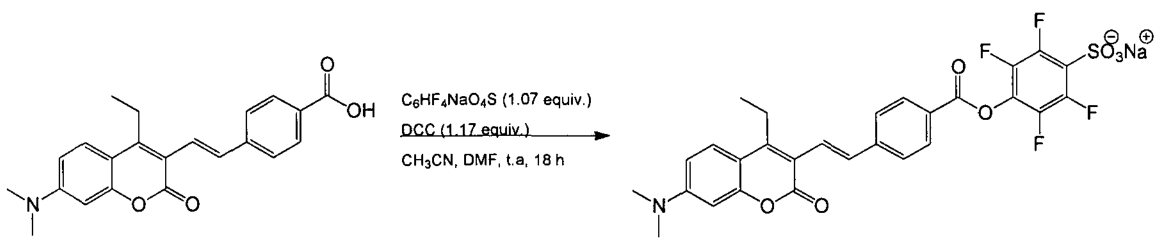


(V-3) (VI-3)

To a solution of (*E*)-4-(2-(7-(dimethylamino)-4-ethyl-coumarin-3-yl)vinyl) benzoic acid (V-3) (104 mg, 0.286 mmol), 4-sulfotetrafluorophenol sodium salt (81.3 mg, 0,302mmol) and *N,N'*-dicyclohexylcarbodiimide (69.2 mg, 0.335

mmol) in $CH_3CN$ (4 mL) and DMF (1mL) are added and stirred at room temperature for a period of 18 h. The reaction mixture is evaporated to dryness and the residue is purified by flash column chromatography on silica gel (230-400 mesh; $CHCl_3/MeOH/H_2O$ (65:20:2) to yield sodium (*E*)-4-(4-(2-(7-(dimethylamino)-4-ethyl-coumarin-3-yl)vinyl)-benzoyl)-2,3,5,6-tetrafluorobenzene-sulfonate (VI-3).

**Example 13: Synthesis of *(E)*-2,5-dioxopyrrolidin-1-yl-4-(2-(7-(dimethylamino)-4-ethyl-coumarin-3-yl)vinyl)benzoate (IX-3)**

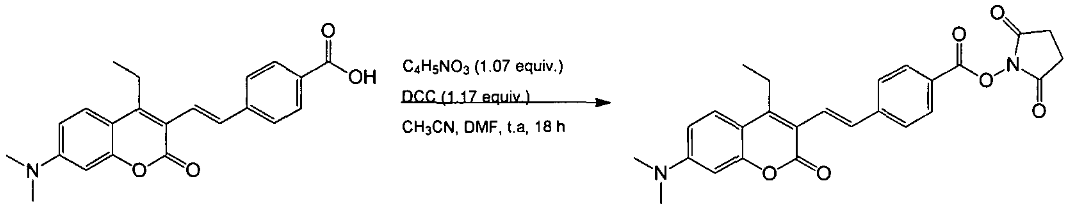


(V-3) (IX-3)

To a solution of (*E*)-4-(2-(7-(dimethylamino)-4-ethyl-coumarin-3-yl)vinyl) benzoic acid (V-3) (104 mg, 0.286 mmol), *N*-hydroxysuccinimide (34.7 mg, 0,302mmol), *N,N'*-dicyclohexylcarbodiimide (69.2 mg, 0.335 mmol) in $CH_3CN$ (4 mL) and DMF (1mL) are added and stirred at room temperature for a period of 18 h. The reaction mixture is evaporated to dryness and the residue is purified by flash column chromatography on silica gel (230-400 mesh; $CHCl_3/MeOH/H_2O$ (65:20:2) to yield *(E)*-2,5-dioxopyrrolidin-1-yl-4-(2-(7-(dimethylamino)-4-ethyl-coumarin-3-yl)vinyl)benzoate (IX-3).

**Example 14: Synthesis of sodium 4-((4-((E)-4-((E)-2-(7-(dimethylamino)-4-ethyl-coumarin-3-yl)vinyl)styryl)benzoyl)oxy)-2,3,5,6-tetrafluorobenzenesulfonate (XII-3)**

Step 1: Preparation of methyl 4-((*E*)-4-((*E*)-2-(7-(dimethylamino)-4-ethyl-coumarin-3-yl)vinyl)styryl)benzoate (X-3)

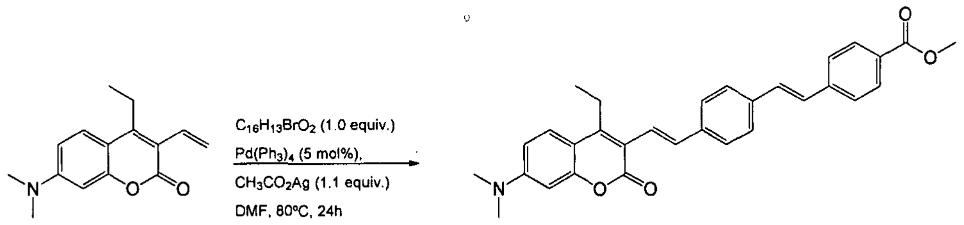


(III-3) (X-3)

To a solution of 7-dimethylamino-4-ethyl-3-vinyl coumarin (III-3) (106 mg, 0.436 mmol), (E)-methyl 4-(4-bromostyryl)benzoate (125.6 mg, 0.396 mmol), $Pd(PPh_3)_4$ (22 mg, 0.020 mmol), and $CH_3CO_2Ag$ (72.8 mg, 0.436 mmol) in DMF (2.0 mL) are added and stirred at 80 °C, under inert atmosphere, for a period of 24 h. After cooling to room temperature, the reaction mixture is evaporated to dryness and the residue is purified by flash column chromatography on silica gel (230-400 mesh; $CH_2Cl_2$) to yield methyl 4-((*E*)-4-((*E*)-2-(7-(dimethylamino)-4-ethyl-coumarin-3-yl)vinyl)styryl)benzoate (X-3).

Step 2: Preparation of 4-((E)-4-((E)-2-(7-(dimethylamino)-4-ethyl-coumarin-3-yl)vinyl)styryl)benzoic acid (XI-3)

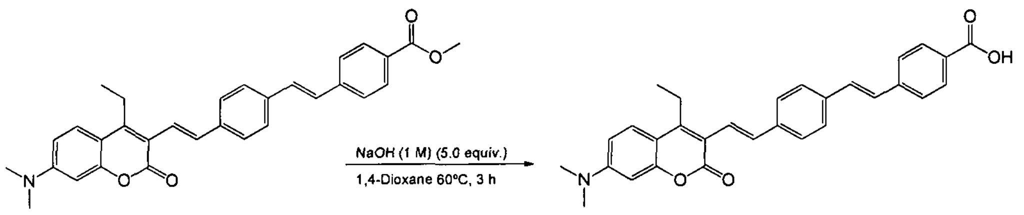


(X-3) (XI-3)

To a solution of methyl 4-((E)-4-((E)-2-(7-(dimethylamino)-4-ethyl-coumarin-3-yl)vinyl)styryl)benzoate (X-3) (132 mg, 0.275 mmol) in 1,4-dioxane (1.3 mL) is added 1 M sodium hydroxide (1.3 mL, 1.277 mmol) and stirred at 60 °C for about 3 h. After cooling to room temperature, the reaction is neutralized with HCl (10%) solution. The reaction mixture is evaporated to dryness and the residue is purified by flash column chromatography on silica gel (230-400 mesh; $CHCl_3$/MeOH (9:1) to yield 4-((E)-4-((E)-2-(7-(dimethylamino)-4-ethyl-coumarin-3-yl)vinyl)styryl)benzoic acid (XI-3).

Step 3: Preparation of sodium 4-((4-((E)-4-((E)-2-(7-(dimethylamino)-4-ethyl-coumarin-3-yl)vinyl)styryl)benzoyl)oxy)-2,3,5,6-tetrafluorobenzenesulfonate (XII-3)

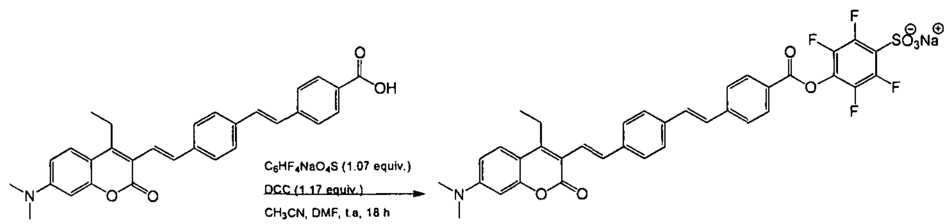


(XI-3) (XII-3)

To a solution of 4-((E)-4-((E)-2-(7-(dimethylamino)-4-ethyl-coumarin-3-yl)vinyl)styryl)benzoic acid (XI-3) (133 mg, 0.286 mmol), 4-sulfotetrafluorophenol sodium salt (81.3 mg, 0,302mmol) and *N,N'*-dicyclohexylcarbodiimide (69.2 mg, 0.335 mmol) in $CH_3CN$ (4 mL) and DMF (1mL) are added and stirred at room temperature for a period of 18 h. The reaction mixture is evaporated to dryness and the residue is purified by flash column chromatography on silica gel (230-400 mesh; $CHCl_3$/MeOH/$H_2O$ (65:20:2) to yield sodium 4-((4-((E)-4-((E)-2-(7-(dimethylamino)-4-ethyl-coumarin-3-yl)vinyl)styryl)benzoyl)oxy)-2,3,5,6-tetrafluorobenzenesulfonate (XII-3).

**Example 15: Synthesis of,5-dioxopyrrolidin-1-yl-4-((E)-4-((E)-2-(7-(dimethylamino)-4-ethyl-coumarin-3-yl)vinyl) styryl)benzoate (XIII-3)**

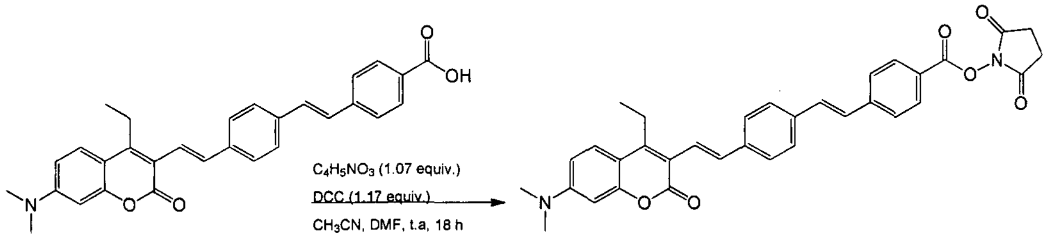


(XI-3) (XIII-3)

To a solution of 4-((E)-4-((E)-2-(7-(dimethylamino)-4-ethyl-coumarin-3-yl)vinyl)styryl)benzoic acid (XI-3) (133 mg, 0.286 mmol), N-hydroxysuccinimide (34.7 mg, 0,302mmol) and N,N'-dicyclohexylcarbodiimide (69.2 mg, 0.335 mmol) in $CH_3CN$ (4 mL) and DMF (1mL) are added and stirred at room temperature for a period of 18 h. The reaction

mixture is evaporated to dryness and the residue is purified by flash column chromatography on silica gel (230-400 mesh; $CHCl_3/MeOH/H_2O$ (65:20:2) to yield 5-dioxopyrrolidin-1-yl-4-((E)-4-((E)-2-(7-(dimethylamino)-4-ethyl-coumarin-3-yl)vinyl)styryl)benzoate (XIII-3).

**Example 16: Synthesis of 2-cyano-3-(4-((*E*)-2-(7-(dimethylamino)-4-ethyl-coumarin-3-yl)vinyl)phenyl)acrylic acid (VIII-3)**

Step 1: Preparation of *(E)*-4-(2-(7-(dimethylamino)-4-ethyl-coumarin-3-yl)vinyl)benzaldehyde (VII-3)

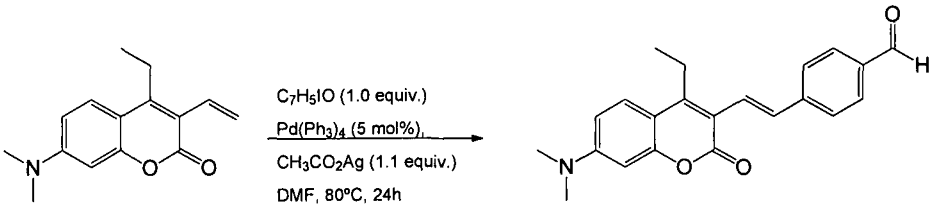


(III-3) (VII-3)

To a solution of 7-dimethylamino-4-ethyl-3-vinyl coumarin (III-3) (95 mg, 0.389 mmol), 4-lodobenzaldehyde (82.0 mg, 0.353 mmol), $Pd(PPh_3)_4$ (20.0 mg, 0.018 mmol), and $CH_3CO_2Ag$ (65.0 mg, 0.389 mmol) in DMF (2.0 mL) are added and stirred at 80 °C, under inert atmosphere, for a period of 24 h. After cooling to room temperature, the reaction mixture is evaporated to dryness and the residue is purified by flash column chromatography on silica gel (230-400 mesh; $CH_2Cl_2$) to yield (*E*)-4-(2-(7-(dimethylamino)-4-ethyl-coumarin-3-yl)vinyl)benzaldehyde (VII-3).

Step 2: Preparation of 2-cyano-3-(4-((E)-2-(7-(dimethylamino)-4-ethylcoumarin-3-yl)vinyl)- phenyl)acrylic acid (VIII-3)

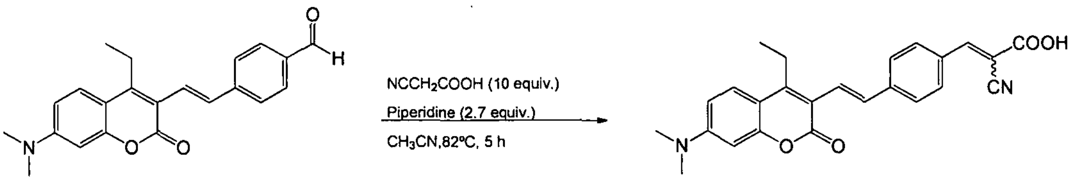


(VII-3) (VIII-3)

To a solution of (*E*)-4-(2-(7-(dimethylamino)-4-ethylcoumarin-3-yl)vinyl)-benzaldehyde (VII-3) (96 mg, 0.277 mmol), cyanoacetic acid (235 mg, 2.77 mmol) and piperidine (0.074 mL, 0.747 mmol) in $CH_3CN$ (12 mL) are added and stirred at 82 °C for a period of 5 h. After cooling to room temperature, the reaction mixture is evaporated to dryness and the residue is purified by flash column chromatography on silica gel (230-400 mesh; $CHCl_3/MeOH/H_2O$ (65:35:5) to yield 2-cyano-3-(4-((*E*)-2-(7-(dimethylamino)-4-ethyl-coumarin-3-yl)vinyl)phenyl)acrylic acid (VIII-3).

**Example 17: Synthesis of 2-cyano-3-(4-((E)-4-((E)-2-(7-(dimethylamino)-4-ethyl-coumarin-3-yl)vinyl)styryl)phenyl)acrylic acid (XV-3)**

Step 1: Preparation of 4-((E)-4-((E)-2-(7-(dimethylamino)-4-ethyl-coumarin-3-yl)vinyl)styryl)benzaldehyde (XIV-3)

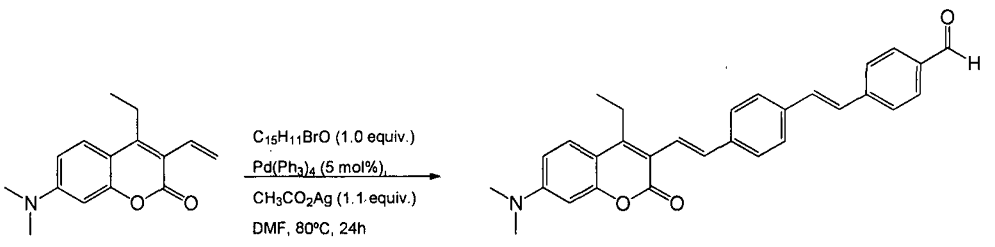


(III-3) (XIV-3)

To a solution of 7-dimethylamino-4-ethyl-3-vinyl coumarin (III-3) (95 mg, 0.389 mmol), (E)-4-(4-bromostyryl)benzaldehyde (101 mg, 0.353 mmol), $Pd(PPh_3)_4$ (20.0 mg, 0.018 mmol), and $CH_3CO_2Ag$ (65.0 mg, 0.389 mmol) in DMF (2.0 mL) are added and stirred at 80 °C, under inert atmosphere, for a period of 24 h. After cooling to room temperature, the reaction mixture is evaporated to dryness and the residue is purified by flash column chromatography on silica gel (230-400 mesh; $CH_2Cl_2$) to yield (4-((E)-4-((E)-2-(7-(dimethylamino)-4-ethyl-coumarin-3-yl)vinyl)styryl)benzaldehyde (XIV-3).

Step 2: Preparation of 2-cyano-3-(4-((E)-4-((E)-2-(7-(dimethylamino)-4-ethyl-coumarin-3-yl)vinyl)styryl)phenyl)acrylic acid (XV-3)

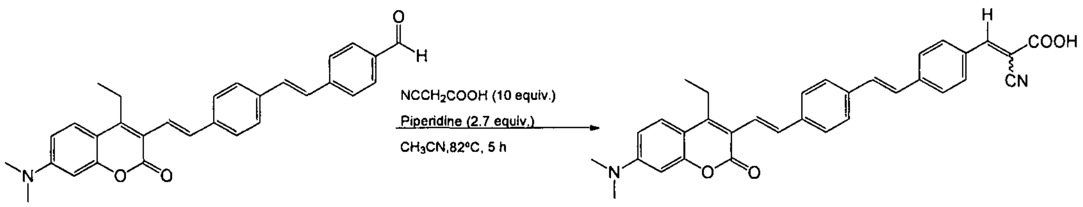


(XIV-3) (XV-3)

To a solution of (4-((E)-4-((E)-2-(7-(dimethylamino)-4-ethyl-coumarin-3-yl)vinyl)styryl)benzaldehyde (XIV-3) (125 mg, 0.277 mmol), cyanoacetic acid (235 mg, 2.77 mmol) and piperidine (0.074 mL, 0.747 mmol) in $CH_3CN$ (12 mL) are added and stirred at 82 °C for a period of 5 h. After cooling to room temperature, the reaction mixture is evaporated to dryness and the residue is purified by flash column chromatography on silica gel (230-400 mesh; $CHCl_3/MeOH/H_2O$ (65:35:5) to yield 2-cyano-3-(4-((E)-4-((E)-2-(7-(dimethylamino)-4-ethyl-coumarin-3-yl)vinyl)styryl)phenyl)acrylic acid (XV-3).

**Example 18: Synthesis of 7-dipropylamino-4-methyl-3-vinyl coumarin (III-4)**

Step 1: Preparation of3-bromo-7-dipropylamino-4-methyl-coumarin (II-4)

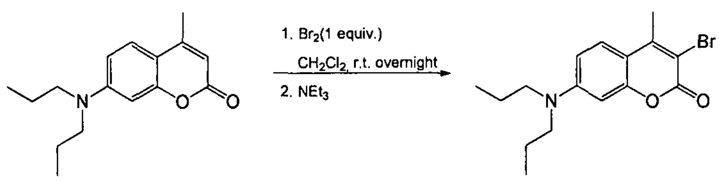


(I-4) (II-4)

To a solution of 7-dipropylamino-4-methyl-coumarin (1-4) (128 mg, 0.492 mmol) in dichloromethane (2 mL) is added bromine (0.025 mL, 0.492 mmol) in an ice bath. After stirring overnight at room temperature, $NEt_3$ (0.200 mL) is added cautiously and the mixture stirred for further 30 minutes. Then, the reaction mixture is evaporated to dryness and the residue is purified by flash column chromatography on silica gel (230-400 mesh; $CHCl_3/MeOH$ (99:1)) to yield 3-bromo-7-dipropylamino-4-methyl-coumarin (II-4).

Step 2: Preparation of 7-dipropylamino-4-methyl-3-vinyl-coumarin (III-4)

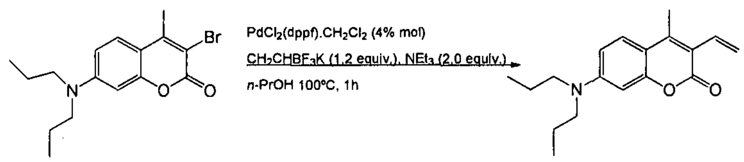


(II-4) (III-4)

To a solution of 3-bromo-7-(dipropylamino)-4-methylcoumarin (II-4) (120 mg, 0.354 mmol), potassium vinyltrifluoroborate (52 mg, 0.425 mmol), $PdCl_2(dppf).CH_2Cl_2$ (12 mg, 0.014 mmol), and $NEt_3$ (0.098 mL, 0.708 mmol) in *n*-PrOH (6 mL) are added and stirred at reflux under inert atmosphere for a period of 1 hour. After cooling to room temperature, the reaction mixture is evaporated to dryness and the residue is purified by flash column chromatography on silica gel (230-400 mesh; $CH_2Cl_2$) to yield 7-dipropylamino-4-methyl-3-vinyl coumarin (III-4).

**Example 19: Synthesis of sodium (E)-4-(4-(2-(7-(dipropylamino)-4-methyl-coumarin-3-yl)vinyl)-benzoyl)-2,3,5,6-tetrafluorobenzene-sulfonate (VI-4)**

Step 1: Preparation of *(E)*-4-(2-(7-(dipropylamino)-4-methyl-coumarin-3-yl)vinyl)methylbenzoate (IV-4)

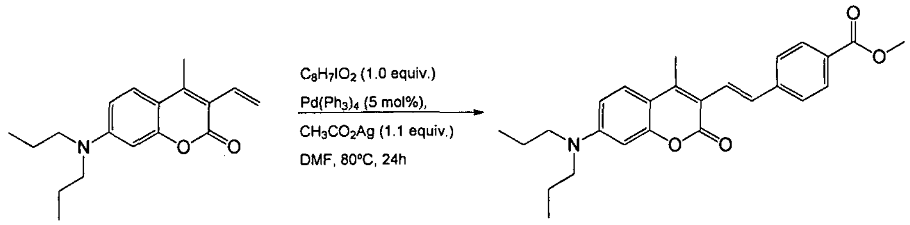


(III-4) (IV-4)

To a solution of 7-dipropylamino-4-methyl-3-vinyl coumarin (III-4) (124 mg, 0.436 mmol), methyl 4-bromobenzoate (85.2 mg, 0.396 mmol), $Pd(PPh_3)_4$ (22 mg, 0.020 mmol), and $CH_3CO_2Ag$ (72.8 mg, 0.436 mmol) in DMF (2.0 mL) are added and stirred at 80 °C, under inert atmosphere, for a period of 24 h. After cooling to room temperature, the reaction mixture is evaporated to dryness and the residue is purified by flash column chromatography on silica gel (230-400 mesh; $CH_2Cl_2$) to yield (E)-4-(2-(7-(dipropylamino)-4-methyl-coumarin-3-yl)vinyl)methylbenzoate (IV-4).

Step 2: Preparation of *(E)*-4-(2-(7-(dipropylamino)-4-methyl-coumarin-3-yl)vinyl)benzoic acid (V-4)

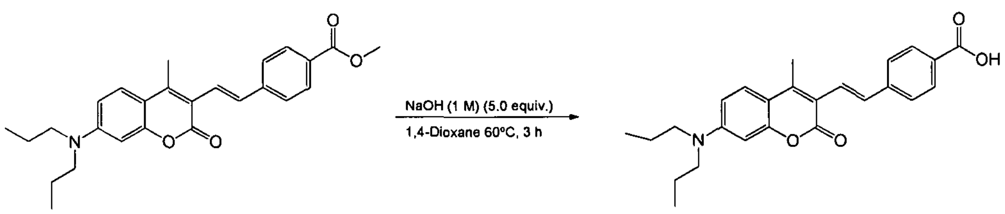


(IV-4) (V-4)

To a solution of *(E)*-methyl-4-(2-(7-(dipropylamino)-4-methyl-coumarin-3-yl)vinyl)benzoate (IV-4) (115 mg, 0.275 mmol) in 1,4-dioxane (1.3 mL) is added 1 M sodium hydroxide (1.3 mL, 1.277 mmol) and stirred at 60 °C for about 3 h. After cooling to room temperature, the reaction is neutralized with HCl (10%) solution. The reaction mixture is evaporated to dryness and the residue is purified by flash column chromatography on silica gel (230-400 mesh; $CHCl_3$/Me-

OH (9:1) to yield ($E$)-4-(2-(7-(dipropylamino)-4-methyl-coumarin-3-yl)vinyl)benzoic acid (V-4).

Step 3: Preparation of *(E)*-4-(4-(2-(7-(dipropylamino)-4-methyl-coumarin-3-yl)vinyl)benzoyl)-2,3,5,6-tetrafluoro-benzenesulfonate (VI-4)

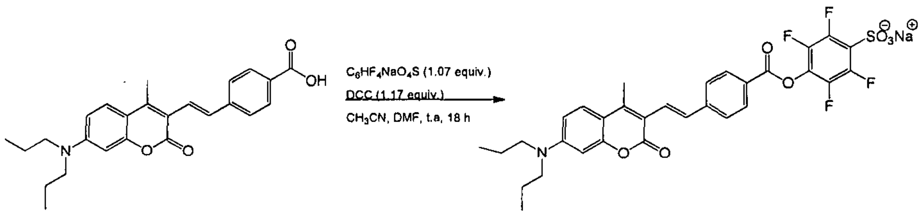


(V-4) (VI-4)

To a solution of ($E$)-4-(2-(7-(dipropylamino)-4-methyl-coumarin-3-yl)vinyl) benzoic acid (V-4) (116 mg, 0.286 mmol), 4-sulfotetrafluorophenol sodium salt (81.3 mg, 0,302mmol) and *N,N'*-dicyclohexylcarbodiimide (69.2 mg, 0.335 mmol) in $CH_3CN$ (4 mL) and DMF (1mL) are added and stirred at room temperature for a period of 18 h. The reaction mixture is evaporated to dryness and the residue is purified by flash column chromatography on silica gel (230-400 mesh; $CHCl_3/MeOH/H_2O$ (65:20:2) to yield sodium ($E$)-4-(4-(2-(7-(dipropylamino)-4-methyl-coumarin-3-yl)vinyl)-benzoyl)-2,3,5,6-tetrafluorobenzene-sulfonate (VI-4).

Example 20: **Synthesis of Preparation of *(E)*-2,5-dioxopyrrolidin-1-yl-4-(2-(7-(dipropylamino)-4-methyl-coumarin-3-yl)vinyl)benzoate (IX-4)**

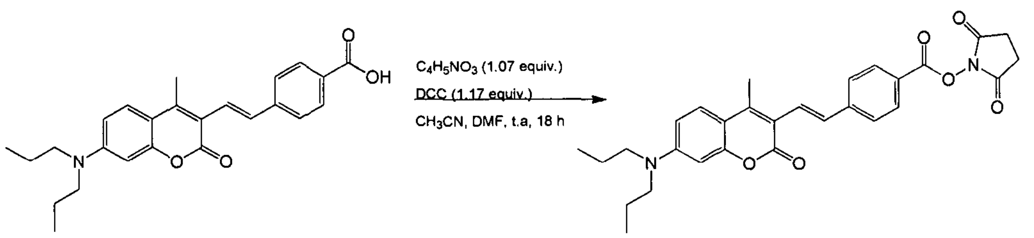


(V-4) (IX-4)

To a solution of (E)-4-(2-(7-(dipropylamino)-4-methyl-coumarin-3-yl)vinyl) benzoic acid (V-4) (116 mg, 0.286 mmol), N-hydroxysuccinimide (34.7 mg, 0,302mmol), N,N'-dicyclohexylcarbodiimide (69.2 mg, 0.335 mmol) in $CH_3CN$ (4 mL) and DMF (1mL) are added stirred at room temperature for a period of 18 h. The reaction mixture is evaporated to dryness and the residue is purified by flash column chromatography on silica gel (230-400 mesh; $CHCl_3/MeOH/H_2O$ (65:20:2) to yield (E)-2,5-dioxopyrrolidin-1-yl-4-(2-(7-(dipropylamino)-4-methyl-coumarin-3-yl)vinyl)benzoate (IX-4).

**Example 21: Synthesis of sodium 4-((4-((E)-4-((E)-2-(7-(dipropylamino)-4-methyl-coumarin-3-yl)vinyl)styryl)benzoyl)oxy)-2,3,5,6-tetrafluorobenzenesulfonate (XII-4)**

Step 1: Preparation of methyl 4-(($E$)-4-(($E$)-2-(7-(dipropylamino)-4-methyl-coumarin-3-yl)vinyl)styryl)benzoate (X-4)

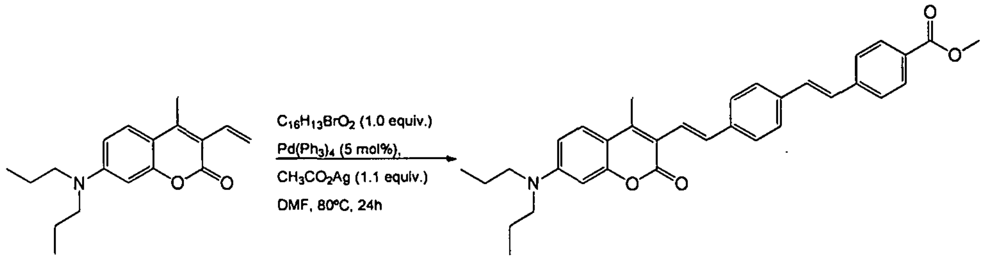


(III-4) (X-4)

To a solution of 7-dipropylamino-4-methyl-3-vinyl coumarin (III-4) (124 mg, 0.436 mmol), (E)-methyl 4-(4-bromostyryl)benzoate (125.6 mg, 0.396 mmol), $Pd(PPh_3)_4$ (22 mg, 0.020 mmol), and $CH_3CO_2Ag$ (72.8 mg, 0.436 mmol) in DMF (2.0 mL) are added and stirred at 80 °C, under inert atmosphere, for a period of 24 h. After cooling to room temperature, the reaction mixture is evaporated to dryness and the residue is purified by flash column chromatography on silica gel (230-400 mesh; $CH_2Cl_2$) to yield methyl 4-((*E*)-4-((*E*)-2-(7-(dipropylamino)-4-methyl-coumarin-3-yl)vinyl)styryl)benzoate (X-4).

Step 2: Preparation of 4-((E)-4-((E)-2-(7-(dipropylamino)-4-methyl-coumarin-3-yl)vinyl)styryl)benzoic acid (XI-4)

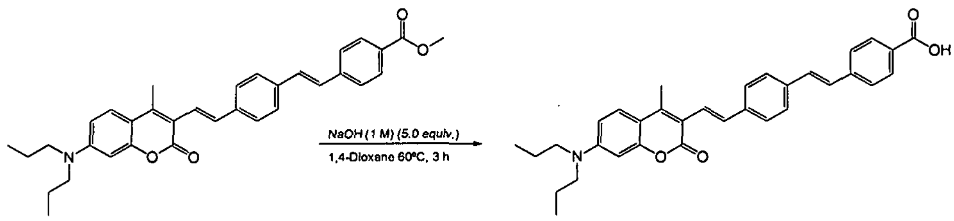


(X-4) (XI-4)

To a solution of methyl 4-((E)-4-((E)-2-(7-(dipropylamino)-4-methyl-coumarin-3-yl)vinyl)styryl)benzoate (X-4) (143 mg, 0.275 mmol) in 1,4-dioxane (1.3 mL) is added 1 M sodium hydroxide (1.3 mL, 1.277 mmol) and stirred at 60 °C for about 3 h. After cooling to room temperature, the reaction is neutralized with HCl (10%) solution. The reaction mixture is evaporated to dryness and the residue is purified by flash column chromatography on silica gel (230-400 mesh; $CHCl_3$/MeOH (9:1) to yield 4-((E)-4-((E)-2-(7-(dipropylamino)-4-methyl-coumarin-3-yl)vinyl)styryl)benzoic acid (XI-4).

Step 3: Preparation of sodium 4-((4-((E)-4-((E)-2-(7-(dipropylamino)-4-methyl-coumarin-3-yl)vinyl)styryl)benzoyl)oxy)-2,3,5,6-tetrafluorobenzenesulfonate (XII-4)

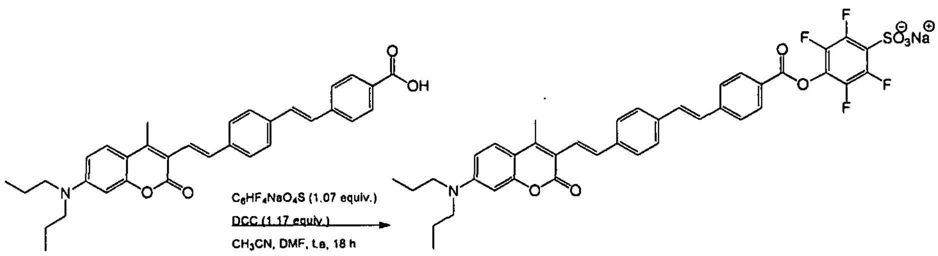


(XI-4) (XII-4)

To a solution of 4-((E)-4-((E)-2-(7-(dipropylamino)-4-methyl-coumarin-3-yl)vinyl)styryl)benzoic acid (XI-4) (145 mg, 0.286 mmol), 4-sulfotetrafluorophenol sodium salt (81.3 mg, 0,302mmol) and *N,N'*-dicyclohexylcarbodiimide (69.2 mg, 0.335 mmol) in $CH_3CN$ (4 mL) and DMF (1mL) are added and stirred at room temperature for a period of 18 h. The

reaction mixture is evaporated to dryness and the residue is purified by flash column chromatography on silica gel (230-400 mesh; $CHCl_3/MeOH/H_2O$ (65:20:2) to yield sodium 4-((4-((E)-4-((E)-2-(7-(dipropylamino)-4-methyl-coumarin-3-yl)vinyl)styryl)benzoyl)oxy)-2,3,5,6-tetrafluorobenzenesulfonate (XII-4).

**Example 22: Synthesis of 5-dioxopyrrolidin-1-yl-4-((E)-4-((E)-2-(7-(dipropylamino)-4-methyl-coumarin-3-yl)vinyl)styryl)benzoate (XIII-4)**

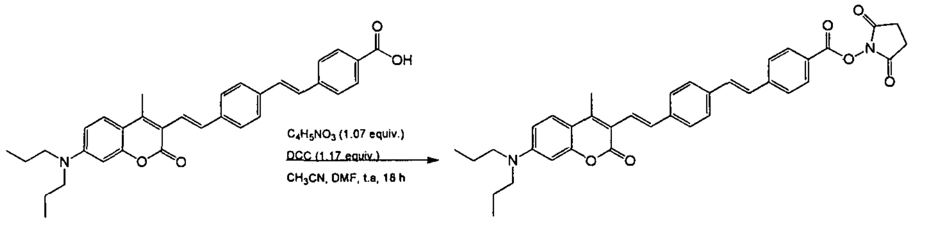


(XI-4) (XIII-4)

To a solution of 4-((E)-4-((E)-2-(7-(dipropylamino)-4-methyl-coumarin-3-yl)vinyl)styryl)benzoic acid (XI-4) (145 mg, 0.286 mmol), *N*-hydroxysuccinimide (34.7 mg, 0,302mmol) and *N,N'*-dicyclohexylcarbodiimide (69.2 mg, 0.335 mmol) in $CH_3CN$ (4 mL) and DMF (1mL) are added and stirred at room temperature for a period of 18 h. The reaction mixture is evaporated to dryness and the residue is purified by flash column chromatography on silica gel (230-400 mesh; $CHCl_3/MeOH/H_2O$ (65:20:2) to yield 5-dioxopyrrolidin-1-yl-4-((E)-4-((E)-2-(7-(dipropylamino)-4-methyl-coumarin-3-yl)vinyl)styryl)benzoate (XIII-4).

**Example 23: Synthesis of 2-cyano-3-(4-(*(E)*-2-(7-(dipropylamino)-4-methylcoumarin-3-yl)vinyl)phenyl)acrylic acid** (VIII-4)

Step 1: Preparation of*(E)*-4-(2-(7-(dipropylamino)-4-methyl-coumarin-3-yl)vinyl)benzaldehyde (VII-4)

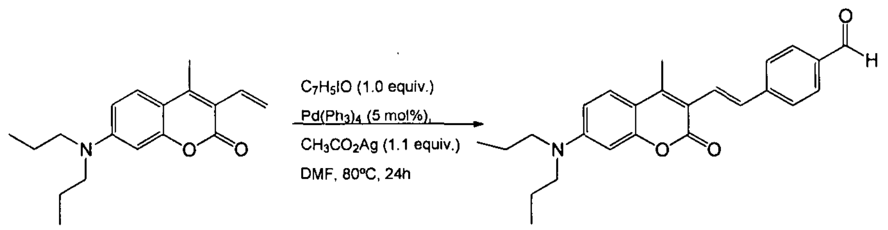


(III-4) (VII-4)

To a solution of 7-dipropylamino-4-methyl-3-vinyl coumarin (III-4) (111 mg, 0.389 mmol), 4-Iodobenzaldehyde (82.0 mg, 0.353 mmol), $Pd(PPh_3)_4$ (20.0 mg, 0.018 mmol), and $CH_3CO_2Ag$ (65.0 mg, 0.389 mmol) in DMF (2.0 mL) are added and stirred at 80 °C, under inert atmosphere, for a period of 24 h. After cooling to room temperature, the reaction mixture is evaporated to dryness and the residue is purified by flash column chromatography on silica gel (230-400 mesh; $CH_2Cl_2$) to yield (E)-4-(2-(7-(dipropylamino)-4-methyl-coumarin-3-yl)vinyl)benzaldehyde (VII-4).

Step 2: Preparation of 2-cyano-3-(4-((E)-2-(7-(dipropylamino)-4-methylcoumarin-3-yl)vinyl)- phenyl)acrylic acid (VIII-4)

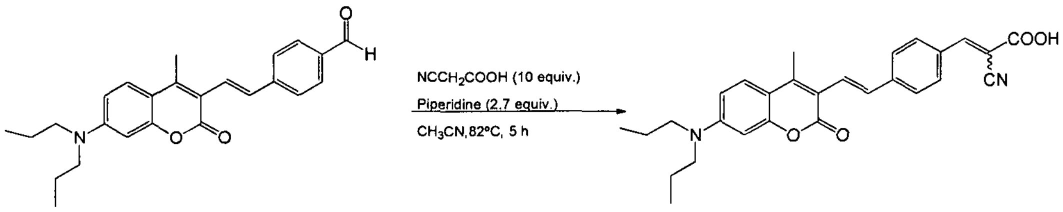


(VII-4) (VIII-4)

To a solution of (*E*)-4-(2-(7-(dipropylamino)-4-methylcoumarin-3-yl)vinyl)-benzaldehyde (VII-4) (108 mg, 0.277 mmol), cyanoacetic acid (235 mg, 2.77 mmol) and piperidine (0.074 mL, 0.747 mmol) in $CH_3CN$ (12 mL) are added and stirred at 82 °C for a period of 5 h. After cooling to room temperature, the reaction mixture is evaporated to dryness and the residue is purified by flash column chromatography on silica gel (230-400 mesh; $CHCl_3$/MeOH/$H_2O$ (65:35:5) to yield 2-cyano-3-(4-((*E*)-2-(7-(dipropylamino)-4-methyl-coumarin-3-yl)vinyl)phenyl)acrylic acid (VIII-4).

**Example 24: Synthesis of 2-cyano-3-(4-((E)-4-((E)-2-(7-(dipropylamino)-4-methyl-coumarin-3-yl)vinyl)styryl)phenyl)acrylic acid (XV-4)**

Step 1: Preparation of 4-((E)-4-((E)-2-(7-(dipropylamino)-4-methyl-coumarin-3-yl)vinyl)styryl)benzaldehyde (XIV-4)

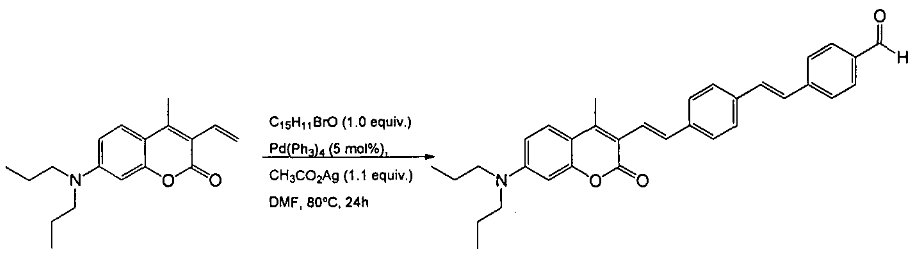


(III-4) (XIV-4)

To a solution of 7-dipropylamino-4-methyl-3-vinyl coumarin (III-4) (111 mg, 0.389 mmol), (E)-4-(4-bromostyryl)benzaldehyde (101 mg, 0.353 mmol), $Pd(PPh_3)_4$ (20.0 mg, 0.018 mmol), and $CH_3CO_2Ag$ (65.0 mg, 0.389 mmol) in DMF (2.0 mL) are added and stirred at 80 °C, under inert atmosphere, for a period of 24 h. After cooling to room temperature, the reaction mixture is evaporated to dryness and the residue is purified by flash column chromatography on silica gel (230-400 mesh; $CH_2Cl_2$) to yield (4-((E)-4-((E)-2-(7-(dipropylamino)-4-methyl-coumarin-3-yl)vinyl)styryl)benzaldehyde (XIV-4).

Step 2: Preparation of 2-cyano-3-(4-((E)-4-((E)-2-(7-(dipropylamino)-4-methyl-coumarin-3-yl)vinyl)styryl)phenyl)acrylic acid (XV-4)

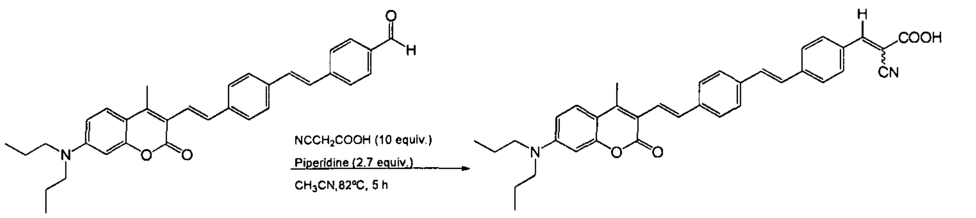


(XIV-4) (XV-4)

To a solution of (4-((E)-4-((E)-2-(7-(dipropylamino)-4-methyl-coumarin-3-yl)vinyl)styryl)benzaldehyde(XIV-4) (136 mg, 0.277 mmol), cyanoacetic acid (235 mg, 2.77 mmol) and piperidine (0.074 mL, 0.747 mmol) in $CH_3CN$ (12

mL) are added and stirred at 82 °C for a period of 5 h. After cooling to room temperature, the reaction mixture is evaporated to dryness and the residue is purified by flash column chromatography on silica gel (230-400 mesh; $CHCl_3$/MeOH/$H_2O$ (65:35:5) to yield 2-cyano-3-(4-((E)-4-((E)-2-(7-(dipropylamino)-4-methyl-coumarin-3-yl)vinyl)styryl)phenyl)acrylic acid (XV-4).

**Example 25: Synthesis of 7-dipropylamino-4-ethyl-3-vinyl-coumarin (III-5)**

Step 1: Preparation of 3-bromo-7-dipropylamino-4-ethyl-coumarin (II-5)

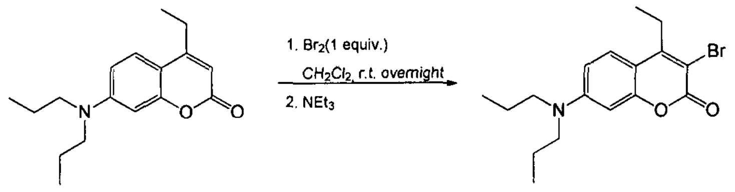


(I-5) (II-5)

To a solution of 7-dipropylamino-4-ethyl-coumarin (1-5) (134 mg, 0.492 mmol) in dichloromethane (2 mL) is added bromine (0.025 mL, 0.492 mmol) in an ice bath. After stirring overnight at room temperature, $NEt_3$ (0.200 mL) is added cautiously and the mixture stirred for further 30 minutes. Then, the reaction mixture is evaporated to dryness and the residue is purified by flash column chromatography on silica gel (230-400 mesh; $CHCl_3$/MeOH (99:1)) to yield 3-bromo-7-dipropylamino-4-ethyl-coumarin (II-5).

Step 2: Preparation of 7-dipropylamino-4-ethyl-3-vinyl-coumarin (III-5)

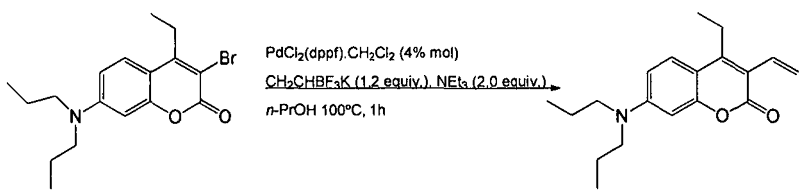


(II-5) (III-5)

To a solution of 3-bromo-7-(dipropylamino)-4-ethylcoumarin (II-5) (125 mg, 0.354 mmol), potassium vinyltrifluoroborate (52 mg, 0.425 mmol), $PdCl_2$(dppf).$CH_2Cl_2$ (12 mg, 0.014 mmol), and $NEt_3$ (0.098 mL, 0.708 mmol) in *n*-PrOH (6 mL) are added and stirred at reflux under inert atmosphere for a period of 1 hour. After cooling to room temperature, the reaction mixture is evaporated to dryness and the residue is purified by flash column chromatography on silica gel (230-400 mesh; $CH_2Cl_2$) to yield 7-dipropylamino-4-ethyl-3-vinyl coumarin (III-5).

**Example 26: Synthesis of *(E)*-4-(4-(2-(7-(dipropylamino)-4-ethyl-coumarin-3-yl)vinyl)benzoyl)-2,3,5,6-tetrafluoro-benzenesulfonate (VI-5)**

Step 1: Preparation of *(E)*-4-(2-(7-(dipropylamino)-4-ethyl-coumarin-3-yl)vinyl)methylbenzoate (IV-5)

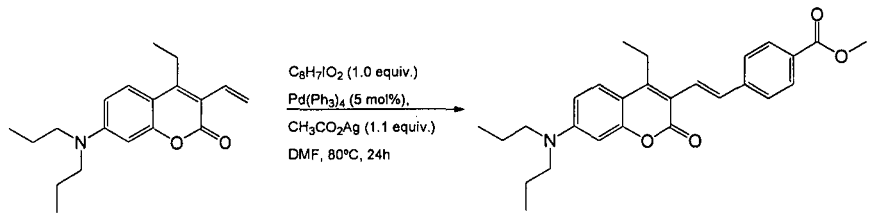


(III-5) (IV-5)

To a solution of 7-dipropylamino-4-ethyl-3-vinyl coumarin (III-5) (130 mg, 0.436 mmol), methyl 4-bromobenzoate (85.2 mg, 0.396 mmol), $Pd(PPh_3)_4$ (22 mg, 0.020 mmol), and $CH_3CO_2Ag$ (72.8 mg, 0.436 mmol) in DMF (2.0 mL) are added and stirred at 80 °C, under inert atmosphere, for a period of 24 h. After cooling to room temperature, the reaction mixture is evaporated to dryness and the residue is purified by flash column chromatography on silica gel (230-400 mesh; $CH_2Cl_2$) to yield (E)-4-(2-(7-(dipropylamino)-4-ethyl-coumarin-3-yl)vinyl)methylbenzoate (IV-5).

Step 2: Preparation of *(E)*-4-(2-(7-(dipropylamino)-4-ethyl-coumarin-3-yl)vinyl)benzoic acid (V-5)

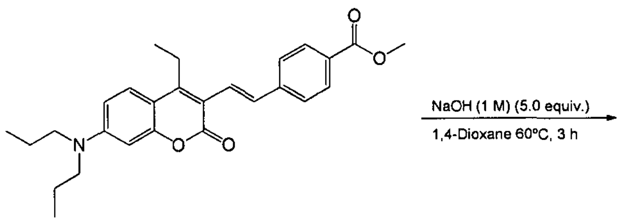


(IV-5) (V-5)

To a solution of *(E)*-methyl-4-(2-(7-(dipropylarnino)-4-ethyl-coumarin-3-yl)vinyl)benzoate (IV-5) (119 mg, 0.275 mmol) in 1,4-dioxane (1.3 mL) is added 1 M sodium hydroxide (1.3 mL, 1.277 mmol) and stirred at 60 °C for about 3 h. After cooling to room temperature, the reaction is neutralized with HCl (10%) solution. The reaction mixture is evaporated to dryness and the residue is purified by flash column chromatography on silica gel (230-400 mesh; $CHCl_3$/MeOH (9:1) to yield (*E*)-4-(2-(7-(dipropylamino)-4-ethyl-coumarin-3-yl)vinyl)benzoic acid (V-5).

Step 3: Preparation of *(E)*-4-(4-(2-(7-(dipropylamino)-4-ethyl-coumarin-3-yl)vinyl)benzoyl)-2,3,5,6-tetrafluoro-benzenesulfonate (VI-5)

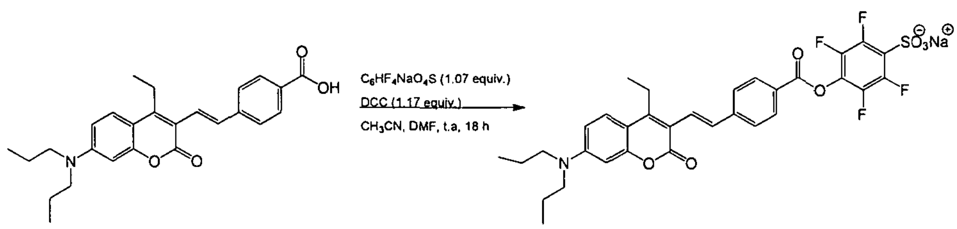


(V-5) (VI-5)

To a solution of (*E*)-4-(2-(7-(dipropylamino)-4-ethyl-coumarin-3-yl)vinyl) benzoic acid (V-5) (120 mg, 0.286 mmol), 4-sulfotetrafluorophenol sodium salt (81.3 mg, 0,302mmol) and *N,N'*-dicyclohexylcarbodiimide (69.2 mg, 0.335 mmol) in $CH_3CN$ (4 mL) and DMF (1mL) are added and stirred at room temperature for a period of 18 h. The reaction mixture is evaporated to dryness and the residue is purified by flash column chromatography on silica gel (230-400 mesh; $CHCl_3$/MeOH/$H_2O$ (65:20:2) to yield sodium (*E*)-4-(4-(2-(7-(dipropylamino)-4-ethyl-coumarin-3-yl)vinyl)-benzoyl)-2,3,5,6-tetrafluorobenzene-sulfonate (VI-5).

**Example 27: Synthesis of *(E)*-2,S-dioxopyrrolidin-1-yl-4-(2-(7-(dipropylamino)-4-ethyl-coumarin-3-yl)vinyl)benzoate (IX-5)**

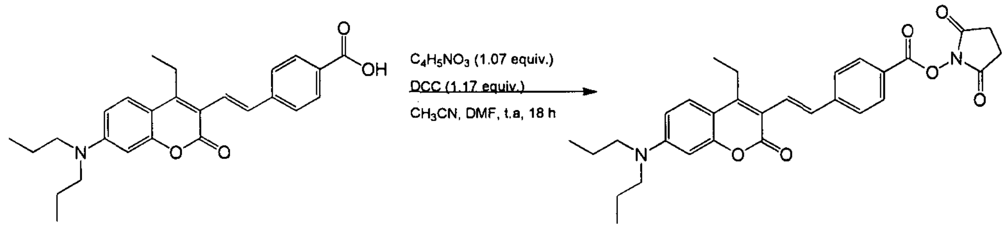


(V-5) (IX-5)

To a solution of (*E*)-4-(2-(7-(dipropylamino)-4-ethyl-coumarin-3-yl)vinyl) benzoic acid (V-5) (120 mg, 0.286 mmol), *N*-hydroxysuccinimide (34.7 mg, 0,302mmol), *N,N'*-dicyclohexylcarbodiimide (69.2 mg, 0.335 mmol) in $CH_3CN$ (4 mL) and DMF (1mL) are added and stirred at room temperature for a period of 18 h. The reaction mixture is evaporated to dryness and the residue is purified by flash column chromatography on silica gel (230-400 mesh; $CHCl_3/MeOH/H_2O$ (65:20:2) to yield *(E)*-2,5-dioxopyrrolidin-1-yl-4-(2-(7-(dipropylamino)-4-ethyl-coumarin-3-yl)vinyl)benzoate (IX-5).

**Example 28: Synthesis of sodium 4-((4-((E)-4-((E)-2-(7-(dipropylamino)-4-ethyl-coumarin-3-yl)vinyl)styryl)benzoyl)oxy)-2,3,5,6-tetrafluorobenzenesulfonate (XII-5)**

Step 1: Preparation of methyl 4-((*E*)-4-((*E*)-2-(7-(dipropylamino)-4-ethyl-coumarin-3-yl)vinyl)styryl)benzoate (X-5)

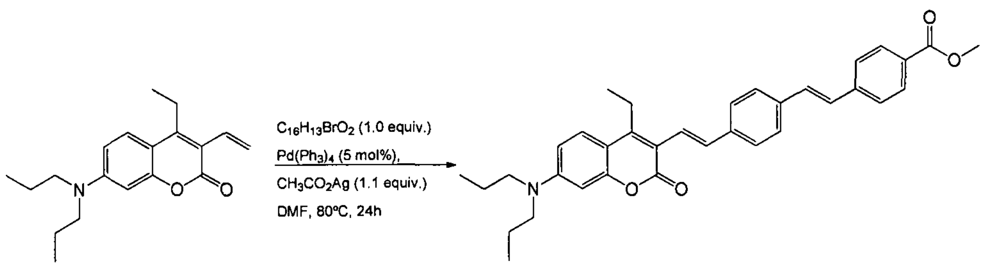


(III-5) (X-5)

To a solution of 7-dipropylamino-4-ethyl-3-vinyl coumarin (III-5) (130 mg, 0.436 mmol), (E)-methyl 4-(4-bromostyryl)benzoate (125.6 mg, 0.396 mmol), $Pd(PPh_3)_4$ (22 mg, 0.020 mmol), and $CH_3CO_2Ag$ (72.8 mg, 0.436 mmol) in DMF (2.0 mL) are added and stirred at 80 °C, under inert atmosphere, for a period of 24 h. After cooling to room temperature, the reaction mixture is evaporated to dryness and the residue is purified by flash column chromatography on silica gel (230-400 mesh; $CH_2Cl_2$) to yield methyl 4-((*E*)-4-((*E*)-2-(7-(dipropylamino)-4-ethyl-coumarin-3 yl)vinyl)styryl)benzoate (X-5).

Step 2: Preparation of 4-((E)-4-((E)-2-(7-(dipropylamino)-4-ethyl-coumarin-3-yl)vinyl)styryl)benzoic acid (XI-5)

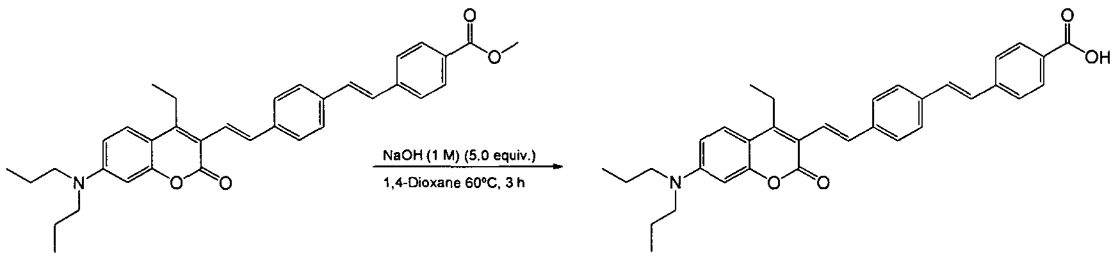


(X-5) (XI-5)

To a solution of methyl 4-((E)-4-((E)-2-(7-(dipropylamino)-4-ethyl-coumarin-3-yl)vinyl)styryl)benzoate (X-5) (147 mg, 0.275 mmol) in 1,4-dioxane (1.3 mL) is added 1 M sodium hydroxide (1.3 mL, 1.277 mmol) and stirred at 60°C for about 3 h. After cooling to room temperature, the reaction is neutralized with HCl (10%) solution. The reaction mixture is evaporated to dryness and the residue is purified by flash column chromatography on silica gel (230-400 mesh; $CHCl_3$/MeOH (9:1) to yield 4-((E)-4-((E)-2-(7-(dipropylamino)-4-ethyl-coumarin-3-yl)vinyl)styryl)benzoic acid (XI-5).

Step 3: Preparation of sodium 4-((4-((E)-4-((E)-2-(7-(dipropylamino)-4-ethyl-coumarin-3-yl)vinyl)styryl)benzoyl)oxy)-2,3,5,6-tetrafluorobenzenesulfonate (XII-5)

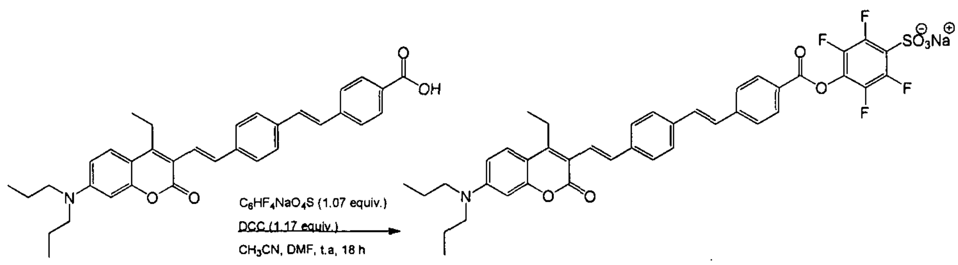


(XI-5) (XII-5)

To a solution of 4-((E)-4-((E)-2-(7-(dipropylamino)-4-ethyl-coumarin-3-yl)vinyl)styryl)benzoic acid (XI-5) (149 mg, 0.286 mmol), 4-sulfotetrafluorophenol sodium salt (81.3 mg, 0,302mmol) and *N,N'*-dicyclohexylcarbodiimide (69.2 mg, 0.335 mmol) in $CH_3CN$ (4 mL) and DMF (1mL) are added and stirred at room temperature for a period of 18 h. The reaction mixture is evaporated to dryness and the residue is purified by flash column chromatography on silica gel (230-400 mesh; $CHCl_3$/MeOH/$H_2O$ (65:20:2) to yield sodium 4-((4-((E)-4-((E)-2-(7-(dipropylamino)-4-ethyl-coumarin-3-yl)vinyl)styryl)benzoyl)oxy)-2,3,5,6-tetrafluorobenzenesulfonate (XII-5).

**Example 29: Synthesis of 5-dioxopyrrolidin-1-yl-4-((E)-4-((E)-2-(7-(dipropylamino)-4-ethyl-coumarin-3-yl)vinyl)styryl)benzoate (XIII-5)**

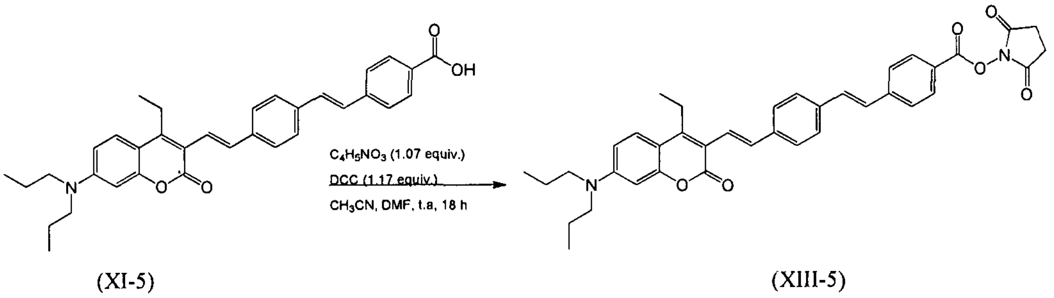


(XI-5) (XIII-5)

To a solution of 4-((E)-4-((E)-2-(7-(dipropylamino)-4-ethyl-coumarin-3-yl)vinyl)styryl)benzoic acid (XI-5) (149 mg, 0.286 mmol), *N*-hydroxysuccinimide (34.7 mg, 0,302mmol) and *N,N'*-dicyclohexylcarbodiimide (69.2 mg, 0.335

mmol) in $CH_3CN$ (4 mL) and DMF (1mL) are added and stirred at room temperature for a period of 18 h. The reaction mixture is evaporated to dryness and the residue is purified by flash column chromatography on silica gel (230-400 mesh; $CHCl_3/MeOH/H_2O$ (65:20:2) to yield 5-dioxopyrrolidin-1-yl-4-((E)-4-((E)-2-(7-(dipropylamino)-4-ethyl-coumarin-3-yl)vinyl)styryl)benzoate (XIII-5).

**Example 30: Synthesis of 2-cyano-3-(4-((*E*)-2-(7-(dipropylamino)-4-ethylcoumarin-3-yl)vinyl)phenyl)acrylic acid (VIII-5)**

Step 1: Preparation of *(E)*-4-(2-(7-(dipropylamino)-4-ethyl-coumarin-3-yl)vinyl)benzaldehyde (VII-5)

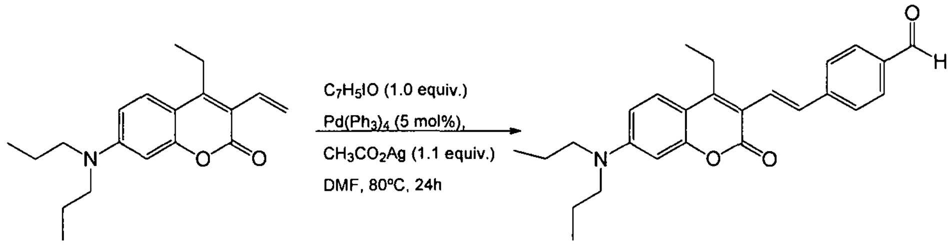


(III-5) (VII-5)

To a solution of 7-dipropylamino-4-ethyl-3-vinyl coumarin (III-5) (116 mg, 0.389 mmol), 4-Iodobenzaldehyde (82.0 mg, 0.353 mmol), $Pd(PPh_3)_4$ (20.0 mg, 0.018 mmol), and $CH_3CO_2Ag$ (65.0 mg, 0.389 mmol) in DMF (2.0 mL) are added and stirred at 80 °C, under inert atmosphere, for a period of 24 h. After cooling to room temperature, the reaction mixture is evaporated to dryness and the residue is purified by flash column chromatography on silica gel (230-400 mesh; $CH_2Cl_2$) to yield (*E*)-4-(2-(7-(dipropylamino)-4-ethyl-coumarin-3-yl)vinyl)benzaldehyde (VII-5).

Step 2: Preparation of 2-cyano-3-(4-((E)-2-(7-(dipropylamino)-4-ethylcoumarin-3-yl)vinyl)- phenyl)acrylic acid (VIII-5)

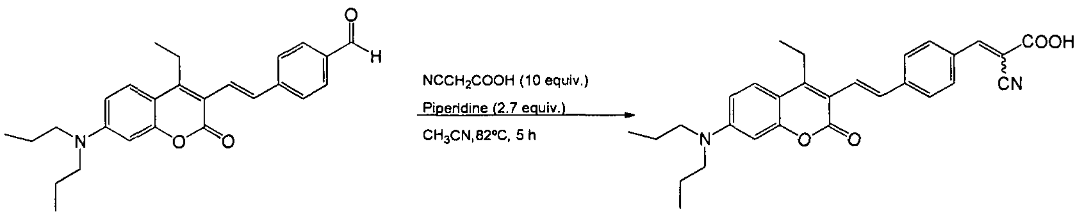


(VII-5) (VIII-5)

To a solution of (*E*)-4-(2-(7-(dipropylamino)-4-ethylcoumarin-3-yl)vinyl)-benzaldehyde (VII-5) (112 mg, 0.277 mmol), cyanoacetic acid (235 mg, 2.77 mmol) and piperidine (0.074 mL, 0.747 mmol) in $CH_3CN$ (12 mL) are added and stirred at 82 °C for a period of 5 h. After cooling to room temperature, the reaction mixture is evaporated to dryness and the residue is purified by flash column chromatography on silica gel (230-400 mesh; $CHCl_3/MeOH/H_2O$ (65:35:5) to yield 2-cyano-3-(4-((*E*)-2-(7-(dipropylamino)-4-ethyl-coumarin-3-yl)vinyl)phenyl)acrylic acid (VIII-5).

**Example 31: Synthesis of2-cyano-3-(4-((E)-4-((E)-2-(7-(dipropylamino)-4-ethyl-coumarin-3-yl)vinyl)styryl)phenyl)acrylic acid (XV-5)**

Step 1: Preparation of4-((E)-4-((E)-2-(7-(dipropylamino)-4-ethyl-coumarin-3-yl)vinyl)styryl)benzaldehyde (XIV-5)

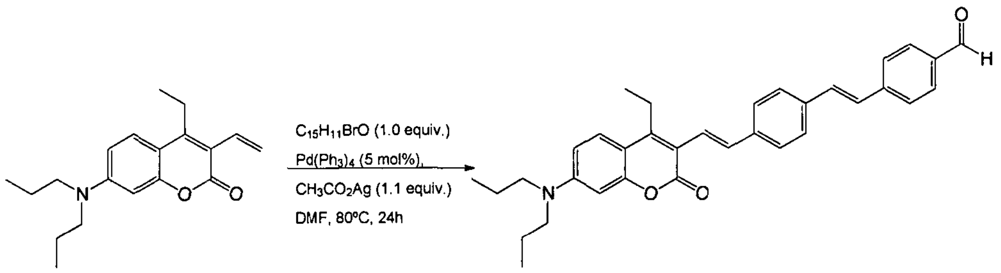


(III-5) (XIV-5)

To a solution of 7-dipropylamino-4-ethyl-3-vinyl coumarin (III-5) (116 mg, 0.389 mmol), (E)-4-(4-bromostyryl)benzaldehyde (101 mg, 0.353 mmol), $Pd(PPh_3)_4$ (20.0 mg, 0.018 mmol), and $CH_3CO_2Ag$ (65.0 mg, 0.389 mmol) in DMF (2.0 mL) are added and stirred at 80 °C, under inert atmosphere, for a period of 24 h. After cooling to room temperature, the reaction mixture is evaporated to dryness and the residue is purified by flash column chromatography on silica gel (230-400 mesh; $CH_2Cl_2$) to yield (4-((E)-4-((E)-2-(7-(dipropylamino)-4-ethyl-coumarin-3-yl)vinyl)styryl)benzaldehyde (XIV-5).

Step 2: Preparation of 2-cyano-3-(4-((E)-4-((E)-2-(7-(dipropylamino)-4-ethyl-coumarin-3-yl)vinyl)styryl)phenyl)acrylic acid (XV-5)

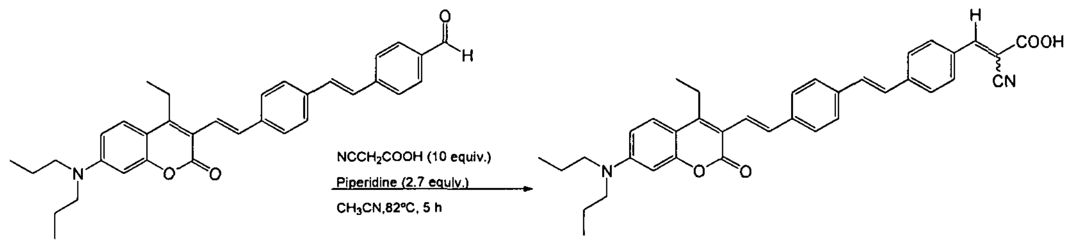


(XIV-5) (XV-5)

To a solution of (4-((E)-4-((E)-2-(7-(dipropylamino)-4-ethyl-coumarin-3-yl)vinyl)styryl)benzaldehyde (XIV-5) (140 mg, 0.277 mmol), cyanoacetic acid (235 mg, 2.77 mmol) and piperidine (0.074 mL, 0.747 mmol) in $CH_3CN$ (12 mL) are added and stirred at 82 °C for a period of 5 h. After cooling to room temperature, the reaction mixture is evaporated to dryness and the residue is purified by flash column chromatography on silica gel (230-400 mesh; $CHCl_3/MeOH/H_2O$ (65:35:5) to yield 2-cyano-3-(4-((E)-4-((E)-2-(7-(dipropylamino)-4-ethyl-coumarin-3-yl)vinyl)styryl)phenyl)acrylic acid (XV-5).

**Example 32: Synthesis of 7-dibutylamino-4-methyl-3-vinyl-coumarin (III-6)**

Step 1: Preparation of 3-bromo-7-dibutylamino-4-methyl-coumarin (II-6)

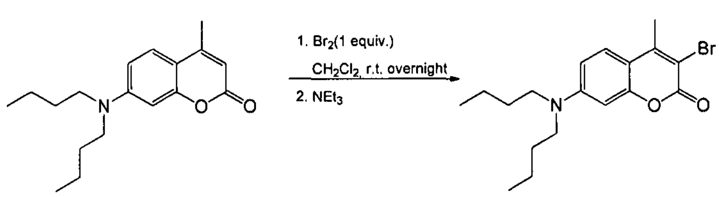


(I-6) (II-6)

To a solution of 7-dibutylamino-4-methyl-coumarin (1-6) (141 mg, 0.492 mmol) in dichloromethane (2 mL) is added bromine (0.025 mL, 0.492 mmol) in an ice bath. After stirring overnight at room temperature, $NEt_3$ (0.200 mL) is added cautiously and the mixture stirred for further 30 minutes. Then, the reaction mixture is evaporated to dryness and

the residue is purified by flash column chromatography on silica gel (230-400 mesh; $CHCl_3$/MeOH (99:1)) to yield 3-bromo-7-dibutylamino-4-methyl-coumarin (II-6).

Step 2: Preparation of 7-dibutylamino-4-methyl-3-vinyl-coumarin (III-6)

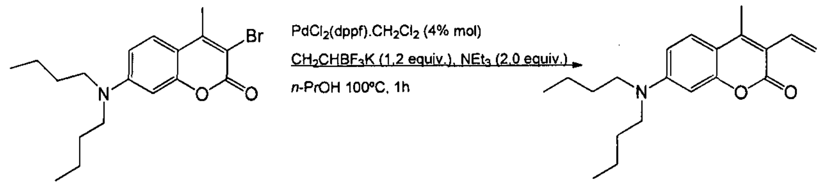


(II-6) (III-6)

To a solution of 3-bromo-7-(dibutylamino)-4-methylcoumarin (II-6) (130 mg, 0.354 mmol), potassium vinyltrifluoroborate (52 mg, 0.425 mmol), $PdCl_2$(dppf).$CH_2Cl_2$ (12 mg, 0.014 mmol), and $NEt_3$ (0.098 mL, 0.708 mmol) in *n*-PrOH (6 mL) are added and stirred at reflux under inert atmosphere for a period of 1 hour. After cooling to room temperature, the reaction mixture is evaporated to dryness and the residue is purified by flash column chromatography on silica gel (230-400 mesh; $CH_2Cl_2$) to yield 7-dibutylamino-4-methyl-3-vinyl coumarin (III-6).

**Example 33: Synthesis of *(E)*-4-(4-(2-(7-(dibutylamino)-4-methyl-coumarin-3-yl)vinyl)benzoyl)-2,3,5,6-tetrafluoro-benzenesulfonate (VI-6)**

Step 1: Preparation of *(E)*-4-(2-(7-(dibutylamino)-4-methyl-coumarin-3-yl)vinyl)methylbenzoate (IV-6)

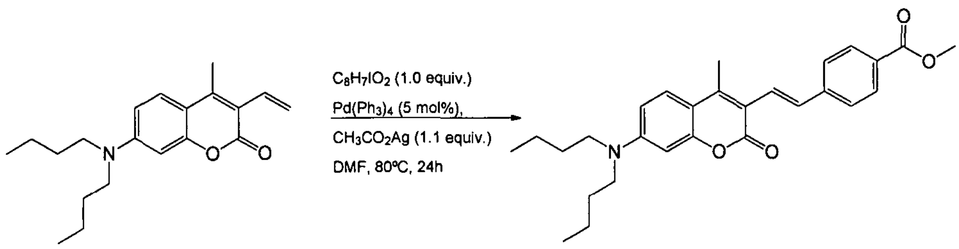


(III-6) (IV-6)

To a solution of 7-dibutylamino-4-methyl-3-vinyl coumarin (III-6) (137 mg, 0.436 mmol), methyl 4-bromobenzoate (85.2 mg, 0.396 mmol), $Pd(PPh_3)_4$ (22 mg, 0.020 mmol), and $CH_3CO_2Ag$ (72.8 mg, 0.436 mmol) in DMF (2.0 mL) are added and stirred at 80 °C, under inert atmosphere, for a period of 24 h. After cooling to room temperature, the reaction mixture is evaporated to dryness and the residue is purified by flash column chromatography on silica gel (230-400 mesh; $CH_2Cl_2$) to yield (E)-4-(2-(7-(dibutylamino)-4-methyl-coumarin-3-yl)vinyl)methylbenzoate (IV-6).

Step 2: Preparation of *(E)*-4-(2-(7-(dibutylamino)-4-methyl-coumarin-3-yl)vinyl)benzoic acid (V-6)

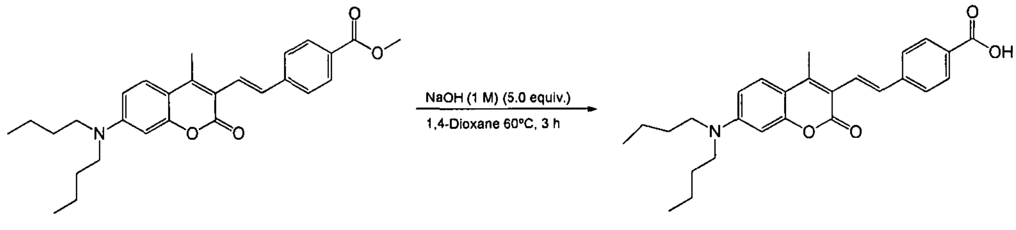


(IV-6) (V-6)

To a solution of *(E)*-methyl-4-(2-(7-(dibutylamino)-4-methyl-coumarin-3-yl)vinyl)benzoate (IV-6) (123 mg, 0.275 mmol) in 1,4-dioxane (1.3 mL) is added 1 M sodium hydroxide (1.3 mL, 1.277 mmol) and stirred at 60 °C for about 3 h. After cooling to room temperature, the reaction is neutralized with HCl (10%) solution. The reaction mixture is evaporated to dryness and the residue is purified by flash column chromatography on silica gel (230-400 mesh; $CHCl_3$/MeOH (9:1) to yield (*E*)-4-(2-(7-(dibutylamino)-4-methyl-coumarin-3-yl)vinyl)benzoic acid (V-6).

Step 3: Preparation of *(E)*-4-(4-(2-(7-(dibutylamino)-4-methyl-coumarin-3-yl)vinyl)benzoyl)-2,3,5,6-tetrafluoro-benzenesulfonate (VI-6)

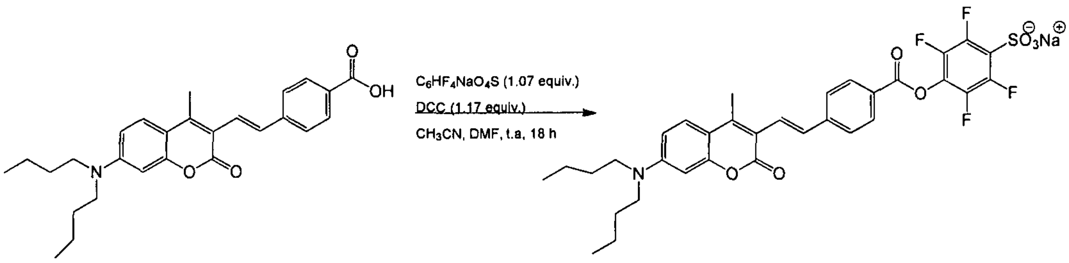


(V-6) (VI-6)

To a solution of (*E*)-4-(2-(7-(dibutylamino)-4-methyl-coumarin-3-yl)vinyl) benzoic acid (V-6) (124 mg, 0.286 mmol), 4-sulfotetrafluorophenol sodium salt (81.3 mg, 0,302mmol) and *N,N'*-dicyclohexylcarbodiimide (69.2 mg, 0.335 mmol) in $CH_3CN$ (4 mL) and DMF (1mL) are added and stirred at room temperature for a period of 18 h. The reaction mixture is evaporated to dryness and the residue is purified by flash column chromatography on silica gel (230-400 mesh; $CHCl_3$/MeOH/$H_2O$ (65:20:2) to yield sodium (*E*)-4-(4-(2-(7-(dibutylamino)-4-methyl-coumarin-3-yl)vinyl)-benzoyl)-2,3,5,6-tetrafluorobenzene-sulfonate (VI-6).

**Example 34: Synthesis of *(E)*-2,5-dioxopyrrolidin-1-yl-4-(2-(7-(dibutylamino)-4-methyl-coumarin-3-yl)vinyl)benzoate (IX-6)**

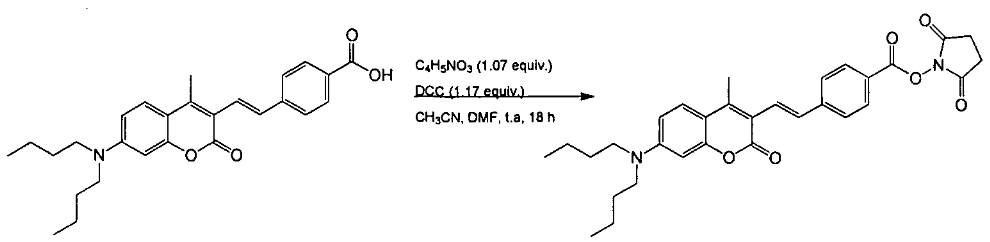


(V-6) (IX-6)

To a solution of (*E*)-4-(2-(7-(dibutylamino)-4-methyl-coumarin-3-yl)vinyl) benzoic acid (V-6) (124 mg, 0.286 mmol), *N*-hydroxysuccinimide (34.7 mg, 0,302mmol), *N,N'*-dicyclohexylcarbodiimide (69.2 mg, 0.335 mmol) in $CH_3CN$ (4 mL) and DMF (1mL) are added and stirred at room temperature for a period of 18 h. The reaction mixture is evaporated to dryness and the residue is purified by flash column chromatography on silica gel (230-400 mesh; $CHCl_3$/MeOH/$H_2O$

(65:20:2) to yield *(E)*-2,5-dioxopyrrolidin-1-yl-4-(2-(7-(dibutylamino)-4-methyl-coumarin-3-yl)vinyl)benzoate (IX-6).

**Example 35: Synthesis of sodium 4-((4-((E)-4-((E)-2-(7-(dibutylamino)-4-methyl-coumarin-3-yl)vinyl)styryl)benzoyl)oxy)-2,3,5,6-tetranuorobenzenesulfonate (XII-6)**

Step 1: Preparation of methyl 4-((*E*)-4-((*E*)-2-(7-(dibutylamino)-4-methyl-coumarin-3-yl)vinyl)styryl)benzoate (X-6)

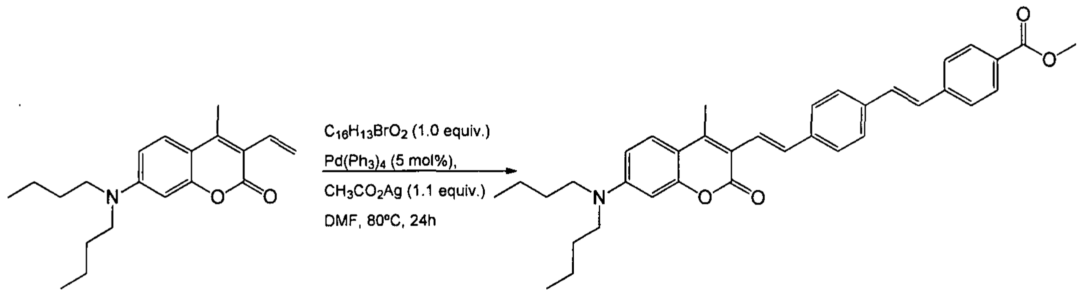


(III-6) (X-6)

To a solution of 7-dibutylamino-4-methyl-3-vinyl coumarin (III-6) (137 mg, 0.436 mmol), (E)-methyl 4-(4-bromostyryl)benzoate (125.6 mg, 0.396 mmol), $Pd(PPh_3)_4$ (22 mg, 0.020 mmol), and $CH_3CO_2Ag$ (72.8 mg, 0.436 mmol) in DMF (2.0 mL) are added and stirred at 80 °C, under inert atmosphere, for a period of 24 h. After cooling to room temperature, the reaction mixture is evaporated to dryness and the residue is purified by flash column chromatography on silica gel (230-400 mesh; $CH_2Cl_2$) to yield methyl 4-((*E*)-4-((*E*)-2-(7-(dibutylamino)-4-methyl-coumarin-3-yl)vinyl)styryl)benzoate (X-6).

Step 2: Preparation of 4-((E)-4-((E)-2-(7-(dibutylamino)-4-methyl-coumarin-3-yl)vinyl)styryl)benzoic acid (XI-6)

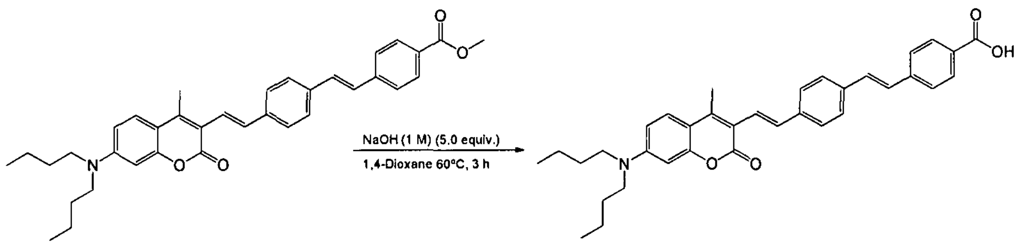


(X-6) (XI-6)

To a solution of methyl 4-((E)-4-((E)-2-(7-(dibutylamino)-4-methyl-coumarin-3-yl)vinyl)styryl)benzoate (X-6) (151 mg, 0.275 mmol) in 1,4-dioxane (1.3 mL) is added 1 M sodium hydroxide (1.3 mL, 1.277 mmol) and stirred at 60 °C for about 3 h. After cooling to room temperature, the reaction is neutralized with HCl (10%) solution. The reaction mixture is evaporated to dryness and the residue is purified by flash column chromatography on silica gel (230-400 mesh; $CHCl_3$/MeOH (9:1) to yield 4-((E)-4-((E)-2-(7-(dibutylamino)-4-methyl-coumarin-3-yl)vinyl)styryl)benzoic acid (XI-6).

Step 3: Preparation of sodium 4-((4-((E)-4-((E)-2-(7-(dibutylamino)-4-methyl-coumarin-3-yl)vinyl)styryl)benzoyl)oxy)-2,3,5,6-tetrafluorobenzenesulfonate (XII-6)

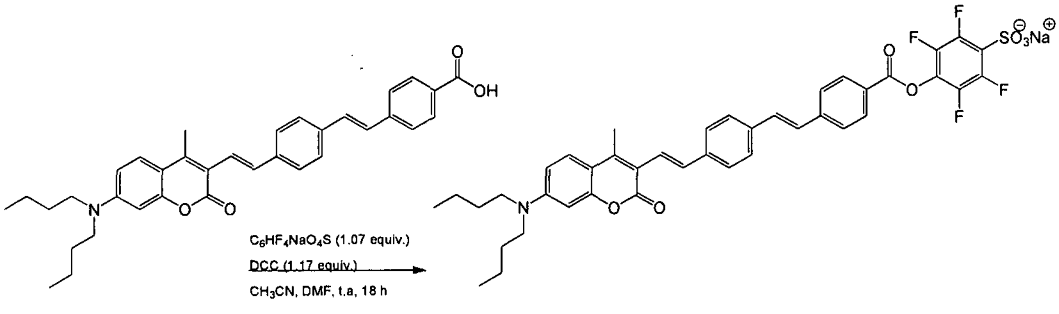


(XI-6) (XII-6)

To a solution of 4-((E)-4-((E)-2-(7-(dibutylamino)-4-methyl-coumarin-3-yl)vinyl)styryl)benzoic acid (XI-6) (153 mg, 0.286 mmol), 4-sulfotetrafluorophenol sodium salt (81.3 mg, 0,302mmol) and N,N'-dicyclohexylcarbodiimide (69.2 mg, 0.335 mmol) in $CH_3CN$ (4 mL) and DMF (1mL) are added and stirred at room temperature for a period of 18 h. The reaction mixture is evaporated to dryness and the residue is purified by flash column chromatography on silica gel (230-400 mesh; $CHCl_3$/MeOH/$H_2O$ (65:20:2) to yield sodium 4-((4-((E)-4-((E)-2-(7-(dibutylamino)-4-methyl-coumarin-3-yl)vinyl)styryl)benzoyl)oxy)-2,3,5,6-tetrafluorobenzenesulfonate (XII-6).

**Example 36: Synthesis of 5-dioxopyrrolidin-1-yl-4-((E)-4-((E)-2-(7-(dibutylamino)-4-methyl-coumarin-3-yl)vinyl)styryl)benzoate (XIII-6)**

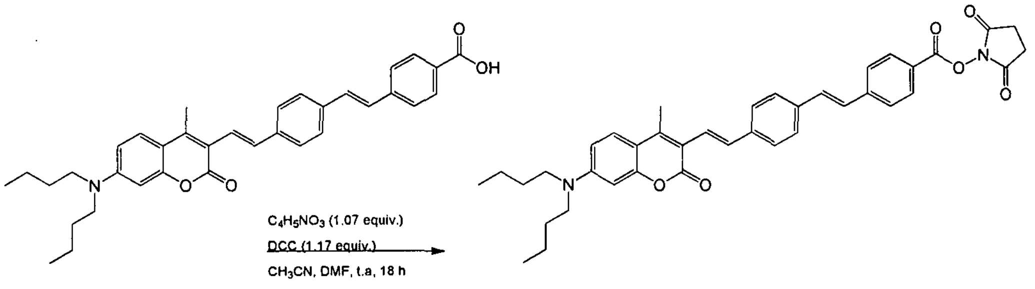


(XI-6) (XIII-6)

To a solution of 4-((E)-4-((E)-2-(7-(dibutylamino)-4-methyl-coumarin-3-yl)vinyl)styryl)benzoic acid (XI-6) (153 mg, 0.286 mmol), *N*-hydroxysuccinimide (34.7 mg, 0,302mmol) and *N,N'*-dicyclohexylcarbodiimide (69.2 mg, 0.335 mmol) in $CH_3CN$ (4 mL) and DMF (1mL) are added and stirred at room temperature for a period of 18 h. The reaction mixture is evaporated to dryness and the residue is purified by flash column chromatography on silica gel (230-400 mesh; $CHCl_3$MeOH/$H_2O$ (65:20:2) to yield 5-dioxopyrrolidin-1-yl-4-((E)-4-((E)-2-(7-(dibutylamino)-4-methyl-coumarin-3-yl)vinyl)styryl)benzoate (XIII-6).

**Example 37: Synthesis of 2-cyano-3-(4-(*(E)*-2-(7-(dibutylamino)-4-methylcoumarin-3-yl)vinyl)phenyl)acrylic acid (VIII-6)**

Step 1: Preparation of *(E)*-4-(2-(7-(dibutylamino)-4-methyl-coumarin-3-yl)vinyl)benzaldehyde (VII-6)

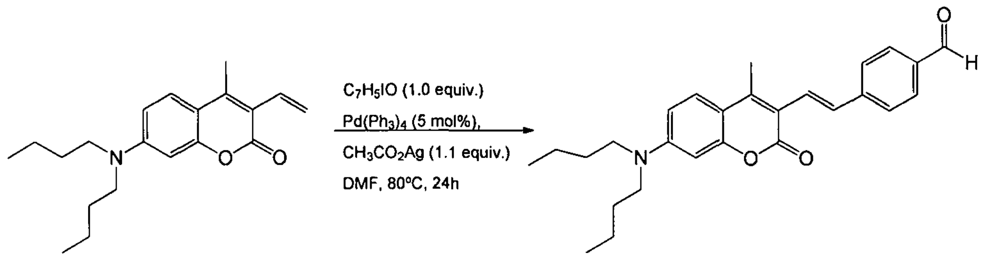


(III-6) (VII-6)

To a solution of 7-dibutylamino-4-methyl-3-vinyl coumarin (III-6) (122 mg, 0.389 mmol), 4-Iodobenzaldehyde (82.0 mg, 0.353 mmol), $Pd(PPh_3)_4$ (20.0 mg, 0.018 mmol), and $CH_3CO_2Ag$ (65.0 mg, 0.389 mmol) in DMF (2.0 mL) are added and stirred at 80 °C, under inert atmosphere, for a period of 24 h. After cooling to room temperature, the reaction mixture is evaporated to dryness and the residue is purified by flash column chromatography on silica gel (230-400 mesh; $CH_2Cl_2$) to yield (*E*)-4-(2-(7-(dibutylamino)-4-methyl-coumarin-3-yl)vinyl)benzaldehyde (VII-6).

Step 2: Preparation of 2-cyano-3-(4-((E)-2-(7-(dibutylamino)-4-methylcoumarin-3-yl)vinyl)- phenyl)acrylic acid (VIII-6)

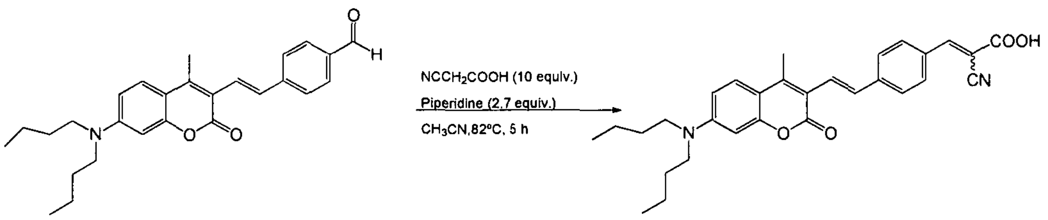


(VII-6) (VIII-6)

To a solution of (*E*)-4-(2-(7-(dibutylamino)-4-methylcoumarin-3-yl)vinyl)-benzaldehyde (VII-6) (116 mg, 0.277 mmol), cyanoacetic acid (235 mg, 2.77 mmol) and piperidine (0.074 mL, 0.747 mmol) in $CH_3CN$ (12 mL) are added and stirred at 82 °C for a period of 5 h. After cooling to room temperature, the reaction mixture is evaporated to dryness and the residue is purified by flash column chromatography on silica gel (230-400 mesh; $CHCl_3$/MeOH/$H_2O$ (65:35:5) to yield 2-cyano-3-(4-((*E*)-2-(7-(dibutylamino)-4-methyl-coumarin-3-yl)vinyl)phenyl)acrylic acid (VIII-6).

**Example 38: Synthesis of2-cyano-3-(4-((E)-4-((E)-2-(7-(dibutylamino)-4-methyl-coumarin-3-yl)vinyl)styryl)phenyl)acrylic acid (XV-6)**

Step 1: Preparation of4-((E)-4-((E)-2-(7-(dibutylamino)-4-methyl-coumarin-3-yl)vinyl)styryl)benzaldehyde (XIV-6)

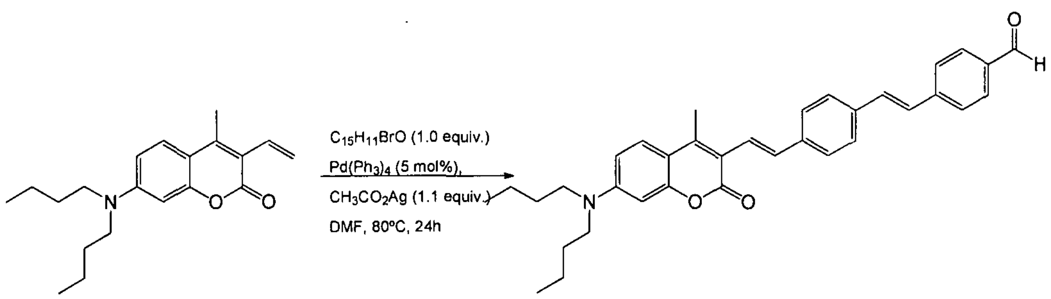


(III-6) (XIV-6)

To a solution of 7-dibutylamino-4-methyl-3-vinyl coumarin (III-6) (122 mg, 0.389 mmol), (E)-4-(4-bromostyryl)benzaldehyde (101 mg, 0.353 mmol), $Pd(PPh_3)_4$ (20.0 mg, 0.018 mmol), and $CH_3CO_2Ag$ (65.0 mg, 0.389 mmol) in

DMF (2.0 mL) are added and stirred at 80 °C, under inert atmosphere, for a period of 24 h. After cooling to room temperature, the reaction mixture is evaporated to dryness and the residue is purified by flash column chromatography on silica gel (230-400 mesh; $CH_2Cl_2$) to yield (4-((E)-4-((E)-2-(7-(dibutylamino)-4-methyl-coumarin-3-yl)vinyl)styryl)benzaldehyde (XIV-6).

Step 2: Preparation of 2-cyano-3-(4-((E)-4-((E)-2-(7-(dibutylamino)-4-methyl-coumarin-3-yl)vinyl)styryl)phenyl)acrylic acid (XV-6)

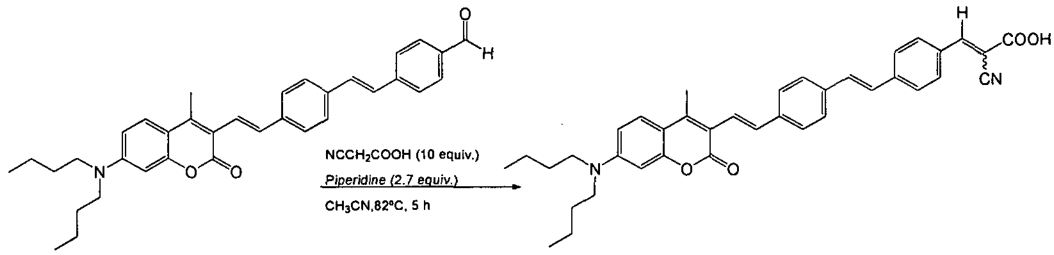


(XIV-6) (XV-6)

To a solution of (4-((E)-4-((E)-2-(7-(dibutylamino)-4-methyl-coumarin-3-yl)vinyl)styryl)benzaldehyde (XIV-6) (144 mg, 0.277 mmol), cyanoacetic acid (235 mg, 2.77 mmol) and piperidine (0.074 mL, 0.747 mmol) in $CH_3CN$ (12 mL) are added and stirred at 82 °C for a period of 5 h. After cooling to room temperature, the reaction mixture is evaporated to dryness and the residue is purified by flash column chromatography on silica gel (230-400 mesh; $CHCl_3/MeOH/H_2O$ (65:35:5) to yield 2-cyano-3-(4-((E)-4-((E)-2-(7-(dibutylamino)-4-methyl-coumarin-3-yl)vinyl)styryl)phenyl)acrylic acid (XV-6).

**Example 39: Synthesis of 7-dibutylamino-4-ethyl-3-vinyl-coumarin (III-7)**

Step 1: Preparation 3-bromo-7-dibutylamino-4-ethyl-coumarin (II-7)

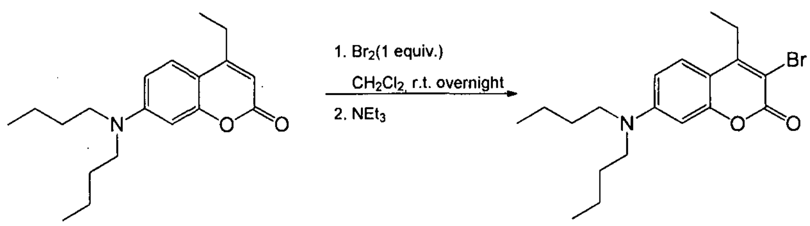


(I-7) (II-7)

To a solution of 7-dibutylamino-4-ethyl-coumarin (I-7) (148 mg, 0.492 mmol) in dichloromethane (2 mL) is added bromine (0.025 mL, 0.492 mmol) in an ice bath. After stirring overnight at room temperature, $NEt_3$ (0.200 mL) is added cautiously and the mixture stirred for further 30 minutes. Then, the reaction mixture is evaporated to dryness and the residue is purified by flash column chromatography on silica gel (230-400 mesh; $CHCl_3/MeOH$ (99:1)) to yield 3-bromo-7-dibutylamino-4-ethyl-coumarin (II-7).

Step 2: Preparation of 7-dibutylamino-4-ethyl-3-vinyl-coumarin (III-7)

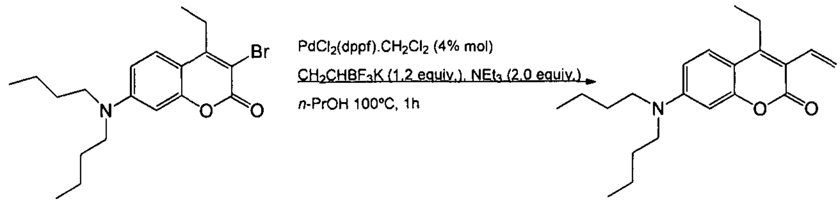


(II-7) (III-7)

To a solution of 3-bromo-7-(dibutylamino)-4-ethylcoumarin (II-7) (135 mg, 0.354 mmol), potassium vinyltrifluoroborate (52 mg, 0.425 mmol), $PdCl_2$(dppf).$CH_2Cl_2$ (12 mg, 0.014 mmol), and $NEt_3$ (0.098 mL, 0.708 mmol) in *n*-PrOH (6 mL) are added and stirred at reflux under inert atmosphere for a period of 1 hour. After cooling to room temperature, the reaction mixture is evaporated to dryness and the residue is purified by flash column chromatography on silica gel (230-400 mesh; $CH_2Cl_2$) to yield 7-dibutylamino-4-ethyl-3-vinyl coumarin (III-7).

**Example 40: Synthesis of *(E)*-4-(4-(2-(7-(dibutylamino)-4-ethyl-coumarin-3-yl)vinyl)benzoyl)-2,3,5,6-tetrafluorobenzenesulfonate (VI-7)**

Step 1: Preparation of *(E)*-4-(2-(7-(dibutylamino)-4-ethyl-coumarin-3-yl)vinyl)methylbenzoate (IV-7)

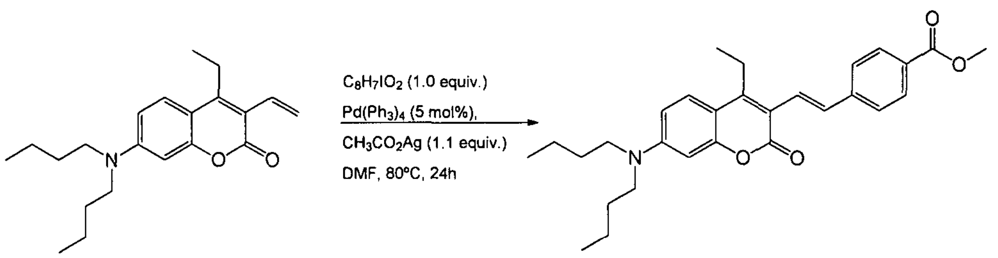


(III-7) (IV-7)

To a solution of 7-dibutylamino-4-ethyl-3-vinyl coumarin (III-7) (143 mg, 0.436 mmol), methyl 4-bromobenzoate (85.2 mg, 0.396 mmol), $Pd(PPh_3)_4$ (22 mg, 0.020 mmol), and $CH_3CO_2Ag$ (72.8 mg, 0.436 mmol) in DMF (2.0 mL) are added and stirred at 80 °C, under inert atmosphere, for a period of 24 h. After cooling to room temperature, the reaction mixture is evaporated to dryness and the residue is purified by flash column chromatography on silica gel (230-400 mesh; $CH_2Cl_2$) to yield (E)-4-(2-(7-(dibutylamino)-4-ethyl-coumarin-3-yl)vinyl)methylbenzoate (IV-7).

Step 2: Preparation of *(E)*-4-(2-(7-(dibutylamino)-4-ethyl-coumarin-3-yl)vinyl)benzoic acid (V-7)

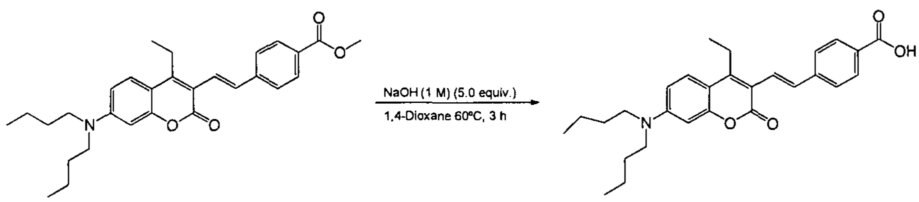


(IV-7) (V-7)

To a solution of *(E)*-methyl-4-(2-(7-(dibutylamino)-4-ethyl-coumarin-3-yl)vinyl)benzoate (IV-7) (127 mg, 0.275 mmol) in 1,4-dioxane (1.3 mL) is added 1 M sodium hydroxide (1.3 mL, 1.277 mmol) and stirred at 60 °C for about 3 h. After cooling to room temperature, the reaction is neutralized with HCl (10%) solution. The reaction mixture is evaporated to dryness and the residue is purified by flash column chromatography on silica gel (230-400 mesh; $CHCl_3$/MeOH (9:1) to yield (*E*)-4-(2-(7-(dibutylamino)-4-ethyl-coumarin-3-yl)vinyl)benzoic acid (V-7).

Step 3: Preparation of *(E)*-4-(4-(2-(7-(dibutylamino)-4-ethyl-coumarin-3-yl)vinyl)benzoyl)-2,3,5,6-tetrafluoro-benzenesulfonate (VI-7)

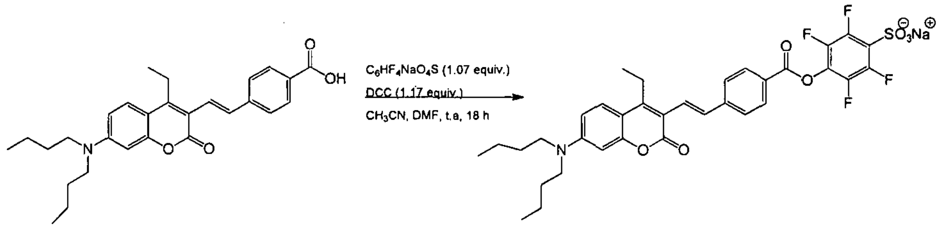


(V-7) (VI-7)

To a solution of (*E*)-4-(2-(7-(dibutylamino)-4-ethyl-coumarin-3-yl)vinyl) benzoic acid (V-7) (128 mg, 0.286 mmol), 4-sulfotetrafluorophenol sodium salt (81.3 mg, 0,302mmol) and *N,N'*-dicyclohexylcarbodiimide (69.2 mg, 0.335 mmol) in $CH_3CN$ (4 mL) and DMF (1mL) are added and stirred at room temperature for a period of 18 h. The reaction mixture is evaporated to dryness and the residue is purified by flash column chromatography on silica gel (230-400 mesh; $CHCl_3$/MeOH/$H_2O$ (65:20:2) to yield sodium (*E*)-4-(4-(2-(7-(dibutylamino)-4-ethyl-coumarin-3-yl)vinyl)-benzoyl)-2,3,5,6-tetrafluorobenzene-sulfonate (VI-7).

**Example 41: Synthesis of (E)-2,5-dioxopyrrolidin-1-yl-4-(2-(7-(dibutylamino)-4-ethyl-coumarin-3-yl)vinyl)benzoate (IX-7)**

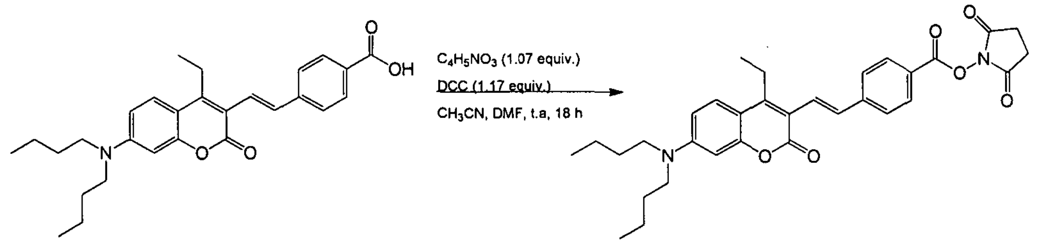


(V-7) (IX-7)

To a solution of (*E*)-4-(2-(7-(dibutylamino)-4-ethyl-coumarin-3-yl)vinyl) benzoic acid (V-7) (128 mg, 0.286 mmol), *N*-hydroxysuccinimide (34.7 mg, 0,302mmol), *N,N'*-dicyclohexylcarbodiimide (69.2 mg, 0.335 mmol) in $CH_3CN$ (4 mL) and DMF (1mL) are added and stirred at room temperature for a period of 18 h. The reaction mixture is evaporated to dryness and the residue is purified by flash column chromatography on silica gel (230-400 mesh; $CHCl_3$/MeOH/$H_2O$ (65:20:2) to yield *(E)*-2,5-dioxopyrrolidin-1-yl-4-(2-(7-(dibutylamino)-4-ethyl-coumarin-3-yl)vinyl)benzoate (IX-7).

**Example 42: Synthesis of sodium 4-((4-((E)-4-((E)-2-(7-(dibutylamino)-4-ethyl-coumarin-3-yl)vinyl)styryl)benzoyl)oxy)-2,3,5,6-tetrafluorobenzenesulfonate (XII-7)**

Step 1: Preparation of methyl 4-((*E*)-4-((*E*)-2-(7-(dibutylamino)-4-ethyl-coumarin-3-yl)vinyl)styryl)benzoate (X-7)

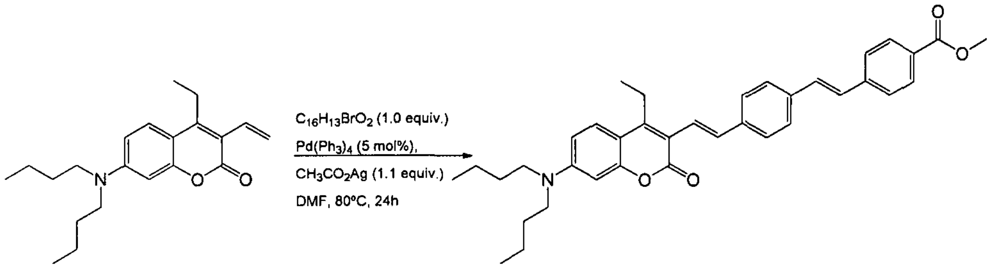


(III-7) (X-7)

To a solution of 7-dibutylamino-4-ethyl-3-vinyl coumarin (III-7) (143 mg, 0.436 mmol), (E)-methyl 4-(4-bromostyryl)benzoate (125.6 mg, 0.396 mmol), $Pd(PPh_3)_4$ (22 mg, 0.020 mmol), and $CH_3CO_2Ag$ (72.8 mg, 0.436 mmol) in DMF (2.0 mL) are added and stirred at 80 °C, under inert atmosphere, for a period of 24 h. After cooling to room temperature, the reaction mixture is evaporated to dryness and the residue is purified by flash column chromatography on silica gel (230-400 mesh; $CH_2Cl_2$) to yield methyl 4-((*E*)-4-((*E*)-2-(7-(dibutylamino)-4-ethyl-coumarin-3-yl)vinyl)styryl)benzoate (X-7).

Step 2: Preparation of 4-((E)-4-((E)-2-(7-(dibutylamino)-4-ethyl-coumarin-3-yl)vinyl)styryl)benzoic acid (XI-7)

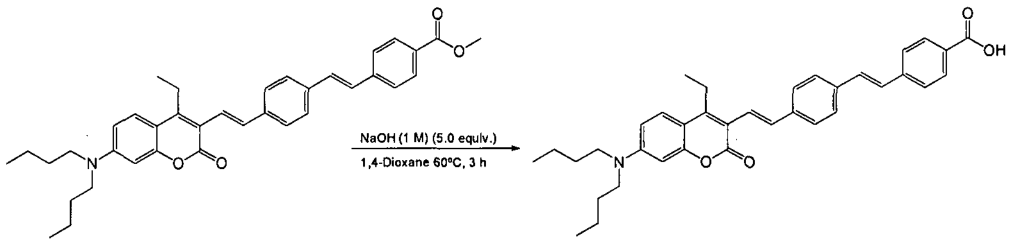


(X-7) (XI-7)

To a solution of methyl 4-((E)-4-((E)-2-(7-(dibutylamino)-4-ethyl-coumarin-3-yl)vinyl)styryl)benzoate (X-7) (155 mg, 0.275 mmol) in 1,4-dioxane (1.3 mL) is added 1 M sodium hydroxide (1.3 mL, 1.277 mmol) and stirred at 60 °C for about 3 h. After cooling to room temperature, the reaction is neutralized with HCl (10%) solution. The reaction mixture is evaporated to dryness and the residue is purified by flash column chromatography on silica gel (230-400 mesh; $CHCl_3$/MeOH (9:1) to yield 4-((E)-4-((E)-2-(7-(dibutylamino)-4-ethyl-coumarin-3-yl)vinyl)styryl)benzoic acid (XI-7).

Step 3: Preparation of sodium 4-((4-((E)-4-((E)-2-(7-(dibutylamino)-4-ethyl-coumarin-3-yl)vinyl)styryl)benzoyl)oxy)-2,3,5,6-tetrafluorobenzenesulfonate (XII-7)

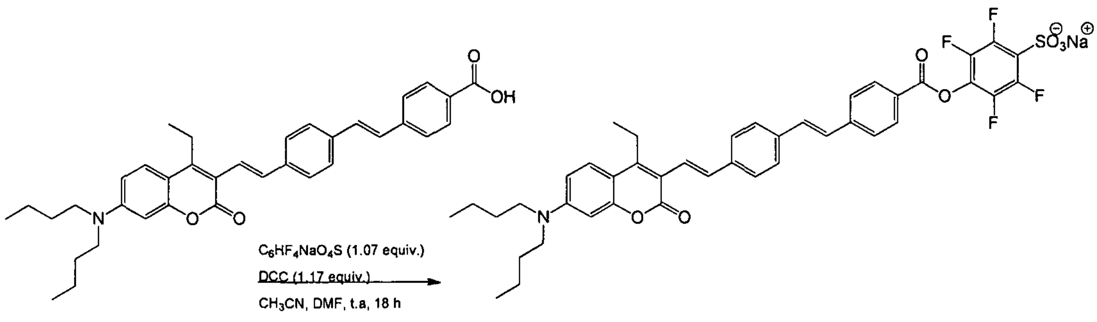


(XI-7) (XII-7)

To a solution of 4-((E)-4-((E)-2-(7-(dibutylamino)-4-ethyl-coumarin-3-yl)vinyl)styryl)benzoic acid (XI-7) (157 mg,

0.286 mmol), 4-sulfotetrafluorophenol sodium salt (81.3 mg, 0,302mmol) and *N,N'*-dicyclohexylcarbodiimide (69.2 mg, 0.335 mmol) in $CH_3CN$ (4 mL) and DMF (1mL) are added and stirred at room temperature for a period of 18 h. The reaction mixture is evaporated to dryness and the residue is purified by flash column chromatography on silica gel (230-400 mesh; $CHCl_3/MeOH/H_2O$ (65:20:2) to yield sodium 4-((4-((E)-4-((E)-2-(7-(dibutylamino)-4-ethyl-coumarin-3-yl)vinyi)styryl)benzoyl)oxy)-2,3,5,6-tetrafluorobenzenesulfonate (XII-7).

**Example 43: Synthesis of5-dioxopyrrolidin-1-yl-4-((E)-4-((E)-2-(7-(dibutylamino)-4-ethyl-coumarin-3-yl)vinyl)styryl)benzoate (XIII-7)**

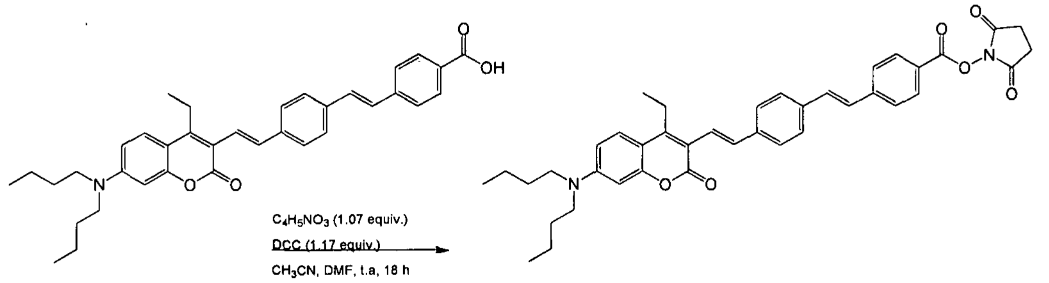


(XI-7) (XIII-7)

To a solution of 4-((E)-4-((E)-2-(7-(dibutylamino)-4-ethyl-coumarin-3-yl)vinyl)styryl)benzoic acid (XI-7) (157 mg, 0.286 mmol), N-hydroxysuccinimide (34.7 mg, 0,302mmol) and *N,N'*-dicyclohexylcarbodiimide (69.2 mg, 0.335 mmol) in $CH_3CN$ (4 mL) and DMF (1mL) are added and stirred at room temperature for a period of 18 h. The reaction mixture is evaporated to dryness and the residue is purified by flash column chromatography on silica gel (230-400 mesh; $CHCl_3/MeOH/H_2O$ (65:20:2) to yield 5-dioxopyrrolidin-1-yl-4-((E)-4-((E)-2-(7-(dibutylamino)-4-ethyl-coumarin-3-yl)vinyl)styryl)benzoate (XIII-7).

**Example 44: Synthesis of 2-cyano-3-(4-(*(E)*-2-(7-(dibutylamino)-4-ethylcoumarin-3-yl)vinyl)phenyl)acrylic acid (VIII-7)**

Step 1: Preparation of *(E)*-4-(2-(7-(dibutylamino)-4-ethyl-coumarin-3-yl)vinyl)benzaldehyde (VII-7)

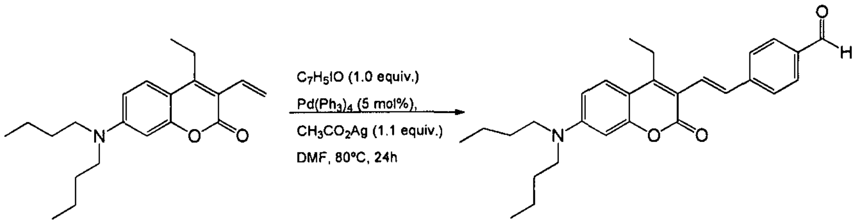


(III-7) (VII-7)

To a solution of 7-dibutylamino-4-ethyl-3-vinyl coumarin (III-7) (127 mg, 0.389 mmol), 4-Iodobenzaldehyde (82.0 mg, 0.353 mmol), $Pd(PPh_3)_4$ (20.0 mg, 0.018 mmol), and $CH_3CO_2Ag$ (65.0 mg, 0.389 mmol) in DMF (2.0 mL) are added and stirred at 80 °C, under inert atmosphere, for a period of 24 h. After cooling to room temperature, the reaction mixture is evaporated to dryness and the residue is purified by flash column chromatography on silica gel (230-400 mesh; $CH_2Cl_2$) to yield (*E*)-4-(2-(7-(dibutylamino)-4-ethyl-coumarin-3-yl)vinyl)benzaldehyde (VII-7).

Step 2: Preparation of 2-cyano-3-(4-((E)-2-(7-(dibutylamino)-4-ethylcoumarin-3-yl)vinyl)- phenyl)acrylic acid (VIII-7)

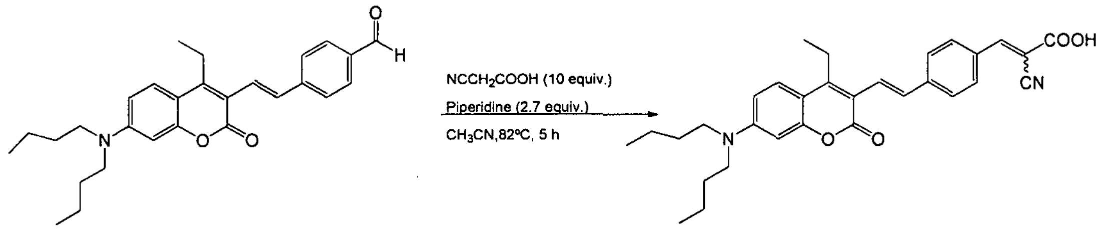


(VII-7) (VIII-7)

To a solution of (*E*)-4-(2-(7-(dibutylamino)-4-ethylcoumarin-3-yl)vinyl)-benzaldehyde (VII-7) (125 mg, 0.277 mmol), cyanoacetic acid (235 mg, 2.77 mmol) and piperidine (0.074 mL, 0.747 mmol) in $CH_3CN$ (12 mL) are added and stirred at 82 °C for a period of 5 h. After cooling to room temperature, the reaction mixture is evaporated to dryness and the residue is purified by flash column chromatography on silica gel (230-400 mesh; $CHCl_3/MeOH/H_2O$ (65:35:5) to yield 2-cyano-3-(4-((*E*)-2-(7-(dibutylamino)-4-ethyl-coumarin-3-yl)vinyl)phenyl)acrylic acid (VIII-7).

**Example 45: Synthesis of 2-cyano-3-(4-((E)-4-((E)-2-(7-(dibutylamino)-4-ethyl-coumarin-3-yl)vinyl)styryl)phenyl)acrylic acid (XV-7)**

Step 1: Preparation of 4-((E)-4-((E)-2-(7-(dibutylamino)-4-ethyl-coumarin-3-yl)vinyl)styryl)benzaldehyde (XIV-7)

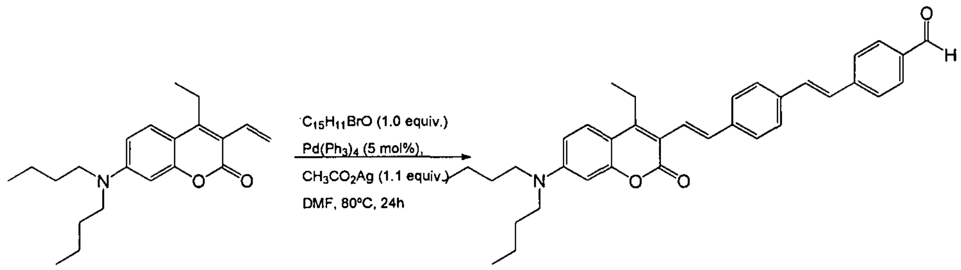


(III-7) (XIV-7)

To a solution of 7-dibutylamino-4-ethyl-3-vinyl coumarin (III-7) (127 mg, 0.389 mmol), (E)-4-(4-bromostyryl)benzaldehyde (101 mg, 0.353 mmol), $Pd(PPh_3)_4$ (20.0 mg, 0.018 mmol), and $CH_3CO_2Ag$ (65.0 mg, 0.389 mmol) in DMF (2.0 mL) are added and stirred at 80 °C, under inert atmosphere, for a period of 24 h. After cooling to room temperature, the reaction mixture is evaporated to dryness and the residue is purified by flash column chromatography on silica gel (230-400 mesh; $CH_2Cl_2$) to yield (4-((E)-4-((E)-2-(7-(dibutylamino)-4-ethyl-coumarin-3-yl)vinyl)styryl)benzaldehyde (XIV-7).

Step 2: Preparation of 2-cyano-3-(4-((E)-4-((E)-2-(7-(dibutylamino)-4-ethyl-coumarin-3-yl)vinyl)styryl)phenyl)acrylic acid (XV-7)

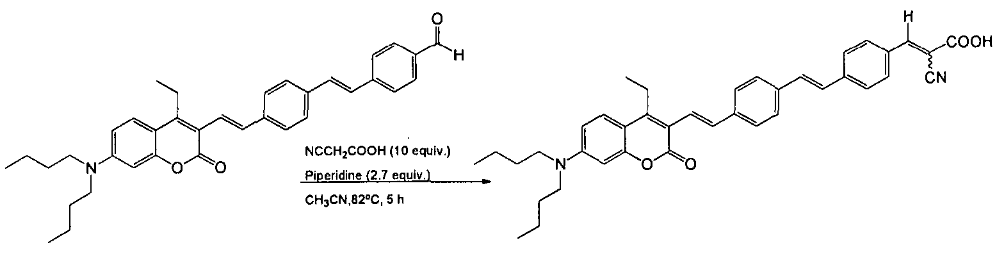


(XIV-7) (XV-7)

To a solution of (4-((E)-4-((E)-2-(7-(dibutylamino)-4-ethyl-coumarin-3-yl)vinyl)styryl)benzaldehyde (XIV-2) (148

mg, 0.277 mmol), cyanoacetic acid (235 mg, 2.77 mmol) and piperidine (0.074 mL, 0.747 mmol) in $CH_3CN$ (12 mL) are added and stirred at 82 °C for a period of 5 h. After cooling to room temperature, the reaction mixture is evaporated to dryness and the residue is purified by flash column chromatography on silica gel (230-400 mesh; $CHCl_3/MeOH/H_2O$ (65:35:5) to yield 2-cyano-3-(4-((E)-4-((E)-2-(7-(dibutylamino)-4-ethyl-coumarin-3-yl)vinyl)styryl)phenyl)acrylic acid.

## Claims

1. A compound of formula (III)

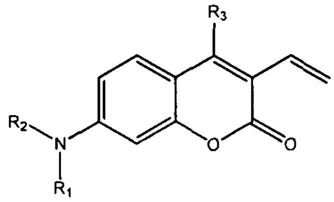

(III)

wherein:

$R_1$ and $R_2$ are independently $-(CH_2)_n-CH_3$, wherein n is 0, 1, 2 or 3; and
$R_3$ is $-(CH_2)_n-CH_3$, wherein n is 0 or 1.

2. The compound according to claim 1, wherein $R_1$ and $R_2$ are $-(CH_2)-CH_3$ and $R_3$ is $-CH_3$.

3. A method for synthesis of compounds of claim 1 comprising the following steps:

- reacting a compound of formula (I) wherein:

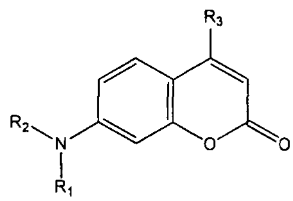

(I)

$R_1$ and $R_2$ are independently $-(CH_2)_n-CH_3$, wherein n is 0, 1, 2 or 3; and
R3 is $-(CH_2)_n-CH_3$, wherein n is 0 or 1,

with bromine in a reaction solvent selected from the group comprising chloroform, dichloromethane, 1,2-dichloroethane, acetonitrile, a mixed solvent thereof and the like, under stirring at a temperature between 0 °C and 5 °C for 10 hours to 24 hours;
- adding to the reactional mixture a base selected from the group comprising triethylamine, 4-(dimethylamino)pyridine, piperidine, piperazine, pyridine, 1,4-diazabicyclo[2.2.2]octane (DABCO), 1,8-diazabicyclo[5.4.0]undec-7-ene (DBU) and the like, and stirring the reactional mixture at a temperature between 20 °C and 25 °C for 30 minutes to 3 hours;
- evaporating the reaction mixture to dryness and purifying the residue to produce a compound of formula (II)

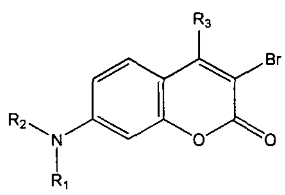

(II);

- reacting a compound of formula (II) with potassium vinyltrifluoroborate and [1,1'-bis(diphenylphosphino)ferrocene]dichloropalladium(II) complex with dichloromethane in a reaction solvent selected from the group comprising n-propanol, 2-propanol, ethanol, tert-butanol, 2-butanol, acetonitrile, a mixed solvent thereof and the like, and stirring under inert atmosphere at a temperature between 78 °C and 98 °C for 15 minutes to 2 hours; and
- evaporating the reaction mixture to dryness and purifying the residue to produce a compound of formula (III)

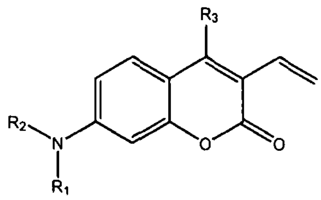

(III).

4. A compound of formula (A)

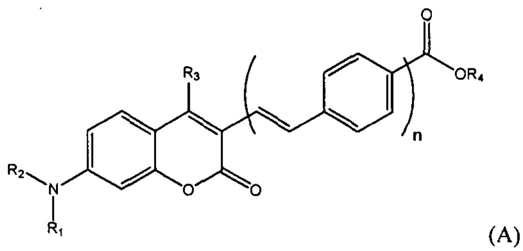

(A)

wherein:

n is 1 or 2,
$R_1$ and $R_2$ are independently $-(CH_2)_n-CH_3$, wherein n is 0, 1, 2 or 3;
$R_3$ is $-(CH_2)_n-CH_3$, wherein n is 0 or 1; and
R4 is

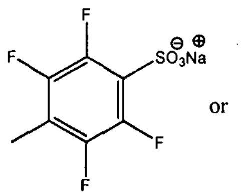

or

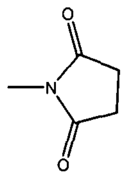

.

5. Compound according to claim 4, wherein n is 1, $R_1$ and $R_2$ are $-(CH_2)-CH_3$, $R_3$ is $-CH_3$ and $R_4$ is

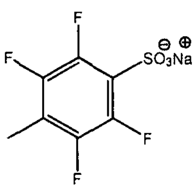

.

6. A compound of formula (B)

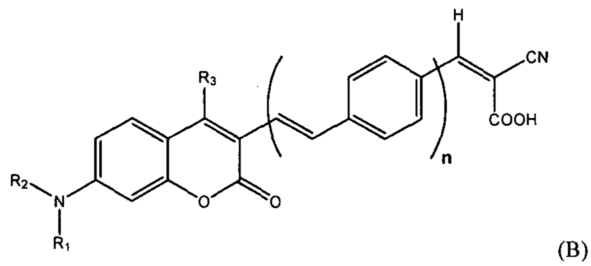

(B)

wherein:

n is 1 or 2,
$R_1$ and $R_2$ are independently $-(CH_2)_n-CH_3$, wherein n is 0, 1, 2 or 3; and
$R_3$ is $-(CH_2)_n-CH_3$, wherein n is 0 or 1.

**7.** Compound according to claim 6, wherein n is 1, $R_1$ and $R_2$ are $-(CH_2)-CH_3$ and $R_3$ is $-CH_3$

**Patentansprüche**

**1.** Verbindung der Formel (III)

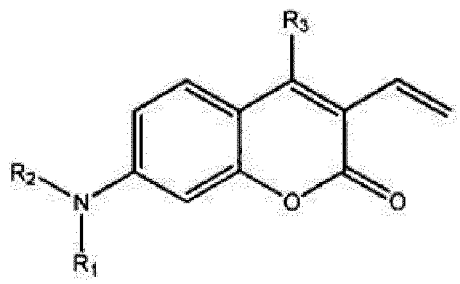

(III)

wobei:

$R_1$ und $R_2$ unabhängig $-(CH_2)_n-CH_3$ sind, worin n 0, 1, 2 oder 3 ist; und
$R_3$ $-(CH_2)_n-CH_3$ ist, worin n 0 oder 1 ist.

**2.** Verbindung nach Anspruch 1, wobei $R_1$ und $R_2$ $-(CH_2)-CH_3$ sind und $R_3$ $-CH_3$ ist.

**3.** Verfahren zur Synthese von Verbindungen nach Anspruch 1, umfassend die folgenden Schritte:

- Umsetzen einer Verbindung der Formel (I)

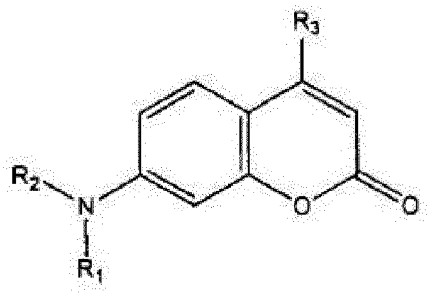

(I)

wobei:

$R_1$ und $R_2$ unabhängig $-(CH_2)_n-CH_3$ sind, worin n 0, 1, 2 oder 3 ist; und
$R_3$ $-(CH_2)_n-CH_3$ ist, worin n 0 oder 1 ist,
mit Brom in einem Reaktionslösungsmittel, ausgewählt aus der Gruppe umfassend Chloroform, Dichlormethan, 1,2-Dichlorethan, Acetonitril, einem gemischten Lösungsmittel davon und dergleichen, unter Rühren bei einer Temperatur zwischen 0 °C und 5 °C für 10 Stunden bis 24 Stunden;

- Zugeben einer Base, ausgewählt aus der Gruppe umfassend Triethylamin, 4-(Dimethylamino)pyridin, Piperidin, Piperazin, Pyridin, 1,4-Diazabicyclo[2.2.2]octan (DABCO), 1,8-Diazabicyclo-[5.4.0]undec-7-en (DBU) und dergleichen, zu dem Reaktionsgemisch und Rühren des Reaktionsgemischs bei einer Temperatur zwischen 20 °C und 25 °C für 30 Minuten bis 3 Stunden;
- Verdampfen des Reaktionsgemisches zur Trocknen und Reinigen des Rückstands unter Bildung einer Verbindung der Formel (II)

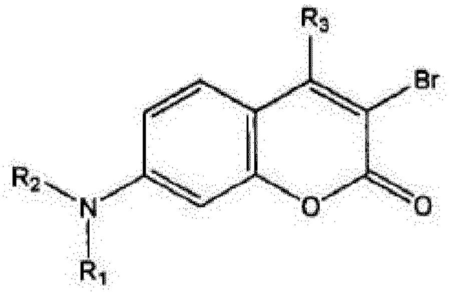

(II);

- Umsetzen einer Verbindung der Formel (II) mit Kaliumvinyltrifluorborat und einem [1,1'-bis (diphenylphosphino)-ferrocene]dichloropalladium(II) -Komplex mit Dichlormethan in einem Reaktionslösungsmittel, das aus der Gruppe ausgewählt ist, die n-Propanol, 2-Propanol, Ethanol, tert-Butanol, 2-Butanol, Acetonitril, ein gemischtes Lösungsmittel davon und dergleichen umfasst, und Rühren unter inerter Atmosphäre bei einer Temperatur zwischen 78 °C und 98 °C für 15 Minuten bis 2 Stunden; und
- Verdampfen des Reaktionsgemisches zur Trocknen und Reinigen des Rückstands unter Bildung einer Verbindung der Formel (III)

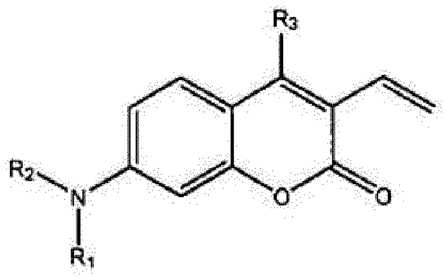

(III).

**4.** Verbindung der Formel (A)

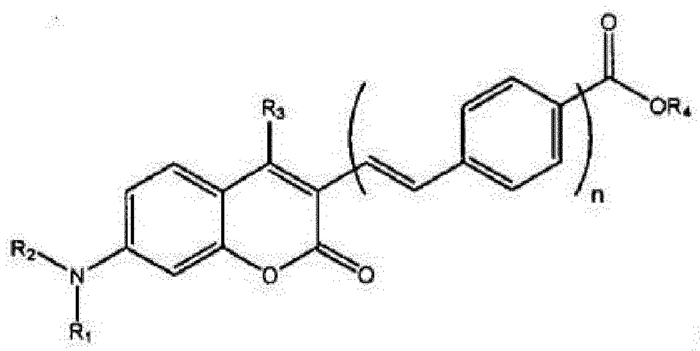

(A)

wobei:

n 1 oder 2 ist,
$R_1$ und $R_2$ unabhängig $-(CH_2)_n-CH_3$ sind, worin n 0, 1, 2 oder 3 ist;
$R_3$ $-(CH_2)_n-CH_3$ ist, worin n 0 oder 1 ist; und
$R_4$

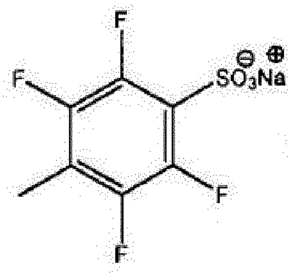

or

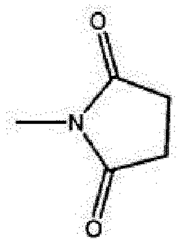

ist.

5. Verbindung nach Anspruch 4, wobei n 1 ist, $R_1$ und $R_2$ $-(CH_2)-CH_3$ sind, $R_3$ $-CH_3$ ist und $R_4$

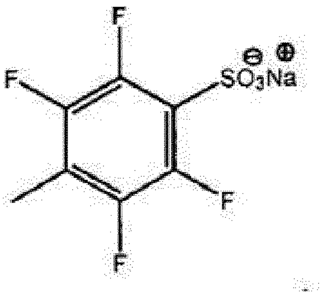

ist.

6. Verbindung der Formel (B)

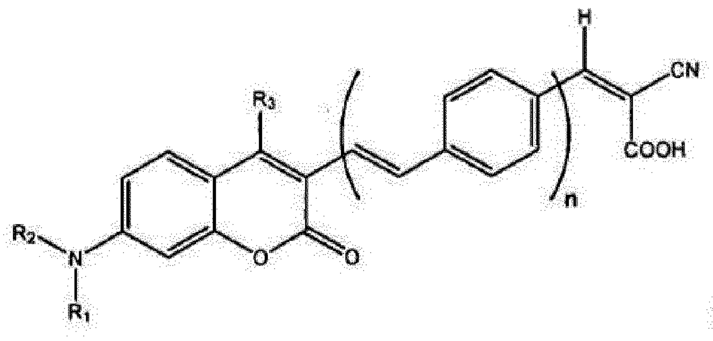

(B)

wobei:

n 1 oder 2 ist,
$R_1$ und $R_2$ unabhängig $-(CH_2)_n-CH_3$ sind, worin n 0, 1, 2 oder 3 ist; und
$R_3$ $-(CH_2)_n-CH_3$ ist, worin n 0 oder 1 ist.

7. Verbindung nach Anspruch 6, wobei n 1 ist, $R_1$ und $R_2$ $-(CH_2)-CH_3$ sind und $R_3$ $-CH_3$ ist.

## Revendications

1. Composé de formule (III)

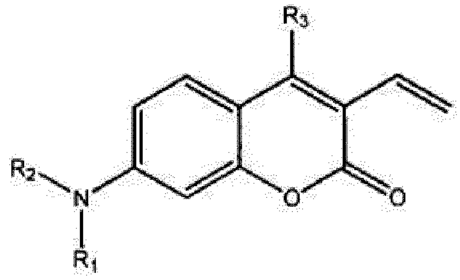

(III)

dans lequel:

$R_1$ et $R_2$ sont indépendamment $-(CH_2)n-CH_3$, où n est 0, 1, 2 ou 3; et
$R_3$ est $-(CH_2)_n-CH_3$, où n est 0 ou 1.

2. Composé selon la revendication 1, dans lequel $R_1$ et $R_2$ sont - $(CH_2)-CH_3$ et $R_3$ est $-CH_3$.

3. Procédé de synthèse de composés selon la revendication 1, comprenant les étapes suivantes:

- Réaction d'un composé de formule (I)

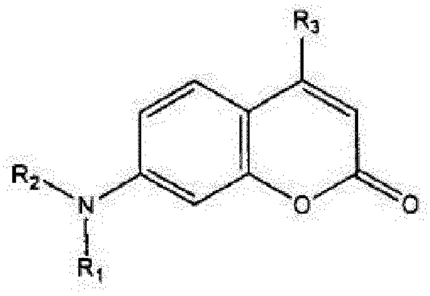

(I)

dans laquelle:

$R_1$ et $R_2$ sont indépendamment $-(CH_2)_n-CH_3$, où n est 0, 1, 2 ou 3; et
$R_3$ est $-(CH_2)n-CH_3$, où n est 0 ou 1,
Avec du brome dans un solvant de réaction choisi dans le groupe comprenant le chloroforme, le dichlorométhane, le 1,2-dichloroéthane, l'acétonitrile, un solvant mixte de ceux-ci et similaires, sous agitation à une température entre 0 °C et 5 °C pendant 10 heures à 24 heures;

- Ajouter au mélange réactionnel une base choisie dans le groupe comprenant la triéthylamine, la 4-(diméthylamino)pyridine, la pipéridine, la pipérazine, la pyridine, le 1,4-diazabicyclo-[2.2.2]octane (DABCO), le 1,8-diazabicyclo[5.4.0]undéc-7-ène (DBU) et similaires et agiter le mélange réactionnel à une température entre 20 °C et 25 °C pendant 30 minutes à 3 heures;
- Évaporation du mélange réactionnel à sec et purification du résidu pour produire un composé de formule (II)

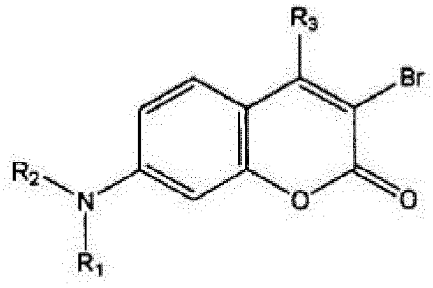

(II);

- Faire réagir un composé de formule (II) avec du vinyltrifluoroborate de potassium et un complexe de [1,1'-bis(diphenylphosphino)ferrocene]dichloropalladium(II) avec du dichlorométhane dans un solvant de réaction choisi dans le groupe comprenant le n-propanol, le 2-propanol, l'éthanol, le tert-butanol, le 2-butanol, l'acétonitrile, un mélange de solvants de ceux-ci et similaires et agiter sous atmosphère inerte à une température comprise entre 78 °C et 98 °C pendant 15 minutes à 2 heures; et
- Évaporation du mélange réactionnel à sec et purification du résidu pour produire un composé de formule (III)

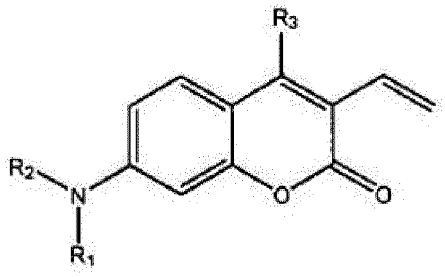

(III).

**4.** Composé de formule (A)

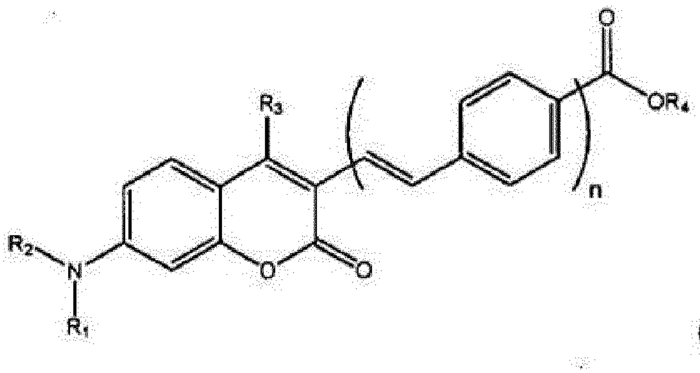

(A)

dans lequel:

n est 1 ou 2,
$R_1$ et $R_2$ sont indépendamment $-(CH_2)_n-CH_3$, où n est 0, 1, 2 ou 3;
$R_3$ est $-(CH_2)_n-CH_3$, où n est 0 ou 1; et
$R_4$ est

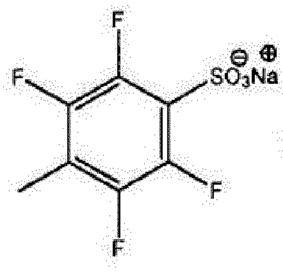

or

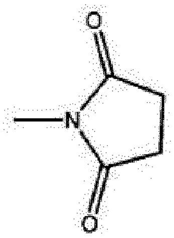

**5.** Composé selon la revendication 4, dans lequel n est 1, $R_1$ et $R_2$ sont $-(CH_2)-CH_3$, $R_3$ est $-CH_3$ et $R_4$ est

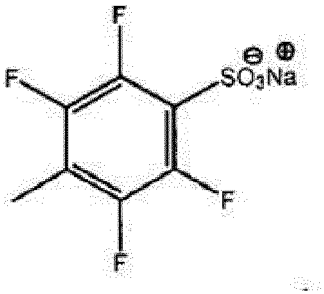

**6.** Composé de formule (B)

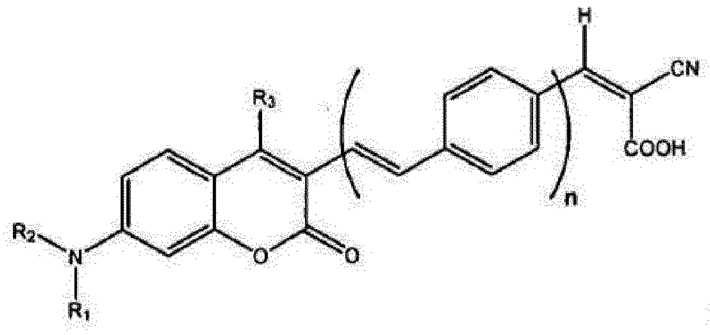

(B)

dans lequel:

n est 1 ou 2,
$R_1$ et $R_2$ sont indépendamment $-(CH_2)n-CH_3$, où n est 0, 1, 2 ou 3; et
$R_3$ est $-(CH_2)n-CH_3$, où n est 0 ou 1.

**7.** Composé selon la revendication 6, dans lequel n est 1, $R_1$ et $R_2$ sont $-(CH_2)-CH_3$ et $R_3$ est $-CH_3$.

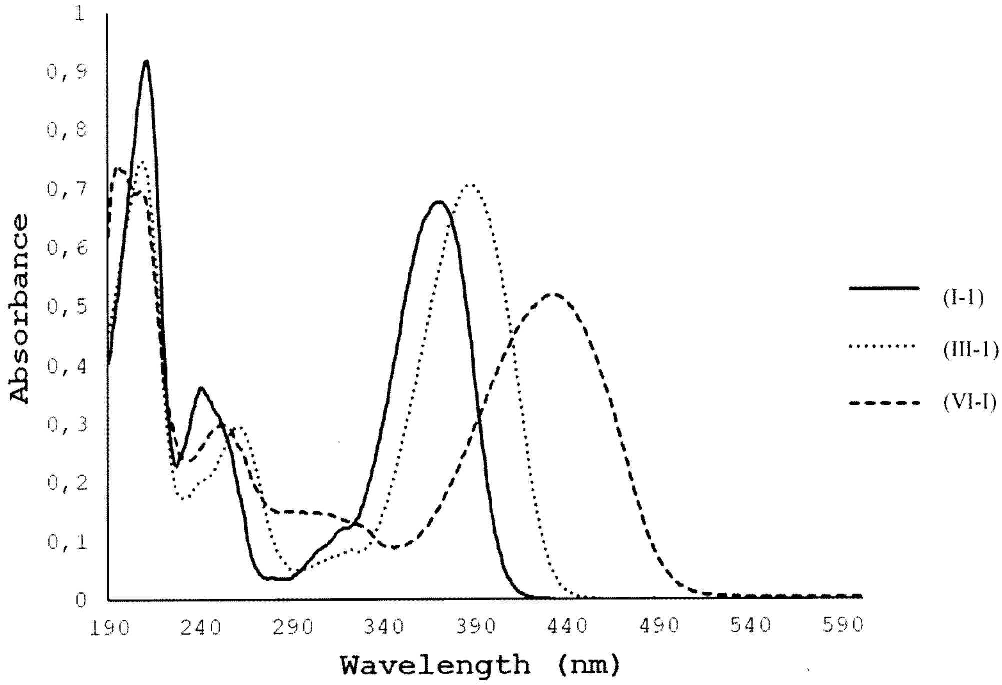


Figure 1

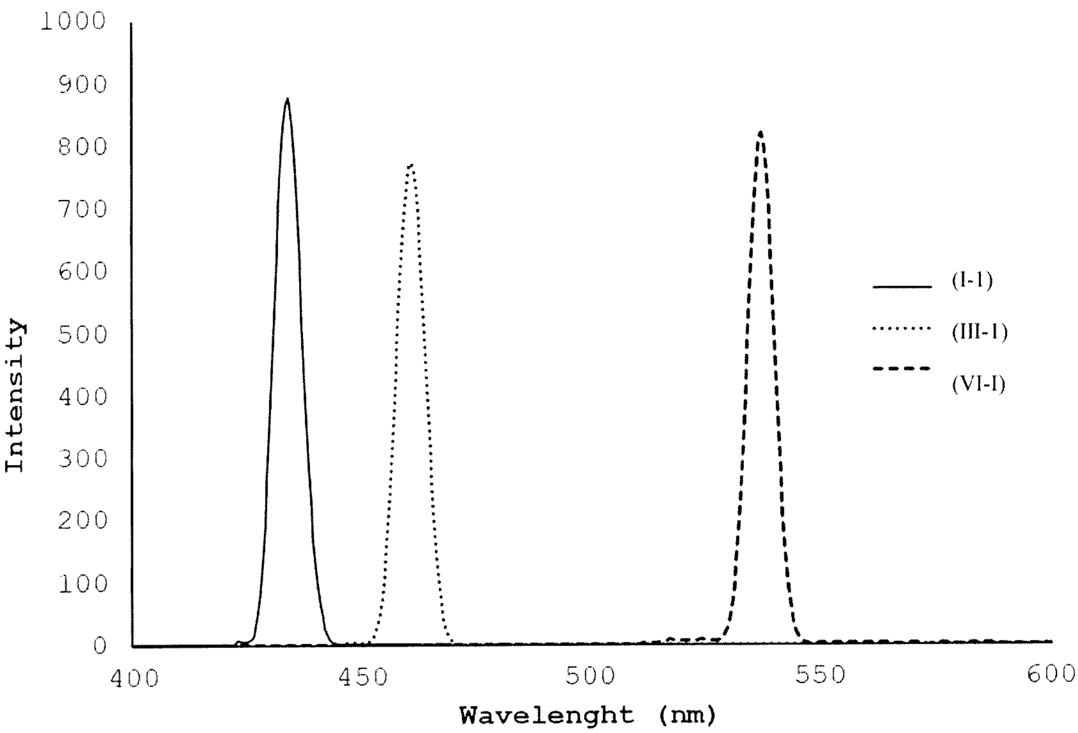


Figure 2

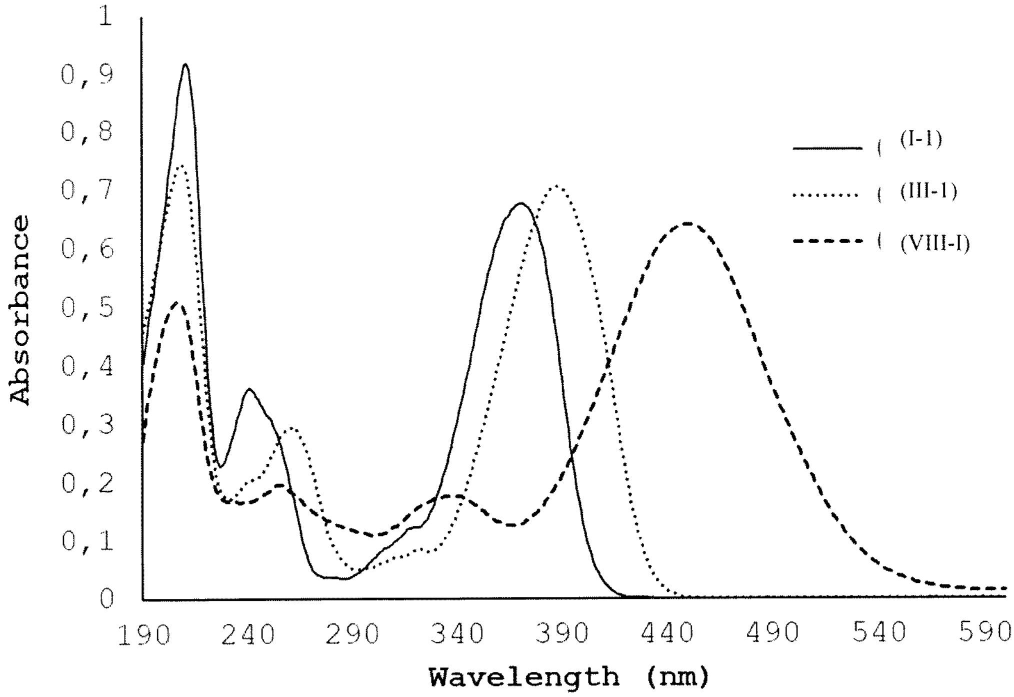


Figure 3

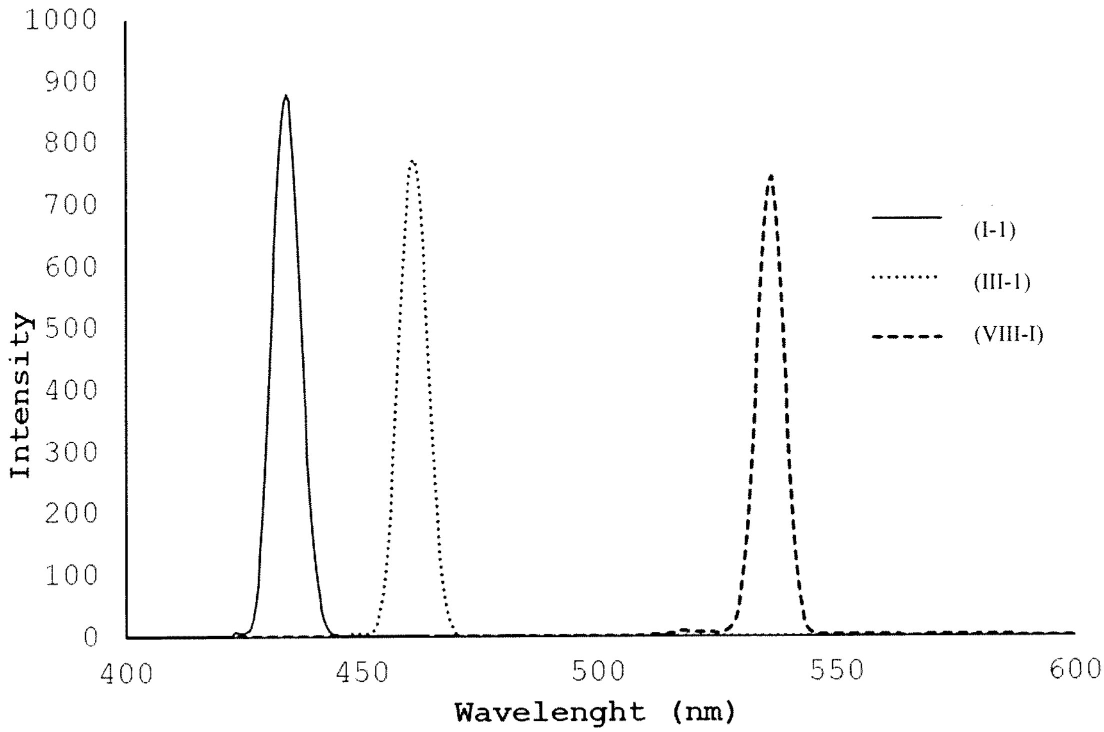


Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4956480 A


### Non-patent literature cited in the description


- **MARTINS et al.** Styryl and phenylethynyl based coumarin chromophores for dye sensitized solar cells. *Journal of Photochemistry and Photobiology A: Chemistry,* 2018, vol. 353, 546-569
- **GORDO et al.** Convenient Synthesis of 3-Vinyl and 3-Styryl Coumarins. *Organic Letters,* 2011, vol. 13 (19), 5112-5115
- **AVÓ et al.** A Family of Styrylcoumarins: Synthesis, Spectroscopic, Photophysical and Photochemical Properties. *ChemPlusChem,* 2013, vol. 78, 789-792